(19) Europäisches Patentamt
European Patent Office
Office européen des brevets

(11) EP 4 299 049 B1

# (12) EUROPEAN PATENT SPECIFICATION

(45) Date of publication and mention of the grant of the patent:
**06.05.2026 Bulletin 2026/19**

(21) Application number: **21928099.7**

(22) Date of filing: **17.12.2021**

(51) International Patent Classification (IPC):
***A61K 8/02** (2006.01)* ***A61K 8/19** (2006.01)*
***A61Q 5/00** (2006.01)* ***A45D 19/00** (2006.01)*
***A45D 7/04** (2006.01)* ***A45D 20/12** (2006.01)*
***A61Q 5/04** (2006.01)*

(52) Cooperative Patent Classification (CPC):
**A45D 20/12; A45D 2/001; A45D 7/02; A45D 7/04; A45D 7/06; A45D 19/00; A45D 19/005; A45D 19/0066; A45D 19/16; A61K 8/0241; A61Q 5/04**

(86) International application number:
**PCT/JP2021/046773**

(87) International publication number:
**WO 2022/181006 (01.09.2022 Gazette 2022/35)**

## (54) HAIR/SCALP CARE METHOD AND HAIR/SCALP CARE DEVICE

HAAR-/SKALPPFLEGEVERFAHREN UND HAAR-/SKALPPFLEGEVORRICHTUNG

PROCÉDÉ DE SOIN DES CHEVEUX/DU CUIR CHEVELU ET DISPOSITIF DE SOINS DES CHEVEUX/DU CUIR CHEVELU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2021 JP 2021030013**
**04.11.2021 JP 2021180322**

(43) Date of publication of application:
**03.01.2024 Bulletin 2024/01**

(73) Proprietor: **AISIN CORPORATION**
**Kariya-shi, Aichi 448-8650 (JP)**

(72) Inventors:
- **YOKOYAMA Keiko**
**Kariya-shi, Aichi 448-8650 (JP)**
- **INOUE Shinsuke**
**Kariya-shi, Aichi 448-8650 (JP)**

(74) Representative: **Cabinet Beau de Loménie**
**103, rue de Grenelle**
**75340 Paris Cedex 07 (FR)**

(56) References cited:
**WO-A1-2020/054100**
**JP-A- 2010 082 213**
**JP-A- 2014 212 871**
**JP-A- 2019 000 824**
**JP-A- 2019 063 192**
**JP-A- 2019 077 628**
**JP-A- 2019 077 628**
**JP-A- 2020 116 032**
**JP-A- 2020 116 032**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### TECHNICAL FIELD

The present invention relates to a hair/scalp care method and a hair/scalp care device for care for hair or a scalp.

### BACKGROUND ART

When a hair care method is performed in order to care for hair to allow the hair to be in a desired state by performing an operation such as perming, bleaching, treatment, or coloring on the hair, an agent used for each treatment operation is applied to the hair. At this time, the agent may not sufficiently permeate into the hair, and the reaction of the agent may not sufficiently proceed. In such cases, the effect brought by the agent may be reduced.

Document JP 2019 077628 A relates to a method for applying straight permanent to hair with less damage to hair.

Document JP 2020 116032 A relates to a heating and blowing apparatus to perform heating and blowing including fine water with a simple structure.

Patent Literature 1 discloses a hair coloring method configured to increase a hair temperature by applying steam to hair and to bring the periphery of the hair into a high humidity state, during agent application. By increasing the hair temperature, the reaction of the agent applied to the hair is promoted, and by placing the hair under a high humidity environment, the hair is wetted and the permeation of the agent into the hair is promoted. Further, a device for hair coloring according to Patent Literature 1 is configured to be able to deliver only steam at a set temperature by mixing steam generated in a steam generating device with outside air in a mixing chamber to set a temperature of the steam, and by separating, in a space inside the mixing chamber, condensed water generated through cooling of the steam at the time of the temperature setting. Accordingly, the condensed water generated through the cooling of the steam is removed, and thus an agent applied to hair is prevented from being subjected to dilution due to the condensed water.

### CITATIONS LIST

### PATENT LITERATURE

Patent Literature 1: JP 2000-201731 A

### SUMMARY OF INVENTION

### TECHNICAL PROBLEMS

When steam is applied to hair as described in Patent Literature 1, the hair is exposed to a high temperature to be damaged. In addition, according to Patent Literature 1, although the condensed water is removed, the steam adhering to the hair is dew condensed on the hair surface. The dew condensation water thus generated grows on the hair surface to become large water droplets, and remains on the hair surface without permeating into the hair. When the water droplets remain on the hair surface, moisture is not sufficiently supplied into the hair, and thus damage of the hair cannot be covered by moisture replenishment. Further, when an agent is applied to the hair, the water droplets remaining on the hair surface are mixed with the agent to dilute the agent. Thus, the effect brought by the agent may be reduced.

The present invention provides a hair/scalp care method capable of reducing damage to hair or repairing damaged hair, and further enhancing an effect of an agent in a case of using the agent.

### SOLUTIONS TO PROBLEMS

The present disclosure provides a hair/scalp care method including a washing step of washing a target head part that is one or both of hair and a scalp of a human body, a drying step of drying the target head part that has been washed in the washing step, and a fine water particle application step of applying fine water particles having a temperature of 40°C or lower and having a size of 50 nanometers or less to the target head part.

According to the present disclosure, the size of the fine water particles applied to the target head part is as very small as 50 nanometers or less, and the applied fine water particles enter the target head part. At this time, when the fine water particles are uncharged particles, the fine water particles are not attracted to the positively charged surface of the hair, and further easily enter the target head part. Thus, by executing the fine water particle application step, moisture is supplied to the target head part, and damage to the target head part can be reduced or the damaged target head part can be repaired. Further, the fine water particles applied to the target head part by executing the fine water particle application step efficiently permeate into the target head part, and thus the surface of the target head part is substantially not wet after the execution of the fine water particle application step. Therefore, when an agent is used, the agent is not subjected to dilution due to the moisture remaining on the surface of the target head part. In addition, the agent efficiently permeates into the hair or the scalp along with the fine water particles, and thus the effect of the agent can be enhanced.

The hair/scalp care method according to the present disclosure may not include an agent application step of applying an agent to the target head part. This is because, without applying an agent, it is possible to reduce damage to the originally damaged target head part or repair the target head part, through the fine water particle application step. For example, with regard to hair, by executing the fine water particle application step, it is possible to moisturize the hair to soften the hair, and further, it is possible to reduce the uplift of cuticles to reduce damage to the hair or repair the damaged hair, improving the glossiness of the hair.

The hair/scalp care method according to the present disclosure may further include an agent application step of applying an agent to the target head part, and may be configured such that the washing step is executed when a predetermined time period has passed after the completion of execution of the agent application step. According to this, in a hair/scalp care method involving agent application, by executing the fine water particle application step, it is possible to further enhance the effect brought by the agent. In addition, with regard to hair, by executing the fine water particle application step, it is possible to moisturize the hair to soften the hair, and further, it is possible to reduce the uplift of cuticles to reduce damage to the hair or repair the damaged hair, thereby improving the glossiness of the hair. Meanwhile, with regard to a scalp, by executing the fine water particle application step, stimulation caused by an agent is reduced while damage to the scalp is reduced.

The agent application step may be executed a plurality of times in a series of processes. When the agent application step is executed a plurality of times, the washing step may be executed after the completion of execution of each agent application step, or may be executed only once after the completion of execution of the last agent application step. Further, when the agent application step is executed a plurality of times, the fine water particle application step can be executed at a timing before a start of or after completion of execution of any of the agent application steps. For example, when the agent application step is executed a plurality of times, the fine water particle application step can be executed after the completion of execution of one agent application step and before the start of execution of a next agent application step (that is, between agent application and agent application). When the washing step is executed after the completion of execution of the one agent application step and before the start of execution of the next agent application step, the fine water particle application step can be executed after the completion of the execution of the one agent application step and before the start of the execution of the washing step, or can be executed after the completion of the execution of the washing step and before the start of the execution of the next agent application step.

In addition, when the agent application step is executed a plurality of times, the agents used in respective agent application steps may be different types of agents, or may be the same type of agent. For example, an agent used in a certain agent application step may be a perming agent, and an agent used in another agent application step may be a bleaching agent. It can also be said that the agent application step is executed a plurality of times even when the agent application is completed by applying an agent a plurality of times. For example, when a two-part (two-liquid) agent including a first agent and a second agent that are liquid is used, an agent application step of applying the first agent and an agent application step of applying the second agent are executed in a respective manner. In addition, for example, all agents used in a plurality of agent application steps may be the same type of agent. In this case, the component of the agent used in each agent application step may be changed, or an agent having the same component may be used.

When the target head part is hair, the agent used in the agent application step may be a coloring agent. According to this, by executing the fine water particle application step, it is possible to enhance the color development of the coloring agent applied to the hair, and it is possible to reduce color fading. In addition, damage to the hair due to the application of the coloring agent is reduced.

Further, when the target head part is hair, the agent used in the agent application step may be a perming agent. According to this, damage to the hair due to the application of the perming agent is reduced by the application of fine water particles, and the hair is easily formed into a desired shape.

In addition, the agent used in the agent application step may be any of a treatment agent, a bleaching agent, and a hair straightening agent. When the agent used in the agent application step is a treatment agent, hair can be further softened by execution of the fine water particle application step, and a hair tip portion fits well with the hand. Here, the state of "fitting well with the hand" means a state in which "hair has high flexibility, and the hair shape is likely to change in accordance with the degree of force when the hair is touched with the hand". In addition, when the agent used in the agent application step is a bleaching agent, the decoloring effect on hair can be enhanced by executing the fine water particle application step. When the agent used in the agent application step is a hair straightening agent, with execution of the fine water particle application step, it is possible to obtain an effect of easily changing a hair shape to a desired shape, an effect of easily retaining the hair shape in a desired shape, and an effect of softening the hair and improving the texture at the time of finish.

When the target head part is hair, and when the fine water particle application step is executed after the completion of execution of the agent application step (preferably after the completion of execution of the agent application step and before the start of execution of the drying step, and more preferably after the completion of execution of the agent application step and before the start of execution of the washing step), fine water particles may be applied to the hair in a direction from a hair tip side toward a hair root side of the hair, in the fine water particle application step. According to this, the fine water particles flow from the hair tip side toward the hair root side of the hair, and thus efficiently permeate into the hair. With this, an agent applied to the hair also efficiently permeates into the hair. As a result, it is possible to enhance the effect brought by the agent.

Further, when the target head part is hair, and when the fine water particle application step is executed simultaneously with execution of the drying step or after the completion of execution of the drying step, fine water particles may be applied to the hair in a direction from a hair root side toward a hair tip side of the hair, in the fine water particle application step. According to this, it is possible to allow the fine water particles to permeate into the hair, and it is possible to adjust cuticles opened toward the hair tip side, thereby repairing damage of the hair.

Further, the hair/scalp care method according to the present disclosure may also be configured as follows. That is, the target head part may be hair, the agent may be a perming agent including a first agent and a second agent that are liquid, the agent application step may include a first agent application step of applying the first agent to the hair and a second agent application step of applying the second agent to the hair after completion of execution of the first agent application step, and the fine water particle application step may be executed before a start of execution of the first agent application step, or after completion of execution of the first agent application step and before a start of execution of the second agent application step. According to this, by applying the fine water particles to the hair before applying the first agent to the hair, it is possible to promptly exert the function provided in the first agent on the hair. In addition, by applying the fine water particles to the hair after applying the first agent to the hair and before applying the second agent to the hair, it is possible to promptly exert the function provided in the second agent on the hair.

In this case, the first agent may have a function of cutting internal tissue of the hair and the second agent may have a function of binding the internal tissue of the hair cut by the first agent, and the hair/scalp care method may include a first leaving step of leaving the hair to stand for a first predetermined time period immediately after the completion of execution of the first agent application step and a second leaving step of leaving the hair to stand for a second predetermined time period immediately after the completion of execution of the second agent application step. The fine water particle application step can be executed after the completion of execution of the first leaving step and before the start of execution of the second agent application step.

By leaving the hair to stand for the first predetermined time period after applying the first agent to the hair, the internal tissue of the hair is cut, and the hair shape can be freely changed. In addition, by leaving the hair to stand for the second predetermined time period after applying the second agent to the hair, the cut internal tissue of the hair is recombined. As a result, the hair is allowed to conform to an intended winding shape, and the hair is fixed to the shape. Then, by applying fine water particles to the hair after the cutting of the internal tissue of the hair through the application of the first agent and before the recombination of the internal tissue of the hair through the application of the second agent, the cut internal tissue is allowed to be easily movable, whereby the hair shape is easily allowed to conform to the intended winding shape. Thus, the time period required for fixing the hair to the intended winding shape through the subsequent application of the second agent, that is, the second predetermined time period is shortened. Therefore, it is possible to shorten an operation time period for a perming operation including the fine water particle application step. In addition, it is possible to shorten the time period (second predetermined time period) for exposing the hair to the second agent, and thus it is possible to reduce the magnitude of damage given to the hair by the second agent.

Further, when the target head part is hair, and when the agent is a bleaching agent, the fine water particle application step can be executed after the completion of execution of the washing step and before the start of execution of the drying step. According to this, by applying fine water particles to the hair in a state where the hair is wet after the completion of execution of the washing step and before the start of execution of the drying step, it is possible to reduce or eliminate unruly condition of the hair.

Further, when the target head part is hair, the hair/scalp care method may include a finishing step of being executed after the completion of execution of the drying step and arranging the hair. In this case, the fine water particle application step is preferably executed in a period from before the start of execution of the agent application step to after the completion of execution of the finishing step. According to this, by executing the fine water particle application step at a predetermined timing in the hair care method including the finishing step, it is possible to change the texture of the hair. In addition, by executing the fine water particle application step before the start of execution of the finishing step, for example, after the completion of execution of the drying step and before the start of execution of the finishing step, it is possible to finish the hair with a fluffy and soft texture. Further, by executing the fine water particle application step after the completion of execution of the finishing step, it is possible to finish the hair with a moist texture.

Further, when the target head part is hair, and when agents are a plurality of treatment agents of different types, the hair/scalp care method can include a plurality of agent application steps of applying the plurality of respective treatment agents to the hair, and the fine water particle application step can be executed after any of the plurality of agent application steps or after the drying step, in accordance with the state of the hair. In addition, when the hair/scalp care method includes the finishing step, the fine water particle application step can be executed at least at any one of a timing after any of the plurality of agent application steps, a timing after the drying step, or a timing after the finishing step, in accordance with the state of the hair and the intended finish.

According to this, by applying fine water particles to the hair at any one of the timing after application of any of the plurality of treatment agents, the timing after completion of execution of the drying step, or the timing after the completion of execution of the finishing step, it is possible to enhance the effect brought by executing any of the previous steps. Thus, it is possible to perform an appropriate treatment operation in accordance with a state of hair (for example, whether damage of the hair is high or low) or an intended finish of the hair (for example, an airy finish, a heavy finish, a fluffy finish, or the like.).

Further, when the target head part is hair, the hair/scalp care method can be configured to include a heat treatment step of heat-treating the hair. In this case, the fine water particle application step can be executed before the start of execution of the heat treatment step. In addition, the fine water particle application step may include a first fine water particle application step executed before the start of execution of the heat treatment step, and a second fine water particle application step executed after the completion of execution of the heat treatment step. Here, the heat treatment step is a step of maintaining a hair shape in a desired shape by applying heat to the hair.

According to this, for example, by applying fine water particles to hair before performing the heat treatment on the hair in a hair straightening operation, it is possible to soften the hair at the time of finishing, and it is possible to enhance an effect of arranging the hair to a predetermined shape by the heat treatment. In addition, by applying fine water particles to the hair again after the heat treatment, it is possible to further soften the hair at the time of finishing.

The fine water particle application step may be executed after the completion of execution of the washing step or after the completion of execution of the drying step. Preferably, the fine water particle application step may be executed at least at any one of a timing after the completion of execution of the washing step and before the start of execution of the drying step, a timing simultaneously with execution of the drying step, or a timing after the completion of execution of the drying step. As a result, fine water particles are applied to the target head part, and damage to the target head part is reduced or the damaged target head part is repaired. In addition, by executing the fine water particle application step simultaneously with execution of the drying step, fine water particles are applied to the target head part while the wet target head part (for example, hair) is subjected to drying. As a result, it is possible to shorten a time period for the steps. In addition, by executing the fine water particle application step after the completion of execution of the drying step, fine water particles are applied to the dried target head part (for example, hair). As a result, it is possible to allow the fine water particles to efficiently permeate into the target head part, and it is possible to enhance an effect of repairing damage of the target head part.

Further, in the fine water particle application step, fine water particles having a temperature not exceeding 40°C are applied to the target head part. According to this, in the fine water particle application step, the temperature of the fine water particles applied to the target head part is 40°C or lower, which is not a high temperature, and thus the target head part is not exposed to a high temperature. Thus, execution of the fine water particle application step does not damage the target head part.

Further, the present disclosure provides a hair/-scalp care device including: a fine water particle generating element (11) caused to be, by a temperature decrease, in an absorption state of absorbing moisture on a surface, and caused to be, by a temperature increase, in a release state of releasing moisture, having been absorbed, as fine water particles having a temperature of 40°C or lower and having a size of 50 nanometers or less; applying means (12) for applying the fine water particles having been released from the fine water particle generating element (11) to a target head part that is one or both of hair and a scalp of a human body; controlling means (23) for controlling the fine water particle generating element (11) and the applying means (12); and an operation part (21) operated for causing the fine water particles to be applied to the target head part between a period before execution of washing of the target head part and a period after execution of drying of the target head part. According to this, by applying the fine water particles having a size of 50 nanometers or less having been released from the fine water particle generating element in the release state to the target head part, it is possible to reduce damage to the target head part or repair the damaged target head part.

The hair/scalp care device according to the present disclosure may be configured such that the operation part (21) is operated for causing the fine water particles to be applied to the target head part between a period before execution of agent application to the target head part prior to the execution of the washing and a period after execution of drying of the target head part. According to this, it is possible to further enhance the effect of an agent.

The hair/scalp care device according to the present disclosure is configured such that the controlling means (23) applies fine water particles having a temperature not exceeding 40°C to the target head part. According to this, the temperature of the fine water particles released from the fine water particle generating element and applied to the target head part is 40°C or lower, which is not a high temperature, and thus the target head part is not exposed to a high temperature. Therefore, by executing the fine water particle application step, the target head part is not damaged.

The hair/scalp care device according to the present disclosure may be configured such that the controlling means (23) applies fine water particles having a temperature equal to or higher than a glass-transition temperature of a protein structure of hair to the target head part. According to this, the fine water particles released from the fine water particle generating element and applied to the target head part permeate into the hair, and thus the structural change in hair is likely to occur. Therefore, it is possible to enhance an effect of reducing damage to hair or repairing the damaged hair.

The hair/scalp care device according to the present disclosure may be configured such that the target head part to which fine water particles are applied by the applying means (12) includes hair, and such that the fine water particle generating element (11) releases the fine water particles larger than a single molecule of water to allow the fine water particles to remain in hair and to improve a state of cuticles of hair.

The hair/scalp care device according to the present disclosure may be configured such that the target head part to which fine water particles are applied by the applying means (12) includes hair, and such that the fine water particle generating element (11) releases the fine water particles that are uncharged to allow the fine water particles to permeate into hair and to improve a state of cuticles of hair.

## BRIEF DESCRIPTION OF DRAWINGS

FIG. 1 is a view showing a schematic configuration of a fine water particle release device.

FIG. 2 is a view showing a schematic configuration of a fine water particle generating element.

FIG. 3 is a schematic cross-sectional view of the fine water particle generating element.

FIG. 4A is a diagram showing respective execution orders of steps in a care method according to a first embodiment.

FIG. 4B is a diagram showing examples of execution orders of steps, when an agent application step is executed twice, according to the first embodiment.

FIG. 4C is a diagram showing other examples of execution orders of steps, when the agent application step is executed twice, according to the first embodiment.

FIG. 5 is a diagram showing, using a bar graph, color differences for samples A1, B1, C1, D1 subjected to a coloring operation through respective processes A to D.

FIG. 6 is a diagram comparing color differences S7 and S14 for each of samples A2, C2, D2 and a conventional sample.

FIG. 7 shows photographs of outer appearances of samples A3, B3, C3 subjected to a perming operation after a bleaching operation.

FIG. 8 shows SEM images (magnification: 1000×) of hairs after the bleaching operation through the process B.

FIG. 9 shows SEM images (magnification: 1000×) of hairs after the bleaching operation through a conventional process.

FIG. 10 is a diagram showing execution orders of steps in a care method according to a second embodiment.

FIG. 11 is a graph showing a result of evaluation on presence or absence of glossiness for a hair root side of each sample before and after a fine water particle application step.

FIG. 12 is a graph showing a result of evaluation on presence or absence of glossiness for a hair tip of each hair sample before and after the fine water particle application step.

FIG. 13 is a graph showing subjective evaluation on stiffness of each sample.

FIG. 14 is a graph showing changes regarding rigidity before water application, rigidity immediately after water application, and rigidity one day after water application calculated for each sample.

FIG. 15 shows SEM images (magnification: 1000×) of a hair extracted from a sample A5.

FIG. 16 shows SEM images (magnification: 1000×) of a hair extracted from a sample B5.

FIG. 17 shows SEM images (magnification: 1000×) of a hair extracted from a sample C5.

FIG. 18 is a schematic diagram showing an orientation of cuticles of a hair.

FIG. 19 is a graph comparing flexural rigidity reduction rates obtained for samples A6, B6, C6, D6, E6, N6.

FIG. 20 is a graph comparing hysteresis change ranges obtained for the samples A6, B6, C6, D6, E6, N6.

FIG. 21 is a diagram showing an execution order of each step in a treatment operation while showing each execution timing of the fine water particle application step executed for a corresponding one of samples in Example 10.

FIG. 22 is a diagram showing an example of properties of each agent used in a corresponding one of agent application steps in Example 10.

FIG. 23 is a table showing an effect on hair, a finish, and a suited hair type, after a treatment operation, when the fine water particle application step is executed at each of the timings C6, D6, E6, F6 in FIG. 21, the suited hair type being each hair type suited for being subjected to application of fine water particles at a corresponding one of the timings C6, D6, E6, F6 in FIG. 21.

FIG. 24 is a diagram showing relationship between a hysteresis change range and a flexural rigidity value change rate measured for each of the samples C6, D6 and samples F6, P6.

FIG. 25A shows each step in a conventionally-performed perming operation using two liquids (a first agent and a second agent).

FIG. 25B shows each step in a perming operation using the two liquids (the first agent and the second agent), according to a fourth embodiment.

FIG. 26 is a diagram showing steps in each of a conventional process, a process F1, a process F2, and a process F3 in Example 11.

FIG. 27 is a diagram comparing respective waving efficiencies of samples A7, B7, C7, D7 subjected to a perming operation through the respective processes.

FIG. 28 is a diagram showing each step in a bleaching operation according to a fifth embodiment.

FIG. 29A is a diagram showing each step in a bleaching operation through a first comparative process.

FIG. 29B is a diagram showing each step in a bleaching operation through a second comparative process.

FIG. 30 is a photograph in which photographing was made for samples A8, B8, C8, subjected to the bleaching operations through processes (a present example process, the first comparative process, and the second comparative process).

FIG. 31 is a diagram showing examples of operations involving heat treatment.

FIG. 32 is a diagram showing steps in a hair straightening process performed in

Example 13.

FIG. 33 is a graph comparing flexural rigidity reduction rates obtained for each of samples A9, B9, C9, D9, N9.

DESCRIPTION OF EMBODIMENTS

(First Embodiment)

In a first embodiment, a hair care method involving application of an agent, that is, a hair care method including an agent application step will be described.

The hair care method involving the application of an agent is performed through at least the following four steps, in the present embodiment.

(1) Agent application step

In the agent application step, an agent is applied to hair. For example, when a coloring operation is performed, a coloring agent is applied to hair, when treatment is performed, a treatment agent is applied to hair, when a perming operation is performed, a perming agent is applied to hair, and when a bleaching operation is performed, a bleaching agent is applied to hair. The application method is typically application by brushing, but may be application by spraying.

(2) Washing step

In the washing step, hair is washed to remove an agent applied to the hair. This washing is typically water washing, but an agent (washing agent) for washing an agent applied in the agent application step may be used. In this case, the washing agent may be applied to the hair before the water washing, or the hair may be washed in a state where the washing agent is mixed with water. The washing method typically includes water washing by shower. Thus, the hair on which the execution of the washing step is completed is wet.

(3) Drying step

In the drying step, the wet hair washed in the washing step is dried. For a drying method, a method is typically used in which the wet hair is blown by warm air or heated air using a hair dryer to blow off or evaporate moisture from the hair, thereby removing the moisture.

(4) Fine water particle application step

In the fine water particle application step, fine water particles are applied to hair. The fine water particle application step will be described later.

Among the above four steps, the agent application step, the washing step, and the drying step are executed in this order. Here, the washing step is executed after a predetermined time period has passed from the completion of execution of the agent application step. That is, after the completion of the execution of the agent application step, hair is left to stand for a certain time period. Thus, a leaving step is executed until the predetermined time period has passed, after the completion of the execution of the agent application step. The predetermined time period (standing time period) varies depending on an agent to be applied, but may be, for example, 5 minutes to 30 minutes. During this predetermined time period, the agent applied to the hair permeates into the hair and reacts. Thus, it can be said that the permeation and reaction of the agent are in progress during this predetermined time period (standing time period). That is, it can be said that the leaving step is a permeation/reaction step for an agent. When the agent permeates into and reacts with the hair in the leaving step, this exerts a predetermined effect on the hair. This effect is, for example, an effect of dyeing the hair in a predetermined color in a case of a coloring operation, an effect of nourishing the hair and softening the hair in a case of a treatment operation, an effect of decoloring the hair in a case of a bleaching operation, and an effect of forming a wave having an appropriate shape in the hair in a case of a perming operation. Note that when it is found in advance, through hair diagnosis or the like, that the hair is damaged, before execution of the agent application step, a preprocess step for repairing the damage of the hair may be performed before the care method according to the present embodiment is performed.

In the fine water particle application step, as described above, fine water particles are applied to hair. Specifically, in the fine water particle application step, a plurality of fine water particles, having a temperature not exceeding 40°C (preferably a temperature lower than 40°C, and more preferably a temperature of 25°C or higher and lower than 40°C) and a size of 50 nm or less, are carried along with air to the hair by, for example, air blowing, and are applied to the hair. In the present embodiment, as an example, uncharged fine water particles are applied along with air to hair, using a fine water particle release device.

FIG. 1 is a view showing a schematic configuration of a fine water particle release device. As shown in FIG. 1, a fine water particle release device 1 includes a fine water particle release unit 10 and a control unit 20.

The fine water particle release unit 10 included in the fine water particle release device 1 includes a fine water particle generating element 11, a fan 12, an inlet filter 13a, an outlet filter 13b, and a case 14.

The case 14 is formed in a substantially cylindrical shape, and a flow path 14a communicating from one end to the other end is formed inside the case 14. The inlet filter 13a is attached to the one end side of the case 14, and the outlet filter 13b is attached to the other end side. Further, the case 14 includes a first case portion 141 and a second case portion 142, which are formed to be connected with each other along the axial direction. The opening of the first case portion 141 forms an inlet 14in that is an opening at the one end of the case 14, and the opening of the second case portion 142 forms an outlet 14out that is an opening at the other end of the case 14.

The fan 12 is a propeller fan that is rotationally driven by a motor (not shown), and is housed in the flow path 14a inside the first case portion 141 of the case 14. Note that the fan 12 may be a sirocco fan or the like. The fan 12 rotates in conjunction with the rotation of the motor, and is configured to be able to suck air from the inlet 14in of the case 14 into the flow path 14a and discharge the sucked air from the outlet 14out of the case 14.

The fine water particle generating element 11 is disposed in the flow path 14a of the case 14 along with the fan 12. The fine water particle generating element 11 is disposed in the flow path 14a inside the second case portion 142 of the case 14. In FIG. 1, the fine water particle generating element 11 is disposed on the downstream side of the flow path 14a (the side closer to the outlet 14out) with respect to the fan 12.

FIG. 2 is a view showing a schematic configuration of the fine water particle generating element 11 disposed in the second case portion 142. As shown in FIG. 2, the fine water particle generating element 11 is disposed to extend over the entire cross section of the flow path 14a in the second case portion 142. However, the fine water particle generating element 11 is formed such that air can flow therethrough. Thus, the air flowing through the flow path 14a from the inlet 14in toward the outlet 14out passes through the fine water particle generating element 11.

FIG. 3 is a schematic cross-sectional view of the fine water particle generating element 11. As shown in FIG. 3, the fine water particle generating element 11 includes a base material 111, and a conductive polymer film 112 formed on one surface or both surfaces (formed on one surface in FIG. 3) of the base material 111. The base material 111 is made of a material having conductivity, such as a metal material such as a stainless-steel-based metal or a copper-based metal, a carbon-based material, a conductive ceramic material (for example, ITO), or a conductive resin material (for example, a metal-deposited resin film, a nano-silver coating resin, or a carbon nanotube (CNT) coating resin). In the present embodiment, a metal foil made of stainless steel to which aluminum is added is used. The base material 111 is formed in such a shape that air in the flow path 14a can flow when the base material 111 is disposed in the flow path 14a. Further, when the base material 111 is disposed in the flow path 14a, the base material 111 is formed such that the contact area with the air flowing through the flow path 14a becomes as large as possible, that is, the surface area becomes as large as possible. In this case, the base material 111 may be formed of, for example, a plurality of flat plates. In addition, the base material 111 may be formed such that a cross-sectional shape perpendicular to the flow path 14a has a honeycomb shape or a spiral shape.

The conductive polymer film 112 is formed in a film shape by being made of a polymer compound having conductivity such as, for example, a thiophene-based conductive polymer compound. In the present embodiment, the conductive polymer film is made of PEDOT/PSS (poly(3,4-ethylenedioxythiophene)/poly(styrene sulfonic acid)) among thiophene-based conductive polymers. PEDOT/PSS has a core-shell structure in which a hydrophilic PSS (shell) surrounds a core formed by aggregation of water-insoluble PEDOT, and the shape of a single core-shell is substantially ellipsoidal. Such ellipsoidal particles (core-shell particles) are aligned to form a stacked structure, so that the conductive polymer film 112 is formed in a film shape. A gap having a nanometer size of about 2 nm is formed between adjacent core-shell particles, and each gap is connected to form a nanochannel opened on the surface of the conductive polymer film 112. In addition, the central core (PEDOT) of each of the core-shell particles is hydrophobic, and thus many hydrophilic sulfonic acid groups exist on the outer periphery of the shell (PSS). Therefore, many sulfonic acid groups exist in the nanochannel surrounded by the outer wall of the core-shell particles. The sulfonic acid group is a polar functional group and can be hydrogen-bonded. Thus, moisture in the air in the nanochannel can be hydrogen-bonded to the sulfonic acid groups to be retained in the nanochannel as bound water.

When a moisture amount of the surface of the conductive polymer film 112 is larger than the moisture amount of the bound water in the nanochannel, the moisture on the surface moves into the nanochannel by using a moisture concentration difference therebetween as a drive source, and is retained as bound water. As a result, the moisture is absorbed into the nanochannel. In contrast, when the moisture amount of the surface is smaller than the moisture amount of the bound water in the nanochannel, the bound water in the nanochannel moves toward the surface by using the moisture concentration difference therebetween as a drive source. As a result, the moisture is released from the nanochannel. Thus, the conductive polymer film 112 is configured such that an absorption state in which water is absorbed and a release state in which water is released can be switched by the moisture concentration difference.

Further, when the temperature of the conductive polymer film 112 is increased, water release is further promoted as compared with a case in which water is released by the moisture concentration difference. When the temperature of the conductive polymer film is decreased, water absorption is further promoted as compared with a case in which water is absorbed by the moisture concentration difference. Thus, the conductive polymer film 112 is also configured such that the absorption state and the release state can be switched by a temperature change.

In addition, the channel width of the nanochannel is about 2 nm. Thus, the moisture released from the nanochannel is nanoparticles having a size of 2 nm or less. The nanoparticles (fine water particles) having a size of 2 nm remain at a size of 50 nm or less, even when the nanoparticles are aggregated (clustered) in the vicinity of the opening of the nanochannel. Thus, the size (for example, the particle diameter) of fine water particles released from the conductive polymer film 112 is 50 nm or less. In addition, the bound water retained in the nanochannel is not charged. Thus, fine water particles that have a size of 50 nm or less and that are uncharged are released from the conductive polymer film 112.

As shown in FIG. 1, the control unit 20 included in the fine water particle release device 1 includes an operation part 21, a power supply circuit 22, and a control part 23. The operation part 21 includes, for example, a plurality of operation buttons provided on a surface of a housing or the like that supports the fine water particle release unit 10. These operation buttons are operated by a user to turn on and turn off power, to select an operation mode, and to perform other operations.

Power such as AC 100 V is supplied to the power supply circuit 22. The power supply circuit 22 is electrically connected to the motor of the fan 12 through a first electric wire 24, and is electrically connected to the base material 111 of the fine water particle generating element 11 through a second electric wire 25. The power supply circuit 22 is configured to convert the supplied power into power suitable for driving the motor of the fan 12, and is configured to be able to output the converted power to the first electric wire 24. Further, the power supply circuit 22 is configured to convert the supplied power into power suitable for supply to the base material 111, and is configured to be able to output the converted power to the second electric wire 25.

A first normally-open changeover switch 26 is interposed in the first electric wire 24, and a second normally-open changeover switch 27 is interposed in the second electric wire 25. The first normally-open changeover switch 26 is operated to be opened to cut off conduction of the first electric wire 24, and is operated to be closed to allow conduction of the first electric wire 24. The second normally-open changeover switch 27 is operated to be opened to cut off conduction of the second electric wire 25, and is operated to be closed to allow conduction of the second electric wire 25.

An operation status of the operation part 21 is input to the control part 23. The control part 23 controls switching states of the first normally-open changeover switch 26 and the second normally-open changeover switch 27, in accordance with the input operation status of the operation part 21.

The fine water particle release device 1 having the above configuration is configured to be able to operate in accordance with any of the operation modes that are a "water absorption mode" and a "water release mode". In this case, the mode may be switched through automatic selection by determining the state of hair or a scalp, or the mode may be switched through manual selection by a user operating the operation buttons of the operation part 21. Further, the water absorption mode and the water release mode can be switched at a predetermined timing on the basis of an operation of a user. Note that in addition to the water absorption mode and the water release mode, there may be another operation mode.

When the water release mode is selected, the control part 23 controls each changeover switch such that both the first normally-open changeover switch 26 and the second normally-open changeover switch 27 are operated to be closed. As a result, power is supplied from the power supply circuit 22 to both the motor of the fan 12 and the base material 111 of the fine water particle generating element 11. When power is supplied to the motor of the fan 12, the motor rotates and the fan 12 rotates in conjunction with the rotation of the motor, and air is sucked into the flow path 14a from the inlet 14in of the case 14. The air sucked into the flow path 14a is discharged from the outlet 14out after the air has passed through the fine water particle generating element 11. Further, when the base material 111 of the fine water particle generating element 11 is energized and a current flows through the conductive base material 111, the base material 111 generates Joule heat to generate heat. The heat generated from the base material 111 is transferred to the conductive polymer film 112 on the base material 111, whereby the temperature of the conductive polymer film 112 increases. Note that the conductive polymer film 112 itself may be energized to cause the conductive polymer film 112 to generate heat and to cause the temperature thereof to increase, or a space in which the conductive polymer film 112 is present may be warmed to cause the temperature to increase. When the temperature of the conductive polymer film 112 increases in this manner, water release from the conductive polymer film 112 is promoted. As a result, fine water particles that are uncharged and that have a size of 50 nm or less are released from the conductive polymer film 112. The released fine water particles are mixed with the air flowing through the flow path 14a, and are released from the outlet 14out along with the air. Further, in the water release mode, the control part 23 controls the temperature of the conductive polymer film 112 such that the temperature of the fine water particles released from the conductive polymer film 112 does not exceed 40°C (preferably the temperature is lower than 40°C, and more preferably the temperature is 25°C or higher and lower than 40°C). Specifically, the control part 23 controls the energization amount of the base material 111 such that the temperature of the fine water particles released from the conductive polymer film 112 does not exceed 40°C (preferably, the temperature is lower than 40°C, and more preferably, the temperature is 25°C or higher and lower than 40°C). Thus, the fine water particles, which are uncharged and have a size of 50 nm or less, and which further have a temperature of 40°C or lower (preferably a temperature lower than 40°C, and more preferably a temperature of 25°C or higher and lower than 40°C), are released from the conductive polymer film 112. Note that in the water release mode, the control part 23 may be configured to be able to adjust the air flow rate for a release from the outlet 14out by controlling the energization amount to the motor of the fan 12.

Meanwhile, when the water absorption mode is selected, the control part 23 controls each changeover switch such that the first normally-open changeover switch 26 is operated to be closed and the second normally-open changeover switch 27 is operated to be opened. When the first normally-open switch 26 is operated to be closed, power is supplied from the power supply circuit 22 to the motor of the fan 12, the motor rotates and the fan 12 rotates in conjunction with the rotation of the motor, and air flows into the flow path 14a from the inlet 14in of the case 14. Then, the flowed air is discharged from the outlet 14out after the flowed air has passed through the fine water particle generating element 11. Further, the second normally-open changeover switch 27 is operated to be opened, and thus no electric power is supplied to the base material 111 of the fine water particle generating element 11. Therefore, the base material 111 does not generate heat, and heat is not transferred from the base material 111 to the conductive polymer film 112. In addition, the conductive polymer film 112 is cooled through the air blown by the rotation of the fan 12, and thus the temperature of the conductive polymer film 112 decreases. When the temperature of the conductive polymer film 112 decreases in this manner, water absorption into the conductive polymer film 112 is promoted. As a result, moisture in the air passing through the fine water particle generating element 11 is absorbed into the conductive polymer film 112.

Thus, when the operation mode of the fine water particle release device 1 is the "water release mode", fine water particles, which are uncharged and have a size of 50 nm or less, and which have a temperature not exceeding 40°C (preferably a temperature lower than 40°C, and more preferably a temperature of 25°C or higher and lower than 40°C), are released along with air, from the outlet 14out of the case 14 of the fine water particle release unit 10. Therefore, when the fine water particle application step is executed, the operation mode of the fine water particle release device 1 is set to the water release mode, and the fine water particle release device 1 is driven while the outlet 14out of the case 14 is caused to face hair. Note that the size of the single molecule of water is about 0.3 nm, and thus the size of the fine water particles released from the fine water particle release device 1 is more than 0.3 nm and 50 nm or less.

In the present embodiment, the fine water particle application step can be executed at any desired timing. In particular, the fine water particle application step can be executed at any one or a plurality of the following four timings:

(A) Before the start of execution of the agent application step (in a case of executing the preprocess step, before the start of execution of the preprocess step, or after the completion of execution of the preprocess step and before the start of execution of the agent application step)

(B) After the completion of execution of the agent application step (for example, after the completion of execution of the agent application step and before the start of execution of the washing step, or after the completion of execution of the washing step and before the start of execution of the drying step)

(C) After the completion of execution of the drying step

(D) Simultaneously with execution of the drying step

When the fine water particle application step is executed at the timing (A), as shown in part (a) of FIG. 4A, the steps are executed in the order of "the fine water particle application step → the agent application step → the washing step → the drying step". A hair care method in which each step is executed in this order is referred to as a process A.

When the fine water particle application step is executed at the timing (B), for example, as shown in part (b) of FIG. 4A, the steps are executed in the order of "the agent application step → the fine water particle application step → the washing step → the drying step". A hair care method in which each step is executed in this order is referred to as a process B. Note that in the process B, the fine water particle application step may be executed substantially simultaneously with execution of the leaving step after agent application (that is, within the standing time period after agent application), or may be executed immediately after the completion of execution of the leaving step (that is, immediately after the standing time period has passed after agent application), between the completion of execution of the agent application step and the start of execution of the washing step. Further, in this process B and the above process A, the fine water particle application step is executed at a timing before the start of execution of the washing step. Note that when the fine water particle application step is executed at the timing (B), the steps may be executed in the order of "the agent application step → the washing step → the fine water particle application step → the drying step".

When the fine water particle application step is executed at the timing (C), the steps are executed in the order of "the agent application step → the washing step → the drying step → the fine water particle application step". A hair care method in which each step is executed in this order and the fine water particle application step is executed from immediately after the completion of execution of the drying step until before six hours pass is referred to as a process C. The execution order of each step in the process C is shown in part (c) of FIG. 4A. In addition, a hair care method in which the fine water particle application step is executed after 6 to 30 hours pass after the completion of execution of the drying step is referred to as a process D. The execution order of each step in the process D is shown in part (d) of FIG. 4A.

When the fine water particle application step is executed at the timing (D), the steps are executed in the order of "the agent application step → the washing step → the drying + fine water particle application step". A hair care method in which each step is executed in this order is referred to as a process E. The execution order of each step in the process E is shown in part (e) of FIG. 4A.

In addition, the agent application step may be executed a plurality of times in a series of processes. When the agent application step is executed a plurality of times, the washing step may be executed after the completion of execution of each agent application step, or may be executed only once after the completion of execution of the last agent application step. Further, when the agent application step is executed a plurality of times, the fine water particle application step can be executed at the timing shown in (A) or (B) above with respect to any of the agent application steps. For example, when the agent application step is executed twice, the steps can be executed in the following orders:

- The first agent application step →the fine water particle application step → the washing step → the second agent application step → the washing step → the drying step (see part (a) of FIG. 4B)
- The first agent application step → the washing step → the second agent application step → the fine water particle application step → the washing step → the drying step (see part (b) of FIG. 4B)
- The first agent application step → the washing step → the fine water particle application step → the second agent application step → the washing step → the drying step (see part (c) of FIG. 4B)
- The fine water particle application step → the first agent application step → the washing step → the second agent application step → the washing step → the drying step (see part (d) of Fig. 4B)
- The first agent application step → the fine water particle application step → the second agent application step → the washing step → the drying step (see part (e) of FIG. 4C)
- The first agent application step → the second agent application step → the fine water particle application step → the washing step → the drying step (see part (f) of FIG. 4C)
- The fine water particle application step → the first agent application step → the second agent application step → the washing step →the drying step (see part (g) of FIG. 4C)

According to each of the orders of the steps shown in parts (a) and (c) of FIG. 4B, and part (e) of FIG. 4C, the fine water particle application step is executed between the first agent application step (one agent application step) and the second agent application step (next agent application step), that is, after the completion of execution of the first agent application step (one agent application step) and before the start of execution of the second agent application step (next agent application step). In addition, according to each of the orders of the steps shown in part (b) of FIG. 4B and part (f) of FIG. 4C, the fine water particle application step is executed after the completion of execution of the second agent application step. Further, according to each of the orders of the steps shown in part (d) of FIG. 4B and part (g) of FIG. 4C, the fine water particle application step is executed before the start of execution of the first agent application step. In addition, according to the order of the steps shown in part (g) of FIG. 4C, the first agent application step and the second agent application step are continuously executed, and thus these agent application steps can be regarded as a single agent application step. In this case, the process performed in the order of the steps shown in part (g) of FIG. 4C is the same as the process B in FIG. 4A. Note that the above examples merely exemplify the orders of the steps when a plurality of agent application steps are executed, and the fine water particle application step can also be executed in another order of the steps other than those exemplified above.

In addition, when the agent application step is executed a plurality of times, the agents used in respective agent application steps may be different types of agents, or may be the same type of agent. For example, an agent used in a certain agent application step may be a perming agent, and an agent used in another agent application step may be a bleaching agent. It can also be said that the agent application step is executed a plurality of times even when the agent application is completed by applying an agent a plurality of times. For example, when a two-part (two-liquid) agent such as a perming agent or a hair straightening agent is used, an agent application step (first agent application step) of applying a first-part agent (first agent) and an agent application step (second agent application step) of applying a second-part agent (second agent) are executed in a respective manner. In addition, for example, all agents used in a plurality of agent application steps may be the same type of agent. In this case, the component of the agent used in each agent application step may be changed, or an agent having the same component may be used.

By executing the fine water particle application step at any of the above timings, fine water particles, which are uncharged, which have a temperature not exceeding 40°C (preferably a temperature lower than 40°C, and more preferably a temperature of 25°C or higher and lower than 40°C), and which have a size of 50 nm or less, are applied to hair. The temperature of the fine water particles applied to hair is 40°C or lower (preferably lower than 40°C, and more preferably 25°C or higher and lower than 40°C), and thus, by executing this step, the hair is not damaged because the hair is not exposed to a high temperature. In addition, by executing this step, fine water particles having a size of 50 nm or less permeate into hair. Further, the fine water particles applied to the hair are uncharged, and thus the fine water particles are not attracted by static electricity or the like of the hair. Therefore, it is also possible to prevent inhibition in which fine water particles are inhibited from permeating into hair by adsorbing onto the hair surface due to static electricity or the like. In this manner, by applying fine water particles to hair in the fine water particle application step according to the present embodiment, it is possible to supply moisture into the hair, whereby the effect of an agent applied in the agent application step can be further enhanced while damage to the hair can be reduced or the damaged hair can be repaired.

The temperature of the fine water particles applied in the fine water particle application step is 40°C or lower, and preferably lower than 40°C. Further, the temperature of the fine water particles applied in the fine water particle application step is preferably 25°C or higher. When hair is damaged, the damage is reduced by causing a structural change in tissue forming the hair (hereinafter referred to as structural change in hair), or the damaged tissue is repaired by the structural change in hair. When fine water particles are supplied into hair in the fine water particle application step according to the present disclosure, the fine water particles bind to the tissue of the hair or permeate into the gap of the tissue, thereby causing the structural change in hair. Further, whether or not the structural change in hair occurs is related to the temperature at which the protein structure of the hair changes (the glass-transition temperature). When the temperature of the protein structure of the hair is equal to or lower than the glass-transition temperature, the structural change in hair is less likely to occur. When fine water particles are applied into hair and a moisture amount in the hair increases, the glass-transition temperature of the protein structure of the hair is considered to be about 25°C. This is because when the temperature of the fine water particles is lower than 25°C, it is difficult to obtain an effect of reducing damage to hair or repairing the damaged hair. Thus, the temperature of the fine water particles applied to hair or a scalp is preferably 25°C or higher. Therefore, the most preferable range of the temperature of the fine water particles applied in the fine water particle application step is a range of 25°C or higher and lower than 40°C.

(Example 1: Confirmation of color development effect in coloring process)

Four bundles of hair samples (each sample is about 50 cm in length, and 25 g in weight) were prepared. Then, a coloring operation was performed on each prepared sample. At this time, the respective processes of the process A, the process B, the process C, and the process D in FIG. 4A were performed on the separate samples, whereby a sample A1 subjected to the coloring operation through the process A, a sample B1 subjected to the coloring operation through the process B, a sample C1 subjected to the coloring operation through the process C, and a sample D1 subjected to the coloring operation through the process D were prepared. Note that each procedure of a corresponding one of the steps is the same among the processes A to D, and each outline thereof is as follows:

The agent application step: a commercially available coloring agent was uniformly applied on the sample by brushing.

The washing step: after 20 minutes had passed from the completion of the execution of the agent application step, the agent was removed from the sample by rubbing the sample with fingers while washing the sample with water by shower. Next, washing was then performed with a shampoo for one minute, then rinsing was performed with water for one minute, a hair rinse was subsequently applied to the sample for one minute, and finally rinsing was performed with water for one minute.

The drying step: after the completion of the execution of the washing step, the sample was subjected to towel drying for 10 seconds, and then moisture was removed from the sample surface by blowing warm air onto the wet sample for about three minutes using a hair dryer.

The fine water particle application step: fine water particles (air flow rate: 0.07 $m^2$/min.) at a temperature of about 35°C were applied to the sample for 20 minutes, using the fine water particle release device 1 shown in FIG. 1.

Further, in the process A, the agent application step was executed immediately after the completion of execution of the fine water particle application step. In the process B, the fine water particle application step was executed immediately after the completion of execution of the agent application step. In the process C, the fine water particle application step was executed immediately after the completion of execution of the drying step. In the process D, the fine water particle application step was executed 24 hours (one day) after the completion of execution of the drying step.

Further, as a comparison, one bundle of hair sample was prepared, and by subjecting the sample to the coloring operation through a conventional process, a conventional sample was prepared. Here, the conventional process is a hair care method in which hair is subjected to a coloring operation by executing the agent application step, the washing step, and the drying step, having respective procedures similar to the above procedures, in this order. That is, the conventional process is a hair care method in which the fine water particle application step is omitted.

The L* value, a* value, and b* value in the L*a*b* color system (CIE 1976 L*a*b* color space) were measured, using a color-difference meter, for each of the samples A1, B1, C1, D1 and the conventional sample subjected to the coloring operation through the respective processes. Then, on the basis of each measured value, a difference in color between each of the samples A1, B1, C1, D1 and the conventional sample was quantified as a color difference for each sample. Here, the color difference for each of the samples A1, B1, C1, D1 is a distance in the L*a*b* color space between the value measured in each of the samples A1, B1, C1, D1 and the value measured in the conventional sample, and is calculated by Formula (1) below.

$$\text{Color difference} = \sqrt{(\Delta L^2 + \Delta a^2 + \Delta b^2)} \quad (1)$$

In Formula (1):

$$\Delta L = L - L0, \Delta a = a - a0, \Delta b = b - b0;$$

L: the L* value of each of the samples A1, B1, C1, D1;
L0: the L* value of the conventional sample;
a: the a* value of each of the samples A1, B1, C1, D1;
a0: the a* value of the conventional sample;
b: the b* value of each of the samples A1, B1, C1, D1; and
b0: the b* value of the conventional sample.

FIG. 5 is a diagram showing, using a bar graph, color differences for the respective samples A1, B1, C1, D1 subjected to the coloring operation through the respective processes A, B, C, D, with respect to the conventional sample. In FIG. 5, the bar graph A1 represents a color difference between the sample A1 and the conventional sample, the bar graph B1 represents a color difference between the sample B1 and the conventional sample, the bar graph C1 represents a color difference between the sample C1 and the conventional sample, and the bar graph D1 represents a color difference between the sample D1 and the conventional sample. According to FIG. 5, it can be seen that each color difference with respect to the conventional sample is two or more, in any case where the coloring operation was performed through any of the processes A to D. Further, it can be seen that the color difference is larger in the order of the process A, the process D, the process B, and the process C.

Further, it was visually observed that the color of the conventional sample was the dullest color, and that the sample was brighter and deeper in color as the color difference of the sample with respect to the conventional sample was larger. Thus, it can be said that as the color difference is larger, the color is brighter and deeper. That is, it can be said that as the color difference is larger, the degree of coloring increases and the color development is more excellent. Therefore, it can be seen that the hair subjected to the coloring operation through the processes A, B, C, D can further improve color development compared with the hair subjected to the coloring operation through the conventional process. That is, the effect of an agent can be further enhanced by using the hair care method according to the present embodiment. In particular, it is possible to further improve color development by performing the coloring operation through the process A or the process D.

The reason why the color development is excellent when the coloring operation is performed through the processes A, B, C, D will be considered. In the fine water particle application step executed in each of the processes A, B, C, D, fine water particles, which have a temperature not exceeding 40°C, which have a size of 50 nm or less, and which are uncharged, are applied to a sample of hair. The temperature of the fine water particles applied to the sample does not exceed 40°C, and thus the application of the fine water particles does not expose the hair to a high temperature. Thus, by executing the fine water particle application step, hair or a scalp is not damaged. Further, the fine water particles applied to the sample are very small, and thus the probability that the fine water particles encounter one another after the fine water particles are released from the fine water particle release device is very low. Therefore, the probability that the fine water particles aggregate is also low. Accordingly, the fine water particles travel toward a hair while the fine water particles maintain the size thereof, that is, while the fine water particles maintain the size of 50 nm or less. Further, cuticles forming the surface of a hair have a multi-layered structure, and a cell membrane complex (hereinafter referred to as a CMC) exists between adjacent cuticles. This CMC serves as a passage for water, an agent, or the like. Thus, water or an agent permeates into a hair through the CMC. The width of the CMC is about 50 nm. Thus, the fine water particles applied to a hair in the fine water particle application step can permeate into the hair through the CMC, while the fine water particles maintain the size of 50 nm or less without aggregation. Further, the fine water particles applied to a hair in the fine water particle application step are uncharged, and thus the fine water particles are not attracted by static electricity or the like of the hair. Thus, most of the fine water particles applied in the fine water particle application step enter the hair, and do not remain on the hair surface.

As described above, the fine water particles efficiently enter the hair by executing the fine water particle application step, and thus damage to the hair is reduced or the damaged hair is repaired. In addition, the fine water particles efficiently enter the hair, and thus the hair surface is substantially not wet. Therefore, the agent applied to the hair is not diluted by the fine water particles, and thus it is possible to effectively prevent occurrence of a problem such as deterioration of the effect of the agent due to dilution of the agent.

In the case of the process A, fine water particles permeate into hair before the agent application, damage to the hair is reduced or the damaged hair is repaired, and the hair is moisturized. Thus, it is considered that the hair can sufficiently absorb the agent through the subsequent agent application, thereby improving the color development. Further, in the case of the process B, by simultaneously performing the application of fine water particles and the permeation of an agent, the permeation of the agent is promoted along with the permeation of the fine water particles into the hair while damage to hair is reduced or the damaged hair is repaired. Thus, it is considered that the agent sufficiently permeates into the hair, and that as a result, the color development is improved. Further, in the case of the processes C and D, it is considered that by supplying moisture (fine water particles) to hair after drying the hair, damage to the hair is reduced or the damaged hair is repaired to cause cuticles on the hair surface to be closed, thereby improving the glossiness of the hair and improving the color development.

Further, in the case of the process B, the fine water particle application step is executed during a predetermined time period (20 minutes in this example) after the completion of execution of the agent application step and before the start of execution of the washing step. The leaving step (permeation/reaction step) for allowing an agent applied to hair to permeate into and react with the hair is executed during the time period from the completion of execution of the agent application step to the start of execution of the washing step. Thus, the predetermined time period (that is, the leaving step) needs to be set also in the other processes A, C, D and the conventional process. Therefore, the process B, in which the fine water particle application step is executed within the predetermined time period (that is, simultaneously with the leaving step), after the completion of execution of the agent application step and before the start of execution of the washing step, that needs to be set, has an advantage that the overall process time period can be shortened as compared with the processes A, C, D, in which a period for executing the fine water particle application step needs to be independently provided.

(Example 2: Confirmation of color-fading reduction effect after coloring operation)

Four bundles of hair samples (each sample is about 50 cm in length, and 25 g in weight) were prepared, and each sample was subjected to a coloring operation through a corresponding one of the processes A, C, D and the conventional process with respective procedures similar to those in Example 1. As a result, a sample A2 subjected to the coloring operation through the process A, a sample C2 subjected to the coloring operation through the process C, a sample D2 subjected to the coloring operation through the process D, and a conventional sample subjected to the coloring operation through the conventional process were prepared.

For each of the prepared samples, the L* value, a* value, and b* value were measured as initial values, using a color-difference meter. Next, a shampoo/rinse step and the drying step were continuously repeated for each sample for which the initial values were measured. Here, the shampoo/rinse step is a step of performing washing with a shampoo, water washing, application of a hair rinse, and water washing in this order. That is, the shampoo/rinse step is a step simulating hair washing in a general household. Further, in the drying step, each sample was dried using a hair dryer.

After the shampoo/rinse step and the drying step were repeatedly executed seven times for each of the samples A2, C2, D2 and the conventional sample, the L* value, a* value, and b* value were measured again as values after seven-time repetition, using the color-difference meter. Then, the shampoo/rinse step and the drying step were further repeated seven times in succession on each of the samples A2, C2, D2 and the conventional sample for which the values after seven-time repetition were obtained. As a result, the shampoo/rinse step and the drying step were repeatedly executed totally fourteen times after the respective processes, on the samples A2, C2, D2 and the conventional sample. Thereafter, for each sample, the L* value, a* value, and b* value were measured again as values after fourteenth-time repetition, using the color-difference meter.

Next, for each sample, a color difference S7 between the initial value and the value after seven-time repetition and a color difference S14 between the initial value and the value after fourteenth-time repetition were calculated on the basis of Formula (1) represented above. Here, in Formula (1):

$$\Delta L = L - L0, \Delta a = a - a0, \Delta b = b - b0;$$

L: the value after seven-time repetition or the value after fourteenth-time repetition of the L* value of each sample;

L0: the initial value of the L* value of each sample;
a: the value after seven-time repetition or the value after fourteenth-time repetition of the a* value of each sample;
a0: the initial value of the a* value of each sample;
b: the value after seven-time repetition or the value after fourteenth-time repetition of the b* value of each sample; and
b0: the initial value of the b* value of each sample.

FIG. 6 is a diagram comparing the color difference S7 and the color difference S14 for each of the samples A2, C2, D2 and the conventional sample, and the vertical axis represents the color difference. Further, in FIG. 6, the graph denoted by "A2" represents a change from the color difference S7 to the color difference S14 for the sample A2, the graph denoted by "C2" represents a change from the color difference S7 to the color difference S14 for the sample C2, the graph denoted by "D2" represents a change from the color difference S7 to the color difference S14 for the sample D2, and the graph denoted by "conventional" represents a change from the color difference S7 to the color difference S14 for the conventional sample.

According to FIG. 6, in the conventional sample, the color difference S14 is considerably larger than the color difference S7. That is, when the number of repetitions of the shampoo/rinse step and the drying step increases from 7 to 14, the color difference becomes larger. In contrast, in each of the samples A2, C2, D2, the color difference S7 and the color difference S14 are not so different from each other. That is, even when the number of repetitions of the shampoo/rinse step and the drying step increases from 7 to 14, the color differences are not so different from each other. Further, when the shampoo/rinse step is repeated on hair subjected to a coloring operation, the color is faded, and thus the magnitude of the change in the color difference can be regarded as the magnitude of the degree of color fading. That is, it is considered that as the color difference is larger, the color applied on the sample through the coloring operation is faded. Thus, it can be seen that when the coloring operation is performed through the conventional process, color fading due to the repetition of the subsequent shampoo/rinse step and drying step is large. In contrast, it can be said that when the coloring operation is performed through the processes A, C, D, the color fading due to the repetition of the subsequent shampoo/rinse step and drying step is small. Therefore, with the coloring operation through the process A, the process C, and the process D, an effect of reducing color fading was recognized.

The reason why the effect of reducing color fading is recognized with the coloring operation through the processes A, C, D is considered to be because by applying the fine water particles to the hair before or after the application of the coloring agent to uniformly distribute the fine water particles in the hair, a structure in the hair is adjusted to reduce damage to the hair. Specifically, in the case of the process A in which a coloring agent is applied after hair is softened without being excessively wet by applying fine water particles, it is considered that by applying the coloring agent to the hair whose damage is reduced to be softened, the coloring agent further permeates into the hair without being diluted, thereby reducing color fading. In addition, in the case of the processes C, D in each of which fine water particles are applied to hair after a coloring agent is applied, it is considered that after the coloring agent is applied, the cuticles of the hair are closed to reduce the outflow of the coloring agent while damage to the hair is reduced, thus reducing color fading.

(Example 3: Confirmation of wave forming effect when perming operation is performed after bleaching)

Three bundles of hair samples (each sample is about 50 cm in length, and 25 g in weight) were prepared. Then, on each sample, a bleaching operation was performed using a bleaching agent, and subsequently, a perming operation was performed using a two-part (two-liquid) perming agent (a first-part agent + a second-part agent). Samples A3, B3, C3 on each of which the perming operation was performed after the bleaching operation were thus prepared. Here, the sample A3 was prepared by performing the bleaching operation through the process B in FIG. 4A and then performing the perming operation through the process B in FIG. 4A, the sample B3 was prepared by performing the bleaching operation through the process B in FIG. 4A and then performing the perming operation through a conventional process, and the sample C3 was prepared by performing the bleaching operation through the conventional process and then performing the perming operation through the conventional process. Further, in this example, the outline of the procedure of each step in the bleaching operation and the perming operation in the process B in FIG. 4A is as follows. Further, the conventional process was performed with a procedure in which the fine water particle application step is omitted from the procedure of the steps in the process B in FIG. 4A.

• Bleaching operation

The agent (bleaching agent) application step: a commercially available bleaching agent was uniformly applied on the sample by brushing.

The fine water particle application step: fine water particles (flow rate: 0.07 $m^2$/min.) at a temperature of about 35°C were applied to the sample for 20 minutes, using the fine water particle release device 1 shown in FIG. 1.

The washing step: after 20 minutes had passed from the completion of the execution of the agent (bleaching agent) application step, the agent was removed from the sample by rubbing the sample with fingers while washing the sample with lukewarm water by shower.

The drying step: after the completion of the execution of the washing step, moisture was removed from the sample surface by blowing warm air onto the wet sample for about two minutes using a hair dryer.

• Perming operation

The agent (perming agent) application step: after the hair sample was subjected to winding (rod-winding), a first-part agent included in a commercially available perming agent was uniformly applied on the sample by brushing, and then the sample was left to stand for 15 minutes. Thereafter, a second-part agent was uniformly applied on the sample by brushing. Note that after the completion of execution of the agent application step, the sample was left to stand for 20 minutes.

The fine water particle application step: immediately after the brushing of the second-part agent (that is, during the leaving of the sample to stand for 20 minutes), fine water particles (flow rate: 0.07 $m^2$/min.) at a temperature of about 35°C were applied to the sample for 20 minutes, using the fine water particle release device 1 shown in FIG. 1.

The washing step: after 20 minutes had passed from the completion the execution of the agent (perming agent) application step (that is, after the completion of the execution of the fine water particle application step), the agent was removed from the sample by rubbing the sample with fingers while washing the sample with water by shower.

The drying step: after the completion of the execution of the washing step, moisture was removed from the sample surface by blowing warm air onto the wet sample for about two minutes using a hair dryer.

FIG. 7 shows photographs of the samples A3, B3, C3. Part (a) of FIG. 7 shows the sample A3, part (b) of FIG. 7 shows the sample B3, and part (c) of FIG. 7 shows the sample C3. As shown in part (c) of FIG. 7, when the bleaching operation and the perming operation were performed through the conventional process, waves were not sufficiently formed in the hair. In contrast, as shown in part (a) of FIG. 7, when the bleaching operation and the perming operation were performed through the process B, waves were appropriately formed in the hair. Thus, by using the process B, it is possible to form waves in hair through a perming operation after a bleaching operation.

The reason why the sample C3 cannot have sufficiently formed waves is considered to be because the hair is highly damaged through the bleaching operation, thereby causing elasticity of the hair to be impaired. In contrast, in the sample A3, fine water particles, which have a size of 50 nm or less, and which are uncharged and have a temperature of 40°C or lower, were applied to the sample at the time of the bleaching operation and the perming operation. The fine water particles permeate into the hair without remaining on the hair surface of the sample as described above. Then, by the moisture permeating into the hair, for example, the cuticles are closed, and the damaged hair is repaired. Thus, the hair is less damaged after the bleaching operation, and the hair has sufficient elasticity. In addition, by applying fine water particles to the hair at the time of the perming operation, damage to the hair is reduced. Thus, it is considered that waves are sufficiently formed in the hair through the perming operation.

(Example 4: Confirmation of state of cuticles of hair after bleaching operation)

A hair was extracted from the sample (the sample A3 or the sample B3 before the perming operation) subjected to the bleaching operation through the process B in Example 3, and the state of cuticles on the surface was confirmed by using an SEM. In addition, a hair was extracted from the sample (the sample C3 before the perming operation) subjected to the bleaching operation through the conventional process in Example 3, and the state of cuticles on the surface was confirmed by using the SEM.

FIG. 8 shows SEM images (magnification: 1000×) of two hairs A41, A42 extracted from the sample subjected to the bleaching operation through the process B, and FIG. 9 shows SEM images (magnification: 1000×) of two hairs N1, N2 extracted from the sample subjected to the bleaching operation through the conventional process.

As shown in FIG. 8, it can be seen that cuticles on the surfaces of the hairs A41, A42 subjected to the bleaching operation through the process B were closed. That is, the uplift of the cuticles was small. In contrast, as shown in FIG. 9, cuticles on the surfaces of the hairs N1, N2 subjected to the bleaching operation through the conventional process were opened. That is, the uplift of the cuticles was conspicuous. When the uplift of cuticles is conspicuous, the hair may be damaged. In contrast, when the uplift of cuticles is small, it is considered that the hair is not damaged. Thus, it can be seen that damage to hair can be reduced or the damaged hair can be repaired by performing a bleaching operation through the process B.

(Second Embodiment)

In a second embodiment, a hair care method without application of an agent, that is, a hair care method without including an agent application step will be described. Here, the hair care method without application of an agent refers to a method of reducing damage to hair or repairing the damaged hair, and improving the glossiness of the hair, without using an agent. Note that conventionally, in order to reduce damage to hair or repair the damaged hair, a treatment agent is typically applied to the hair as an agent after the hair is washed with a shampoo or the like. By applying the treatment agent, the hair is moisturized, and damage to the hair is reduced or the damaged hair is repaired. In contrast, in the present embodiment, damage to the hair can be reduced or the damaged hair can be repaired, without applying an agent such as a treatment agent to the hair.

In the present embodiment, the hair care method without application of an agent is performed through at least the following three steps.

(1) Washing step

In the washing step, hair is washed to remove contaminants such as impurities adhering to the hair. Examples of the washing method include washing of hair or a scalp with a shampoo, and subsequent water washing. The washing step may be executed only by water washing.

(2) Drying step

In the drying step, the wet hair washed in the washing step is dried. For a drying method, a method of removing moisture from wet hair by blowing warm air or heated air to the wet hair using a hair dryer is typically used.

(3) Fine water particle application step

In the fine water particle application step, fine water particles, which are uncharged, which have a temperature not exceeding 40°C (preferably a temperature lower than 40°C, and more preferably a temperature of 25°C or higher and lower than 40°C), and which have a size of 50 nm or less, are applied to hair. In this case, the fine water particles can be applied to hair using the fine water particle release device 1 described in the first embodiment.

Among the above three steps, the washing step and the drying step are executed in this order. Meanwhile, the fine water particle application step is executed after the completion of execution of the washing step or after the completion of execution of the drying step, or is executed simultaneously with execution of the drying step. FIG. 10 is a diagram showing execution orders of the steps necessary for processes according to the present embodiment. Part (a) of FIG. 10 shows the execution order of each step when the fine water particle application step is executed after the completion of execution of the washing step (more precisely, after the completion of execution of the washing step and before the start of execution of the drying step). Part (b) of FIG. 10 shows the execution order of each step when the fine water particle application step is executed after the completion of execution of the drying step. Part (c) of FIG. 10 shows the execution order of each step when the fine water particle application step is executed simultaneously with execution of the drying step.

By executing the fine water particle application step, fine water particles, which are uncharged, which have a temperature not exceeding 40°C (preferably a temperature lower than 40°C, and more preferably a temperature of 25°C or higher and lower than 40°C), and which have a size of 50 nm or less, are applied to hair. The temperature of the fine water particles applied to hair does not exceed 40°C, and thus by executing this step, the hair is not damaged because the hair is not exposed to a high temperature. In addition, by executing this step, fine water particles having a size of 50 nm or less permeate into a hair through a CMC between cuticles of the hair. Further, the fine water particles applied to the hair are uncharged, and thus the fine water particles are not attracted by static electricity or the like of the hair. Accordingly, it is also possible to prevent inhibition in which fine water particles are inhibited from permeating into hair by adsorbing onto the hair surface due to static electricity or the like. In this manner, by applying fine water particles to hair in the fine water particle application step according to the present embodiment, it is possible to supply moisture into the hair to allow the hair to be moisturized, closing cuticles on the hair surface, whereby damage to the hair can be reduced or the damaged hair can be repaired, and the glossiness of the hair can be improved and the hair can be further moisturized.

In addition, as shown in part (c) of FIG. 10, by executing the fine water particle application step simultaneously with execution of the drying step, and by applying fine water particles to hair while drying the wet hair, the overall process time period can be shortened.

(Example 5 ... Subjective evaluation on presence or absence of glossiness of hair)

Eight bundles of hair samples (each sample is about 50 cm in length, and 25 g in weight) were prepared. Then, on each of the prepared samples, the washing step and the drying step were executed in this order, and subsequently, the fine water particle application step was executed on each dried sample. The outline of the procedure of each step is as follows.

The washing step: a predetermined amount of commercially available shampoo was applied to the sample, and then the sample was rubbed with fingers. Thereafter, the sample was rubbed with fingers while the sample was washed with water by shower, and thereby contaminants adhering to the sample and the agent liquid of the shampoo were removed.

The drying step: after the completion of the execution of the washing step, moisture was removed from the sample surface by blowing warm air onto the wet sample for about three minutes using a hair dryer.

The fine water particle application step; fine water particles (flow rate: 0.07 $m^2$/min.) at a temperature of about 35°C were applied to the sample for 20 minutes, using the fine water particle release device 1 shown in FIG. 1.

Next, three hairdressers performed subjective evaluation on presence or absence of the glossiness of the hair-root-side portion and the hair tip portion of each sample before and after execution of the fine water particle application step. The subjective evaluation was performed by the three hairdressers each evaluating presence or absence of the glossiness before and after execution of the fine water particle application step on each sample with five levels of 1, 2, 3, 4, and 5 to give a score. Here, the score is an integer that is any of 1, 2, 3, 4, and 5, and each hairdresser determined the score on the basis of a subjective view and gave the score such that the score becomes higher as the degree of glossiness increases and the score becomes lower as the degree of glossiness decreases. Then, the average value of the scores given by the three hairdressers was determined as the score regarding presence or absence of the glossiness of the sample.

FIG. 11 is a graph showing the result of the evaluation on the presence or absence of the glossiness for the hair-root-side portion of each sample before and after the execution of the fine water particle application step. FIG. 12 is a graph showing the result of evaluation on the presence or absence of the glossiness for the hair tip portion of each sample before and after the execution of the fine water particle application step. The numerical values on the horizontal axis of the graphs in FIGS. 11 and 12 are sample numbers (No. 1, No. 2, No. 3, No. 4, No. 5, No. 6, No. 7, and No. 8). In each of the respective columns corresponding to the numbers, a bar graph (graph A) for the score (average value) regarding the presence or absence of the glossiness evaluated before the start of the execution of the fine water particle application step is arranged on the left hand, and a bar graph (graph B) for the score (average value) regarding the presence or absence of the glossiness evaluated after the completion of the execution of the fine water particle application step is arranged on the right hand. In addition, each of the bar graphs shown in each drawing is drawn to extend upward when the score is higher than the score 3 and extend downward when the score is lower than the score 3, with reference to the score 3.

As shown in FIGS. 11 and 12, in each of both the hair-root-side portion and the hair tip portion of the hair, the proportion of the samples in each of which the score after the execution of the fine water particle application step is higher than the score before the execution of the fine water particle application step is seven samples out of eight samples. That is, in each of the seven bundles out of the eight bundles of the samples, the effect that the glossiness of the hair was improved by executing the fine water particle application step was recognized.

(Example 6 ... Subjective evaluation on stiffness of hair before and after fine water particle application step)

Further, for the samples No. 1, No. 2, No. 3, No. 4, No. 5, No. 6, No. 7, No. 8 used in the above experiment, the stiffness of each of the samples before and after the execution of the fine water particle application step was confirmed by the three hairdressers with fingers. Then, subjective evaluation on the stiffness of the sample before and after the execution of the fine water particle application step was performed in a five-level evaluation similar to that described above, and the average value thereof was calculated. Here, the score is an integer that is any of 1, 2, 3, 4, and 5, and each hairdresser determined the score on the basis of a subjective view and gave the score such that the score becomes higher as the degree of stiffness increases and the score becomes lower as the degree of softness increases.

FIG. 13 is a graph showing the result of the evaluation on the stiffness of each sample before and after the fine water particle application step. The numerical values on the horizontal axis in FIG. 13 are the sample numbers (No. 1, No. 2, No. 3, No. 4, No. 5, No. 6, No. 7, and No. 8). In each of the respective columns corresponding to the numbers, a bar graph (graph C) for the score (average value) of the stiffness evaluated before the start of the execution of the fine water particle application step is arranged on the left hand, and a bar graph (graph D) for the score (average value) of the stiffness evaluated after the completion of the execution of the fine water particle application step is arranged on the right hand. In addition, each of the bar graphs shown in FIG. 13 is drawn to extend upward when the score is higher than the score 3 and extend downward when the score is lower than the score 3, with reference to the score 3.

As can be seen from FIG. 13, in each of six samples out of eight samples, the score of the stiffness of the sample after the completion of the execution of the fine water particle application step is lower than the score of the stiffness of the sample before the start of the execution of the fine water particle application step. From this result, it was confirmed that in each of the six bundles out of the eight bundles of the samples, the hair was softened by executing the fine water particle application step, that is, the flexural rigidity of the hair was decreased.

(Example 7 ... Change in flexural rigidity of hair before and after execution of fine water particle application step)

Two bundles of hair samples No. 9, No. 10 (each sample is about 50 cm in length, and 25 g in weight) were prepared, and each of the prepared samples was subjected to the washing step and the drying step with respective procedures similar to those in Example 5. Thereafter, for each of a plurality of hairs included in each sample, a hair-root-side portion of the hair was mounted, on a one-by-one basis, to a measurement unit of a bending tester. The hair mounted to the measurement unit was bent at a change rate of 0.4 $(cm^{-1} \square s^{-1})$ within a range of curvature K = -2.5 to +2.5 $(cm^{-1})$, and flexural stress at that time was sequentially measured. After the measurement, a flexural stress-curvature curve, which is a relationship between the flexural stress and the curvature K, was obtained from the flexural stress sequentially obtained in accordance with the change in curvature. Then, from the obtained flexural stress-curvature curve, the average of the slopes (gradients) of the curves in a range of curvature K = 0.5 to 1.5 $cm^{-1}$ and a range of curvature K = -1.5 to -0.5 $cm^{-1}$ was calculated as the flexural rigidity (unit: N□$m^2$) of the hair-root-side portion. In this manner, the flexural rigidity was calculated for each of all the hairs included in each sample. Then, values in a range of 10% to 20% in the order of the ones having higher flexural rigidity (top 10% to 20% having higher flexural rigidity) were extracted, and an average value of the extracted values was calculated as a rigidity value before water application.

Next, on the sample No. 9, the fine water particle application step with a procedure similar to that in Example 5 was executed. Meanwhile, on the sample No. 10, a water immersion step of immersing the sample in water for one hour was executed. Subsequently, immediately after the fine water particle application step or the water immersion step was executed on each sample, a bending test similar to that described above was performed again, on a one-by-one basis, on each of the plurality of hairs included in each sample, using the bending tester. From the test result (flexural stress-curvature curve), flexural rigidity of the hair-root-side portion was calculated in a manner similar to that described above. Then, values in a range of the top 10% to 20% having higher flexural rigidity were extracted, and an average value of the extracted values was calculated as a rigidity value immediately after water application.

Subsequently, each sample was left to stand in an atmosphere at a temperature of 24 to 27°C and a humidity of 50 to 65% RH for one day (24 hours), and after the completion of the leaving to stand, a bending test similar to that described above was performed again, on a one-by-one basis, on each of the plurality of hairs included in each sample, using the bending tester. From the test result (flexural stress-curvature polarity), flexural rigidity of the hair-root-side portion was calculated in a manner similar to that described above. Then, values in a range of the top 10% to 20% having higher flexural rigidity were extracted, and an average value of the extracted values was calculated as a rigidity value one day after water application. Note that on the basis of a finding that the hairs having values in the range of the top 10% to 20% of the flexural rigidity have influence on a sense of rigidity (sense of stiffness) of the entire sample, the average value of the values in the range of the top 10% to 20% of the higher flexural rigidity was calculated, as described above, as the value representing the rigidity.

FIG. 14 is a graph showing changes regarding the rigidity value before water application, the rigidity value immediately after water application, and the rigidity value one day after water application calculated for each sample. Here, the vertical axis in FIG. 14 represents a change rate in the flexural rigidity value (the rigidity value immediately after water application or the rigidity value one day after water application) of each sample when the rigidity value before water application of each sample is one. Thus, the rigidity value before water application is one in each sample. In addition, the graph of each sample is denoted by the number of each sample.

As shown in FIG. 14, in each of the sample No. 9 and the sample No. 10, the rigidity value immediately after water application is smaller than the rigidity value before water application. This is because moisture was supplied to the hairs included in the sample to soften the hairs.

Further, in the sample No. 9, the rigidity value one day after water application is smaller than the rigidity value before water application. In contrast, in the sample No. 10, the hair surface included in the sample was wetted because the sample was immersed in water. For this reason, the rigidity value immediately after water application becomes smaller, but the rigidity value one day after water application returns to the same level as the rigidity value before water application. This is considered to be because all the moisture on the hair surface was evaporated after one day had passed after the sample had been immersed in water.

From the above result, it can be seen that in the sample No. 9, the hair was softer even after one day had passed after the fine water particle application step had been executed compared with the hair before the execution of the fine water particle application step. This is considered to be because moisture is taken into the hair by executing the fine water particle application step, and because the taken moisture is retained even after one day has passed. As a result, it was confirmed that the hair was softened and the moisturizing effect of the hair was enhanced by executing the fine water particle application step according to the present embodiment.

(Example 8 ... Comparison of state of cuticles)

Three bundles of hair samples A5, B5, C5 (each sample is about 30 cm in length, and 15 g in weight) were prepared. On the sample A5, the washing step and the drying step with respective procedures similar to those in Example 5 were executed in this order, then a hair a5 was extracted from the sample A5, and the fine water particle application step was executed on the extracted hair a5. Then, the surface of the hair a5 was observed by an SEM at each of the following timings: a timing after the completion of the execution of the drying step and before the start of the execution of the fine water particle application step; a timing immediately after the fine water particle application step was executed for 10 minutes; and a timing immediately after the fine water particle application step was further subsequently executed for 20 minutes (executed for 30 minutes in total). Moreover, on the sample B5, the washing step and the drying step with respective procedures similar to those in Example 5 were executed in this order, then a hair b5 was extracted from the sample B5, and vaporized moisture was applied to the extracted hair b5 for 30 minutes using a vaporization-type humidifier. In addition, the surface of the hair b5 was observed by the SEM at each of the following timings: a timing after the completion of the execution of the drying step and before the application of the vaporized moisture using the vaporization-type humidifier; and a timing immediately after the application of the vaporized moisture for 30 minutes. Further, on the sample C5, the washing step and the drying step with respective procedures similar to those in Example 5 were executed in this order, then a hair c5 was extracted from the sample C5, and fine particle ions containing negative ions were applied to the extracted hair c5 for 10 minutes using a fine particle ion dryer. In addition, the surface of the hair c5 was observed by the SEM at each of the following timings: a timing after the execution of the drying step and before the application of the fine particle ions using the fine particle ion dryer; and a timing immediately after the application of the fine particle ions for 10 minutes. Further, the fine water particle application step with a procedure similar to that in Example 5 was executed for 10 minutes on the hair c5 to which the fine particle ions were applied for 10 minutes. Then, the surface of the hair c5 was observed by the SEM at a timing after the execution of the fine water particle application step.

FIG. 15 shows SEM images (magnification: 1000×) of the hair a5 extracted from the sample A5. Part (a) of FIG. 15 is an SEM image of the hair a5 imaged at the timing before the start of the execution of the fine water particle application step. Part (b) of FIG. 15 is an SEM image of the hair a5 imaged at the timing immediately after the fine water particle application step was executed for 10 minutes after the imaging of part (a) of FIG. 15. Part (c) of FIG. 15 is an SEM image of the hair a5 imaged at the timing immediately after the fine water particle application step was further subsequently executed for 20 minutes (for 30 minutes in total) after the imaging of part (b) of FIG. 15. As can be seen from FIG. 15, after the completion of the execution of the fine water particle application step (parts (b) and (c) of FIG. 15), the uplift of the cuticles was smaller than that before the start of the execution (part (a) of FIG. 15). Comparison between parts (b) and (c) of FIG. 15 shows that as the execution time period of the fine water particle step was longer , the uplift of the cuticles became smaller. From this result, it can be recognized that by executing the fine water particle application step according to the present embodiment, there is an effect of reducing damage to hair or repairing damaged hair, that is, an effect of reducing the uplift of cuticles (closing cuticles) and improving the state of the cuticles. This is because, since the size of the fine water particles, applied to hair by executing the fine water particle application step according to the present embodiment, is as finely small as 50 nm or less and is uncharged, the water particles enter a water passage (CMC) existing between cuticles of the hair to moisturize the hair.

FIG. 16 shows SEM images (magnification: 1000×) of the hair b5 extracted from the sample B5. Part (a) of FIG. 16 is an SEM image of the hair b5 imaged at the timing before the vaporized moisture was applied using the vaporization-type humidifier. Part (b) of FIG. 16 is an SEM image of the hair b5 imaged at the timing immediately after the vaporized moisture was applied for 30 minutes using the vaporization-type humidifier after the imaging of part (a) of FIG. 16. As can be seen from FIG. 16, before and after the application of the vaporized moisture, the cuticles were uplifted largely. In addition, before and after the application of the vaporized moisture, the state of the cuticles showed little change. Thus, it can be said that even when vaporized moisture is applied to hair using a vaporization-type humidifier, an effect of improving the state of the cuticles of the hair (an effect of reducing the uplift of the cuticles) is not recognized. It is considered that vaporized moisture applied to hair from a vaporization-type humidifier is a single molecule of water (size: about 0.3 nanometers), and that the vaporized moisture enters the hair but is present in a state (as free water) in which the vaporized moisture can freely enter and leave due to a temperature change or the like. Thus, it is considered that the vaporized moisture does not contribute to adjusting tissue in the hair. In contrast, it can be said that the fine water particles applied to hair in the fine water particle application step according to the present embodiment or the first embodiment described above are present in the hair in a state of being able to remain in the hair without constantly entering and leaving the hair due to a temperature change or the like, because, as shown in the graph of the sample No. 9 in FIG. 14, the effect brought by applying the fine water particles in the fine water particle application step was sustained even after one day had passed. Thus, it is considered that the fine water particles applied to hair in the fine water particle application step according to the present embodiment or the first embodiment described above are present in a state (as bound water) bound to biological tissue such as a protein in the hair, thereby acting to adjust the tissue in the hair.

FIG. 17 shows SEM images (magnification: 1000×) of the hair c5 extracted from the sample C5. Part (a) of FIG. 17 is an SEM image of the hair c5 imaged at the timing before the fine particle ions were applied using the fine particle ion dryer. Part (b) of FIG. 17 is an SEM image of the hair c5 imaged at the timing immediately after the fine particle ions were applied for 10 minutes using the fine particle ion dryer after the imaging of part (a) of FIG. 17. Part (c) of FIG. 17 is an SEM image of the hair c5 imaged at the timing immediately after the fine water particle application step according to the present embodiment was executed for 10 minutes after the imaging of part (b) of FIG. 17. As can be seen from FIG. 17, before and after the application of the fine particle ions, the cuticles were uplifted largely. In addition, before and after the application of the fine particle ions, the state of the cuticles showed little change. Thus, it can be said that even when fine particle ions are applied to hair using a fine particle ion dryer, an effect of improving the state of the cuticles of the hair is not recognized. This is considered to be because, since the fine particle ions applied to the hair using the fine particle ion dryer are charged, the fine particle ions are attracted to the hair surface by static electricity or the like, before entering a water passage (CMC) existing between the cuticles of the hair, and do not permeate into the hair. Further, as can be seen from comparison between parts (b) and (c) of FIG. 17, it can be seen that by executing the fine water particle application step according to the present embodiment, after the application of the fine particle ions, to apply the fine water particles, the uplift of the cuticles on the hair surface was slightly reduced. This also shows that the uplift of cuticles can be reduced by executing the fine water particle application step according to the present embodiment.

(Example 9 ... Study on application direction of fine water particles)

FIG. 18 is a schematic diagram showing an orientation of cuticles on a hair surface. As shown in FIG. 18, cuticles are formed in a bamboo-shoot shape on a hair surface such that the tip ends of the cuticles face the hair tip side. When a hair is damaged and the cuticles are uplifted, the cuticles are opened toward the hair tip side. Thus, by applying fine water particles to a hair in a direction from the hair root side toward the hair tip side (forward direction), the opened cuticles are closed by the fine water particles or an airflow carrying the fine water particles, and the cuticles are adjusted. In contrast, by applying fine water particles to a hair in a direction from the hair tip side toward the hair root side (reverse direction), the fine water particles can effectively permeate into a CMC between cuticles facing the hair tip side. Thus, in order to adjust cuticles and improve a hair finish, it is preferable to apply fine water particles in the forward direction. In contrast, in a case of using an agent to allow the agent to efficiently permeate into a hair, it is preferable to apply fine water particles in the reverse direction. For example, fine water particles are preferably applied in the reverse direction to promote the permeation of an agent, when the fine water particle application step is executed at a timing after the completion of execution of the agent application step and before the start of execution of the drying step, more preferably, when the fine water particle application step is executed at the timing of the process B in the first embodiment described above, that is, the timing after the completion of execution of the agent application step and before the start of execution of the washing step. According to this, the agent applied to the hair efficiently permeates into the hair along with the fine water particles. Further, for example, fine water particles are preferably applied in the forward direction to adjust cuticles, when the fine water particle application step is executed at the timings of the processes C, D, E in the first embodiment described above, or when the fine water particle application step is executed at the timings shown in parts (b) and (c) of FIG. 10 in the second embodiment, that is, when the fine water particle application step is executed simultaneously with execution of the drying step or after the completion of execution of the drying step. According to this, it is possible to reduce damage to a hair or repair the damaged hair by allowing fine water particles to permeate into the hair, and it is possible to adjust cuticles opened toward the hand tip side. However, in each example, the application direction of fine water particles is not necessarily limited.

(Third Embodiment)

In a third embodiment, a hair care method including a plurality of agent application steps will be described.

(Example 10 ... Treatment operation including plurality of agent application steps)

Seven bundles of hair samples A6, B6, C6, D6, E6, F6, N6 (each sample is about 30 cm in length, and 15 g in weight) were prepared. Then, on each sample, a treatment operation was performed by executing the steps according to the following numerical order:

1) Washing of the sample with a shampoo
2) Rinsing of the sample with lukewarm water, and subsequent towel drying of the sample
3) Application of a first agent (an agent used: the first agent (misty treatment agent containing a nutrient component such as keratin and not containing a silicone component)) to the sample (a first agent application step)
4) Application of a second agent (an agent used: the second agent (liquid treatment agent containing a nutrient component such as keratin, and not containing a silicone component or containing a small amount of silicone component (less silicone component))) to the sample (a second agent application step)
5) Application of a third agent (an agent used: a liquid treatment agent containing a nutrient component such as keratin and a silicone component) to the sample (a third agent application step)
6) Kneading of the agents into the sample using the balls of fingers (a rubbing step)
7) Rinsing of the sample with lukewarm water, and subsequent towel drying of the sample (a first washing step)
8) Application of a fourth agent (an agent used: a treatment agent containing a nutrient component such as keratin and a silicone component) to the sample (a fourth agent application step)
9) Rinsing of the sample with lukewarm water, and subsequent towel drying of the sample (a second washing step)

10) Drying with a hair dryer (a drying step)
11) Setting of the hair in a predetermined shape (a finishing step)

Further, on the sample A6, the fine water particle application step was executed after the above procedure 2) and before the above procedure 3) (that is, before the start of execution of the first agent application step). On the sample B6, the fine water particle application step was executed after the above procedure 3) and before the above procedure 4) (that is, after the completion of execution of the first agent application step and before the start of execution of the second agent application step). Moreover, on the sample C6, the fine water particle application step was executed after the above procedure 4) and before the above procedure 5) (that is, after the completion of execution of the second agent application step and before the start of execution of the third agent application step). On the sample D6, the fine water particle application step was executed after the above procedure 6) and before the above procedure 7) (that is, after the completion of execution of the third agent application step and before the start of execution of the fourth agent application step). Further, on the sample E6, the fine water particle application step was executed after the above procedure 10) (that is, after the completion of execution of the drying step and before the start of execution of the finishing step). On the sample F6, the fine water particle application step was executed after the above procedure 11) (that is, after the completion of execution of the finishing step). The conditions of the fine water particle application steps executed on the samples were the same, and an application time period of the fine water particles was 150 seconds. Further, on the sample N6, the procedures 1) to 11) were sequentially executed while the fine water particle application step was not executed. FIG. 21 is a diagram showing the execution order of each step in the treatment operation while showing each execution timing of the fine water particle application step executed in this example. Respective timings A6, B6, C6, D6, E6, F6 shown in FIG. 21 are timings at which the respective fine water particle application steps were executed on the samples whose respective numbers corresponding to the respective numbers representing the respective timings.

For each sample on which all the procedures were executed, the states of the hair-root-side portion and the hair tip portion were confirmed. Here, in the conventional treatment operation (that is, the treatment operation for the sample N6) in which the procedures 1) to 11) were executed while the fine water particle application step was not executed, the hair-root-side portion became softer than before the operation, but the hair tip portion became stiff. In contrast, for the samples A6, B6, C6, D6, E6 on each of which the fine water particle application step was executed at the predetermined timing, the following effects were observed:

- The sample A6: the hair-root-side portion fitted well with the hand when the hand was run through the sample.
- The sample B6: prevention or reduction of causing the hair tip portion to be stiff was able to be made.
- The sample C6: the hair-root-side portion fitted well with the hand when the hand was run through the sample, and prevention or reduction of causing the hair tip portion to be stiff was able to be made.
- The sample D6: the hair tip portion fitted well with the hand when the hand was run through the sample.
- The sample E6: the hair tip portion fitted well with the hand when the hand was run through the sample, and prevention or reduction of causing the hair tip portion to be stiff was able to be made.

As described above, in any case where the fine water particles were applied at any of the timings, some effects were able to be obtained.

In particular, there was an observed tendency for making a difference in the portion where the effects were exhibited between a case in which the fine water particles were applied before the application of the treatment agent containing a large amount of silicone component (hereinafter referred to as a silicone-component-containing treatment agent) (that is, before the start of the execution of the third agent application step), and a case in which the fine water particles were applied after the application of the silicone-component-containing treatment agent (that is, after the completion of the execution of the third agent application step). Specifically, in the samples (that is, the samples A6, B6, C6) on each of which the fine water particle application step was executed before the application of the silicone-component-containing treatment agent, a moisture replenishment effect to the hair-root-side portion was obtained, and the hair-root-side portion fitted well with the hand. In the samples (that is, the samples D6, E6) on each of which the fine water particle application step was executed after the application of the silicone-component-containing treatment agent, a moisture replenishment effect to the hair tip portion was obtained, and the hair tip portion fitted well with the hand. This is considered to be due to a difference in damage originally given to hair. In a portion of hair in a state of being less damaged and in a state where moisture can be retained even without an effect brought by an agent (for example, a hair-root-side portion), the moisture remains in the hair even when fine water particles are applied before application of the silicone-component-containing treatment agent, and thus an effect brought by applying the fine water particles can be obtained. In contrast, in a portion of hair in a state of being highly damaged originally (for example, a hair tip portion), the hair itself lacks the ability to retain moisture. Thus, the moisture is less likely to remain in the hair in a state before application of the silicone-component-containing treatment agent, and the effect brought by applying fine water particles is less likely to be exhibited. In this case, the fine water particles are applied after the application of the silicone-component-containing treatment agent, and the components contained in the treatment agent are utilized, whereby the moisture is likely to remain in the hair and the effect brought by applying the fine water particles can be further obtained.

Next, a flexural rigidity reduction rate was obtained for each sample. Here, the flexural rigidity reduction rate is a reduction rate of a flexural rigidity value G2 after the treatment operation (after the completion of execution of the finishing step) with respect to a flexural rigidity value G1 in an initial state (before the treatment operation), and can be obtained by the following formula.

$$1-G2/G1$$

A larger value of the flexural rigidity reduction rate obtained from the above formula in the positive direction represents that the hair after the treatment operation is softer than the hair in the initial state. A larger value of the flexural rigidity reduction rate in the negative direction represents that the hair after the treatment operation is stiffer than the hair in the initial state. Note that in obtaining the flexural rigidity reduction rate, 30 hairs were randomly extracted from each sample, and for each of the extracted 30 hairs, the flexural rigidity value G1 in the initial state was measured first. Then, the hairs in the top 20% each having the larger flexural rigidity value G1 were further extracted, and for each of the extracted hairs, the flexural rigidity value G2 after the treatment operation was measured. By using the flexural rigidity values G1 and G2 thus obtained, the flexural rigidity reduction rate was obtained. That is, the obtained flexural rigidity reduction rate is a value for the hair in the top 20% of the flexural rigidity value G1 in the initial state. Further, the method of measuring the flexural rigidity values G1, G2 is as described above, and the flexural rigidity reduction rate was obtained for each of the hair tip portion and the hair-root-side portion of each sample.

FIG. 19 is a graph comparing the flexural rigidity reduction rates obtained for the samples A6, B6, C6, D6, E6, and N6. The horizontal axis of this graph represents the types of samples. In each of respective columns corresponding to the samples, graphs representing the flexural rigidity reduction rates of the corresponding sample are shown. Further, in the two graphs shown in each of the respective columns corresponding to the samples, a graph E on the left hand represents the flexural rigidity reduction rate of the hair-root-side portion, and a graph F on the right hand represents the flexural rigidity reduction rate of the hair tip portion. As can be seen from FIG. 19, in the sample N6 subjected to the conventional treatment operation in which the fine water particle application step was not executed, the hair tip portion was very stiff after the treatment operation. In contrast, in each of the sample B6, the sample C6, and the sample E6, the flexural rigidity reduction rate of the hair tip portion had a positive value, and it can be seen that the hair tip became softer after the treatment operation. Note that hair (for example, a hair-root-side portion) without damage (or with low damage) has a high ability to retain moisture. Thus, it is considered that, even when fine water particles are applied to the hair without damage (or with low damage) before agent application or before application of the agent having a function of forming a film on the hair surface with silicone or the like (for example, the third agent and the fourth agent) among agents, the fine water particles are likely to enter the hair and the effect brought by applying the fine water particles is easily obtained. In contrast, hair having high damage (for example, a hair tip portion) has a low ability to retain moisture. Thus, it is considered that, in a state where the moisture retention ability of hair is enhanced by an agent, for example, when fine water particles are applied after the third agent application or after the fourth agent application, the fine water particles are likely to be retained in the hair and the effect brought by applying the fine water particles is easily obtained.

Next, a hysteresis change range was obtained for each sample. The "hysteresis change range" described herein is an index representing a change amount of magnitude of a difference between a flexural rigidity value measured when hair is bent and a flexural rigidity value measured when the hair is bent back, when the flexural rigidity values of each sample are measured. In this example, in obtaining the hysteresis change range, first, for each of 30 hairs included in each sample, a difference $\Delta\alpha$ (= $\alpha p$ - $\alpha n$) was obtained, which is a difference between a flexural stress $\alpha p$ measured when the curvature K = 1 in the process of bending the hair until the curvature K was shifted from 0 to +2.5 ($cm^{-1}$) and a flexural stress $\alpha n$ measured when the curvature K = 1 in the process of bending back the hair until the curvature K was shifted from + 2.5 to 0 ($cm^{-1}$). Further, for each of the 30 hairs included in each sample, a difference $\Delta\beta$ (= $\beta p$ - $\beta n$) was obtained, which is a difference between a flexural stress $\beta p$ measured when the curvature K = -1 in the process of bending the hair until the curvature K was shifted from 0 to -2.5 ($cm^{-1}$) and a flexural stress $\beta n$ measured when the curvature K = -1 in the process of bending back the hair until the curvature K was shifted from -2.5 to 0 (cm-1). Then, an average value S (= $(\Delta\alpha + \Delta\beta)/2$) of the obtained differences $\Delta\alpha$ and $\Delta\beta$ was calculated as the hysteresis of each hair. Further, for the average value S (hysteresis) calculated for each of the 30 hairs included in each sample, a difference was calculated by subtracting the value S before the process from the value S after the process, and a change amount (difference between the values S) of each of the 30 hairs was calculated. Then, an average value of the change amounts was used as the hysteresis change range. A larger hysteresis change range represents that the flexural rigidity value at the time of bending back the hair is lower than the flexural rigidity value at the time of bending the hair. That is, it is considered that, as the hysteresis change range becomes larger, the hair becomes a state where a force for the hair to try to return is weaker and the hair fits well with the hand, when the hair is touched with the hand (a state where the hair has high flexibility, and the hair shape is likely to change in accordance with the degree of force when the hair is touched with the hand). Thus, the magnitude of the hysteresis change range represents magnitude of a degree to which the hair is felt as being soft when the hair is touched.

FIG. 20 is a graph comparing the hysteresis change ranges obtained for the samples A6, B6, C6, D6, E6, and N6. The horizontal axis of this graph represents the types of samples. In each of respective columns corresponding to the samples, graphs representing the hysteresis change ranges of the corresponding sample are shown. Further, in the two graphs shown in each of the respective columns corresponding to the samples, a graph G on the left hand represents the hysteresis change range of the hair-root-side portion, and a graph H on the right hand represents the hysteresis change range of the hair tip portion.

As shown in FIG. 20, in each of the sample D6 and the sample E6, the hysteresis change range of the hair tip portion was large. From this result, it was confirmed that by applying fine water particles after applying a treatment agent containing a silicon component, the softness of a hair tip portion can be further improved. Further, in the sample C6, the hysteresis change range of the hair-root-side portion was large. This is considered to be because, since the application of the treatment agent was performed twice before the fine water particles were applied, the state of the hair root side was further improved and moisture was able to be sufficiently absorbed by the subsequent application of the fine water particles.

FIG. 22 is a diagram showing an example of properties of each of the agents (the first agent, the second agent, the third agent, and the fourth agent) used in a corresponding one of the agent application steps. According to FIG. 22, the first agent is a misty agent. The second agent is a mild-texture creamy agent containing no silicone component or containing a small amount of silicone component. The third agent is a hard-texture creamy agent containing a larger amount of silicone component. The fourth agent is a smooth-texture creamy agent containing a larger amount of silicone component and a larger amount of oil.

Further, in the present embodiment, the fine water particle application step can be executed, in accordance with a state or an intended finish of hair, at least at one timing of after the completion of execution of any of the agent application steps, after the completion of execution of the drying step, or after the completion of execution of the finishing step.

In the case of the sample B6 on which the fine water particle application step was executed at the timing after the completion of execution of the first agent application step (the timing B6 in FIG. 21), the effect of the first agent can be enhanced. When the fine water particle application step is executed at the timing after the completion of execution of the second agent application step (the timing C6 in FIG. 21), the effect of the second agent can be enhanced. Moreover, when the fine water particle application step is executed at the timing after the completion of execution of the third agent application step (the timing D6 in FIG. 21), the effect of the third agent can be enhanced. When the fine water particle application step is executed at the timing after the completion of execution of the fourth agent application step, the effect of the fourth agent can be enhanced. Further, when the fine water particle application step is executed at the timing after the completion of execution of the drying step (the timing E6 in FIG. 21), or the timing after the completion of execution of the finishing step (the timing F6 in FIG. 21), a finish can be adjusted while the effect of the fourth agent can be enhanced.

FIG. 23 is a table showing an effect on hair, a finish, and a suited hair type, after the treatment operation, when the fine water particle application step is executed at each of the timings C6, D6, E6, F6 in FIG. 21, the suited hair type being each hair type suited for being subjected to application of fine water particles at a corresponding one of the timings C6, D6, E6, F6 in FIG. 21. As shown in FIG. 23, when the fine water particle application step is executed at the timing C6, the softness of hair is improved, and the hair can be finished with an airy finish. In addition, with respect to hair with low damage, fine water particles are preferably applied to the hair at the timing C6. At the timing C6, only the treatment agent containing no silicone component or containing a small amount of silicone component is applied to hair. In the case of hair with low damage, the hair is sufficiently repaired at this stage. Thus, by applying fine water particles to the hair, it is possible to retain moisture in the hair, whereby the effect of the second agent, which is a mild-texture creamy agent, is enhanced. As a result, the hair can be softened.

In addition, when the fine water particle application step is executed at the timing D6, the effect of the third agent, which is a hard-texture creamy agent, is enhanced, and thus hair can be finished with an airy finish while the resilience of the hair, particularly the resilience of the hair-root-side portion, is enhanced. Therefore, with respect to the hair type without resilience, fine water particles are preferably applied at the timing D6.

In addition, when the fine water particle application step is executed at the timing E6 or the timing F6, the effect of the fourth agent, which is a smooth-texture creamy agent, is enhanced, and thus the softness of hair is improved. Further, the timing E6 or the timing F6 is a timing after all the agents are applied, and thus hair is sufficiently repaired and protected when fine water particles are applied even when the hair is highly damaged hair type. Therefore, with respect to the highly damaged hair type, fine water particles are preferably applied at the timing E6 or the timing F6. In addition, by applying fine water particles to dried hair at the timing E6, it is possible to add a volume to the entire hair to finish the hair with a fluffy and soft texture. Further, by applying fine water particles to finished hair at the timing F6, that is, by allowing moisture to be contained in the hair at the final step of the operation, the hair can be finished with a heavy, tidy, and moist texture.

FIG. 24 is a diagram showing relationship between the hysteresis change range and the flexural rigidity reduction rate measured for each of the samples C6, D6, F6, N6. The horizontal axis in FIG. 24 represents the hysteresis change range. An increase from zero toward the positive direction represents that hair is less likely to bent back (that is, the hair further fits well with the hand (the hair fits easily with the hand)). An increase from zero toward the negative direction represents that hair is more likely to bend back (that is, the hair fits less well with the hand (the hair fits less easily with the hand). The vertical axis in FIG. 24 represents the flexural rigidity reduction rate. An increase from zero toward the positive direction represents that hair is more flexible, and an increase from zero toward the negative direction represents that hair is less flexible. In addition, each circle point in FIG. 24 represents the relationship between the hysteresis change range and the flexural rigidity reduction rate of the hair-root-side portion. Each square point in FIG. 24 represents the relationship between the hysteresis change range and the flexural rigidity reduction rate of the hair tip portion. Further, in the vicinities of the points in FIG. 24, the respective numbers of the respective samples corresponding to the points are shown.

As shown in FIG. 24, in the sample N6 on which the fine water particle application step was not executed, it can be seen that the hair tip portion was less flexible and the hair-root-side portion was flexible. Further, in the sample C6, it can be seen that the hair-root-side portion was less likely to bend back (fitted easily). Typically, it can be said that the hair that is flexible and that is less likely to bend back (fits easily) is softer hair. Thus, by executing the fine water particle application step at the timing after the completion of execution of the second agent application step and before the start of execution of the third agent application step, it is possible to finish hair with soft hair. Further, in the sample D6, it can be seen that both the hair tip portion and the hair-root-side portion were less flexible. In particular, the hair-root-side portion of the sample D6 was less flexible and was likely to bend back. It can be said that the hair that is less flexible and that is likely to bend back when the hair bends is resilient hair. Thus, by executing the fine water particle application step at the timing after the completion of execution of the third agent application step and before the start of execution of the fourth agent application step, it is possible to finish hair with the hair-root-side portion being resilient. Further, in the sample F6, the hair tip portion was less likely to bend back. Thus, by executing the fine water particle application step at the timing F6 after the completion of execution of the finishing step, it is possible to finish hair with the hair tip portion being soft. Further, by applying fine water particles to hair after the completion of execution of the finishing step, it is possible to add a moist feeling to the hair, and it is thus possible to finish the hair with heavy and tidy hair.

As described above, in a treatment operation, by executing the fine water particle application step, in accordance with the state of hair (low damage, high damage, presence or absence of resilience, or the like) and an intended finish (airy, heavy, fluffy, tidy, or the like), at least at any one of the timing after the completion of execution of any of the plurality of agent application steps, the timing after the completion of execution of the drying step, or the timing after the completion of execution of the finishing step, it is possible to perform an appropriate treatment operation, and it is possible to further enhance the effect of a treatment agent.

(Fourth Embodiment)

In a fourth embodiment, a hair care method capable of reducing damage to hair in a perming operation to be performed on the hair will be described.

In a perming operation, a first agent (first-part agent) and a second agent (second-part agent), which are two types of liquid agents, are typically used. The first agent is an agent having a function of freely changing a hair shape by cutting internal tissue of the hair, specifically, a cystine bond in the hair. The second agent is an agent having a function of recombining the internal tissue of the hair cut by the application of the first agent to cause the hair to be conformed to an intended shape and to fix the hair to the intended shape. Thus, in the perming operation, the first agent is initially applied to hair, and then the second agent is applied to the hair.

FIG. 25A shows each step in a conventionally-performed perming operation using two liquids (a first agent and a second agent). As shown in FIG. 25A, in the conventional perming operation using the two liquids, a rod-winding step (winding step), a first agent application step, a first leaving step, a washing step (first washing step), a second agent application step, a second leaving step, a washing step (second washing step), and a drying step are executed in this order. In the rod-winding step, hair is wound around a rod, and the hair is bent into a desired winding shape. Next, in the first agent application step, the first agent is applied to the hair, and then, in the first leaving step, the hair is left to stand for a first predetermined time period. By executing the first leaving step, the first agent permeates into the hair, the internal tissue in the hair is cut, and the hair shape can be freely changed. After the completion of the execution of the first leaving step, the hair is washed in the washing step (first washing step). Then, in the second agent application step, the second agent is applied to the hair. Thereafter, in the second leaving step, the hair is left to stand for a second predetermined time period. By executing the second leaving step, the second agent permeates into the hair, the cut internal tissue in the hair is recombined, and the hair is conformed to an intended shape and is fixed to a conformed shape. Each of the first predetermined time period in the first leaving step and the second predetermined time period in the second leaving step is typically about 15 minutes. After the completion of the execution of the second leaving step, the hair is washed in the washing step (second washing step), and then the hair is dried in the drying step.

FIG. 25B shows an example of an execution order of each step in a perming operation, using the two liquids (the first agent and the second agent), according to the present embodiment. As shown in FIG. 25B, in the perming operation, using the two liquids (the first agent and the second agent), according to the present embodiment, the fine water particle application step is executed after the completion of execution of the first leaving step and before the start of execution of the second agent application step (specifically, after the completion of execution of the washing step (first washing step) after the first leaving step and before the start of execution of the second agent application step) shown in FIG. 25A. The order of the other steps is the same as the order of the steps shown in FIG. 25A.

According to the present embodiment, fine water particles are applied to hair wet with the first agent applied by execution of the first agent application step or hair wet by execution of the washing step after the first agent application step. The applied fine water particles enter the hair and adhere to the internal tissue of the hair cut by the first agent. When the fine water particles adhere to the internal tissue of the cut hair, the structure is allowed to be easily movable. As a result of allowing the internal tissue of the hair to be easily movable, the hair shape is easily conformed to an intended winding shape. Thus, the time period required for conforming the hair to the intended winding shape after the application of the second agent is shortened. As a result, the second predetermined time period in the second leaving step can be shortened. For example, as shown in FIG. 25B, although the first predetermined time period in the first leaving step is about 15 minutes, the second predetermined time period in the second leaving step can be shortened to about 5 minutes. At this time, when the time period for applying fine water particles in the fine water particle application step is 10 minutes, it is possible to perform the perming operation, using the two liquids, that includes the fine water particle application step, without extending the operation time period, as compared with the perming operation shown in FIG. 25A. In addition, by applying fine water particles to hair during the perming operation, the effect of the perming agent can be enhanced. Further, by shortening the time period during which hair is exposed to the second agent (the second predetermined time period), the magnitude of damage given to the hair by the second agent can be reduced.

(Example 11 ... Comparison of waving efficiencies)

Four bundles of hair samples A7, B7, C7, N7 (each sample is about 50 cm in length, and 25 g in weight) were prepared, and on each sample, a perming operation was performed using two liquids (a first agent and a second agent). Here, a first agent has a function of cutting the internal tissue of hair, and a second agent has a function of binding the internal tissue of the hair cut by the first agent. In addition, the sample A7 was subjected to the perming operation through a process F1, the sample B7 was subjected to the perming operation through a process F2, the sample C7 was subjected to the perming operation through a process F3, and the sample N7 was subjected to the perming operation through a conventional process. These processes will be described later.

FIG. 26 is a diagram showing steps in each of the conventional process, the process F1, the process F2, and the process F3. As shown in FIG. 26, the conventional process is a process of executing the rod-winding step, the first agent application step, the first leaving step, the first washing step, the second agent application step, the second leaving step, the second washing step, and the drying step in this order. That is, the conventional process does not include the fine water particle application step. The process F1 is a process of executing the rod-winding step, the first agent application step, the first leaving step + the fine water particle application step, the first washing step, the second agent application step, the second leaving step, the second washing step, and the drying step in this order. According to the process F1, the fine water particle application step is executed during execution of the first leaving step. Specifically, the execution time period of the first leaving step is 15 minutes, and the fine water particle application step is executed simultaneously with the first leaving step during a period of 10 minutes from the start of execution of the first leaving step. Thus, after the completion of execution of the fine water particle application step, only the first leaving step is executed for five minutes. The process F2 is a process of executing the rod-winding step, the first agent application step, the first leaving step, the first washing step, the second agent application step, the second leaving step + the fine water particle application step, the second washing step, and the drying step in this order. According to the process F2, the fine water particle application step is executed during execution of the second leaving step. Specifically, the execution time period of the second leaving step is 15 minutes, and the fine water particle application step is executed simultaneously with the second leaving step during a period of 10 minutes from the start of execution of the second leaving step. Thus, after the completion of execution of the fine water particle application step, only the second leaving step is executed for five minutes. The process F3 is a process of executing the rod-winding step, the first agent application step, the first leaving step, the first washing step, the fine water particle application step, the second agent application step, the second leaving step, the second washing step, and the drying step in this order. According to the process F3, the fine water particle application step is executed after the completion of execution of the first washing step and before the start of execution of the second agent application step.

In the rod-winding step, the hair sample was wound around a rod, and the hair was bent into a predetermined winding shape. In the first agent application step, the first agent was uniformly applied to the rod-wound sample. In the first leaving step, the sample to which the first agent had been applied was left to stand for 15 minutes. In the first washing step, the first agent was washed off from the sample by washing the sample with water by shower while rubbing the sample with fingers. In the second agent application step, the second agent was uniformly applied to the sample. In the second leaving step, the sample to which the second agent had been applied was left to stand for the predetermined time period (second predetermined time period). Here, the second predetermined time period in the second leaving step was 15 minutes in the conventional process, the process F1, and the process F2, and was 5 minutes in the process F3. In the second washing step, the second agent was washed off from the sample by washing the sample with water by shower while rubbing the sample with fingers. In the drying step, moisture on the sample surface was removed by blowing warm air onto the wet sample for about two minutes using a hair dryer. In the fine water particle application step, fine water particles (flow rate: 0.07 $m^2$/min.) at a temperature of about 35°C were applied to the sample for 10 minutes, using the fine water particle release device 1 shown in FIG. 1. Note that after the completion of execution of the drying step, the rod was removed from the sample.

FIG. 27 is a diagram comparing respective waving efficiencies of the samples A7, B7, C7, N7 subjected to the perming operation through the respective processes. Here, the waving efficiency is a percentage obtained by dividing the diameter of the rod used in the rod-winding step by the diameter of the wave formed in the sample after the operation. An increase in the waving efficiency represents that a perming agent has a larger effect. As shown in FIG. 27, each of the waving efficiencies of the respective samples A7, B7, C7 subjected to the process F1, the process F2, and the process F3 was higher than the waving efficiency of the sample N7 subjected to the conventional process. The reason why each of the waving efficiencies of the samples A7, B7, C7 was high can be considered to be because the effect of the perming agent was enhanced by applying the fine water particles to the hair to cause damage to the hair to be reduced.

Further, among the respective samples A7, B7, C7 subjected to the perming operation through the process F1, the process F2, and the process F3, the sample C7 had the highest waving efficiency. The reason why the sample C7 had the highest waving efficiency is considered to be because the second predetermined time period in the second leaving step of the process F3 was five minutes that was the shortest and damage to the hair due to the exposure of the hair to the second agent was the least, thus allowing the effect of the perming agent to be enhanced. That is, by performing a perming operation through the process F3 to allow the second predetermined time period (standing time period) in the second leaving step to be shortened, the perming operation with high waving efficiency can be sufficiently performed while damage to hair can be reduced, and an operation time period for the perming operation including the fine water particle application step can be shortened. Incidentally, the reason why the standing time period in the second leaving step (the second predetermined time period) can be shortened in the process F3 is considered to be because, as described above, by applying fine water particles to the sample after the application of the first agent and before the application of the second agent, the internal tissue cut in the hair became easily movable to allow the hair to easily conform to an intended shape. Further, in the process F1, similarly to the process F3, fine water particles were applied to the sample after the application of the first agent and before the application of the second agent. The difference between the process F1 and the process F3 is that the fine water particle application step was executed simultaneously with the first leaving step after the application of the first agent in the process F1 while the fine water particle application step was executed after the completion of the execution of the first leaving step in the process F3. In the process F1 in which fine water particles are applied during execution of the first leaving step, it is considered that, since most of the fine water particles are used for causing the first agent to efficiently permeate into the hair, the contribution of the fine water particles to the effect of allowing internal tissue in the hair to be easily movable is reduced. Thus, it is considered that the second standing time period cannot be adequately shortened in the process F1. In contrast, in the process F3 in which fine water particles are applied after the completion of execution of the first leaving step, the fine water particles are applied after the first agent permeates into the hair by execution of the first leaving step, and thus, most of the applied fine water particles are used for allowing internal tissue in the hair cut by the first agent to be easily movable. Therefore, according to the process F3, it is considered that, by the application of fine water particles, internal tissue of the hair is allowed to be sufficiently and easily movable, and thus it is possible to sufficiently shorten the standing time period after the second agent application (the execution time period of the second leaving step).

Note that in Example 11, an example has been described in which the first washing step is executed after the completion of execution of the first leaving step, but the first washing step may be omitted. In this case, the fine water particle application step is executed after the completion of execution of the first leaving step and before the start of execution of the second agent application step. Further, in addition to the timing after the completion of execution of the first leaving step and before the start of execution of the second agent application step, the fine water particle application step may be executed at a timing before the start of execution of the first agent application step. According to this, fine water particles having already permeated into hair at the time of execution of the first agent application step promote permeation of the first agent into the hair, thereby promoting cutting of internal tissue of the hair. Thus, it is possible to shorten the standing time period in the first leaving step (the first predetermined time period). In addition, by shortening the time period during which hair is exposed to the first agent, it is possible to further reduce damage to the hair.

(Fifth Embodiment)

In a fifth embodiment, a hair care method capable of reducing unruly condition of hair in a bleaching operation will be described.

FIG. 28 is a diagram showing each step in a bleaching operation according to the present embodiment. As shown in FIG. 28, the bleaching operation according to the present embodiment is performed by executing an agent (bleaching agent) application step, a leaving step, a washing step, a fine water particle application step, and a drying step in this order. The fine water particle application step is executed after the completion of execution of the washing step and before the start of execution of the drying step. Thus, by executing the washing step, fine water particles are applied to wet hair. By applying the fine water particles to the wet hair, it is possible to reduce unruly condition of the hair after the bleaching operation.

(Example 12 ... Confirmation of effect of reducing unruly condition of hair after bleaching operation)

Three bundles of hair samples A8, B8, C8 (each sample is about 50 cm in length, and 25 g in weight) collected from a single person were prepared, and on each sample, a bleaching operation was performed using a commercially available bleaching agent. Here, on the sample A8, the bleaching operation was performed by a process according to the order of the steps shown in FIG. 28 (hereinafter referred to as the present example process). Further, on the sample B8, a bleaching operation was performed by a process according to the order of the steps shown in FIG. 29A (hereinafter referred to as a first comparative process). On the sample C8, a bleaching operation was performed by a process according to the order of the steps shown in FIG. 29B (hereinafter referred to as a second comparative process). The first comparative process shown in FIG. 29A is a process of executing a first fine water particle application step, the agent (bleaching agent) application step, the leaving step, the washing step, the drying step, and a second fine water particle application step in this order. Further, the second comparative process shown in FIG. 29B is a process of executing the agent (bleaching agent) application step, the leaving step, the washing step, the drying step, and the fine water particle application step in this order.

Further, in the present example process, the first comparative process, and the second comparative process, in the agent application step, the commercially available bleaching agent was uniformly applied on the sample by brushing. In the fine water particle application step, the first fine water particle application step, and the second fine water particle application step, fine water particles (flow rate: 0.07 $m^2$/min.) at a temperature of about 35°C were applied to the sample, using the fine water particle release device 1 shown in FIG. 1. Here, in the fine water particle application step in each of the present example process and the second comparative process, a time period for applying fine water particles to the sample was 10 minutes. In each of the first fine water particle application step and the second fine water particle application step in the second comparative process, a time period for applying fine water particles to the sample was 5 minutes. In the leaving step, the hair was left to stand for 20 minutes after the application of the agent (bleaching agent). In the washing step, after the completion of the execution of the leaving step, the agent was removed from the sample by washing the sample by shower of lukewarm water while rubbing the sample with the fingers. In the drying step, moisture on the sample surface was removed by blowing warm air onto the wet sample for about two minutes using a hair dryer.

FIG. 30 is a photograph in which photographing was made for the samples A8, B8, C8, subjected to the bleaching operations through the processes (the present example process, the first comparative process, and the second comparative process). In FIG. 30, the sample on the left hand is the sample B8 subjected to the bleaching operation through the first comparative process, the sample on the right hand is the sample C8 subjected to the bleaching operation through the second comparative process, and the sample at the center is the sample A8 subjected to the bleaching operation through the present example process. As shown in FIG. 30, the sample A8 subjected to the bleaching operation through the present example process shows the hair that is straight as compared with those of the other samples B8, C8. Although these samples A8, B8, C8 were collected from the single person, the unruly condition of the hair was eliminated only in the sample A8. Further, in the present example process performed on the sample A8, fine water particles were applied to the wet hair before drying. Thus, it was confirmed that, in a bleaching operation, by executing the fine water particle application step after the completion of execution of the washing step and before the start of execution of the drying step, the unruly condition of hair can be reduced.

The reason why the unruly condition of hair can be reduced by applying fine water particles to the wet hair after the completion of execution of the washing step and before the start of execution of the drying step in a bleaching operation will be considered. When a bleaching agent is applied to hair and the bleaching agent permeates into the hair, the internal tissue of the hair is cut by the bleaching agent. Thereafter, when fine water particles enter the hair, the internal tissue in the hair is allowed to be easily movable in the hair. As a result, the unruly condition of the hair is eliminated. In addition, when the hair is wet, there is little resistance that prevents the tissue in the hair from moving. Thus, it is considered that by applying fine water particles to hair at a timing when the hair is wet after agent application, that is, a timing after the washing step and before the drying step to allow cut tissue inside the hair to be sufficiently movable, the unruly condition is reduced.

(Sixth Embodiment)

In a sixth embodiment, a case will be described in which a fine water particle application step is executed during an operation involving heat treatment.

Each of the operations of hair straightening, digital perming, and hair setting includes a heat treatment step. By applying heat to hair in this heat treatment step, it is possible to arrange the hair to a desired shape. In addition, when the heat treatment step is executed, moisture in hair is removed, and thus the hair tends to dry and becomes stiff.

FIG. 31 is a diagram showing examples of operations involving heat treatment. Part (a) of FIG. 31 shows each step in a hair straightening operation, part (b) of FIG. 31 shows each step in a digital perming operation, and part (c) of FIG. 31 shows each step in a hair setting operation. An ironing step in each of parts (a) and (c) of FIG. 31 is the heat treatment step, and a warming step in part (b) of FIG. 31 is the heat treatment step.

In the present embodiment, the fine water particle application step is executed before the heat treatment step. For example, in the hair straightening operation shown in part (a) of FIG. 31, the fine water particle application step is executed at a timing after the completion of execution of a drying step and before the start of execution of the ironing step (heat treatment step) (at the timing "a" in part (a) of FIG. 31). In the digital perming operation shown in part (b) of FIG. 31, the fine water particle application step is executed after the completion of execution of a rod-winding step and before the start of execution of the warming step (heat treatment step) (at the timing b in part (b) of FIG. 31). In the hair setting process shown in part (c) of FIG. 31, the fine water particle application step is executed after the completion of execution of the dry (drying) step and before the start of execution of the ironing step (heat treatment step) (at the timing c in part (c) of FIG. 31).

By executing the fine water particle application step before the start of execution of the heat treatment step, moisture is supplied into hair before heat treatment. Then, the moisture supplied into the hair by the fine water particle application step is in a state of being bound to biological tissue such as a protein in the hair, that is, is present as bound water, and thus is hardly removed from the hair in the subsequent heat treatment step. That is, even when the heat treatment step is executed, the hair is not dried. Thus, sufficient moisture is contained in the hair at the time of finishing, and therefore it is possible to obtain an effect of allowing the hair to be softened. Further, by replenishing moisture in the hair before the heat treatment step, a tissue structure in the hair is allowed to be easily movable. Thus, the hair shape is more easily arranged in the subsequent heat treatment step.

Additionally, the fine water particle application step may be executed after the completion of execution of the heat treatment step. That is, the fine water particle application step may include a first fine water particle application step executed before the start of execution of the heat treatment step, and a second fine water particle application step executed after the completion of execution of the heat treatment step. By executing the fine water particle application step after the completion of execution of the heat treatment step as well as before the start of execution of the heat treatment step, it is possible to further soften hair to further improve the texture at the time of finishing.

(Example 13 ... Confirmation of effect when fine water particle application step is executed before start of execution of ironing step)

By executing steps in the order of the steps shown in FIG. 32, a sample A9 subjected to a hair straightening operation was prepared. As shown in FIG. 32, the hair straightening operation in this example is performed by executing a shampoo step, a towel drying step, a first agent (first-part agent) application step, a first leaving step, an intermediate water washing step (washing step), a drying step, a fine water particle application step, an ironing step (heat treatment step), a second agent (second-part agent) application step, a second leaving step, a water washing step (washing step), a treatment agent application step, and a finishing step in this order. According to this order of the steps, the fine water particle application step is executed after the completion of execution of the drying step and before the start of execution of the ironing step (heat treatment step).

Note that a hair straightening agent includes a first agent (first-part agent) and a second agent (second-part agent). The first agent has a function of cutting a bond of the internal tissue of hair to allow the hair to be easily movable. Thus, by applying the first agent to hair to allow the first agent to permeate into the hair, it is possible to straighten the hair shape, for example. The second agent has a function of recombining the internal tissue of the hair cut by the first agent. Thus, by applying the second agent to allow the second agent to permeate into hair, the cut internal tissue is bonded, and the hair shape is fixed to a corrected shape.

In addition, samples B9, C9, D9 were prepared on which the hair straightening operation was performed by executing each step shown in FIG. 32, except that on the samples, the respective fine water particle application steps were executed at respective different execution timings. Here, in the preparation of the sample B9, the fine water particle application step was executed after the completion of execution of the ironing step (heat treatment step) and before the start of execution of the second agent application step. In the preparation of the sample C9, the fine water particle application step was executed simultaneously with the first agent application step. In the preparation of the sample D9, the fine water particle application step was executed simultaneously with the second agent application step. In addition, a sample N9 was prepared on which the hair straightening operation was performed in the order of steps in which the fine water particle application step was omitted from the steps in FIG. 32.

For each of the prepared samples A9, B9, C9, D9, N9, the flexural rigidity reduction rate of the hair-root-side portion and the flexural rigidity reduction rate of the hair tip portion were calculated. The method of calculating the flexural rigidity reduction rate is the same as the method of calculating the flexural rigidity reduction rate in Example 10 described above, and thus the description thereof will be omitted.

FIG. 33 is a graph comparing the flexural rigidity reduction rates obtained for each sample. A horizontal axis of this graph represents the types of samples (A9, B9, C9, D9, N9), and in respective columns corresponding to the samples, graphs representing the flexural rigidity reduction rates are shown. In addition, in the two graphs shown in each of the respective columns corresponding to the samples, the graph Ion the left hand represents the flexural rigidity reduction rate of the hair-root-side portion, and the graph J on the right hand represents the flexural rigidity reduction rate of the hair tip portion.

As shown in FIG. 33, in the sample A9, both the flexural rigidity reduction rate of the hair-root-side portion and the flexural rigidity reduction rate of the hair tip portion were large. In the preparation of the sample A9, the fine water particle application step was executed before the start of the execution of the ironing step (heat treatment step). Thus, it was confirmed that by executing the fine water particle application step before the start of execution of the heat treatment step, the flexural rigidity value of hair was reduced to allow the hair to be softened, thereby allowing the hair to be easily arranged.

Although the embodiments of the present invention have been described above, the present invention should not be construed as being limited to the above embodiments. For example, in Example 13, an example has been described in which the fine water particle application step is executed before the ironing step to allow hair to be softened and to be easily arranged. However, the fine water particle application step may be executed at another timing, for example, after the completion of execution of the first agent application step and before the start of execution of the second agent application step. It is considered that by applying fine water particles after application of the first agent, moisture enters a portion, of tissue in hair, that is not bound to soften the tissue, thus obtaining an effect of allowing the tissue to be easily movable. Therefore, by executing the fine water particle application step between the first agent application step and the second agent application step, it is possible to obtain an effect of easily changing a hair shape to a desired shape.

Further, when the hair straightening agent including the first agent and the second agent as described above is used, the fine water particle application step can be executed, for example, after the completion of execution of the second agent application step. It is considered that by supplying moisture into hair by applying fine water particles after application of the second agent, an effect of softening the hair and an effect of reducing strain of tissue occurring when the hair shape is fixed are obtained. Thus, by executing such a fine water particle application step after the completion of execution of the second agent application step, hair becomes softer, and the texture of the hair after the finishing step can be improved.

Further, in the above embodiments, an example in which the fine water particles are applied to hair as the target head part has been described, but fine water particles may be applied to a scalp as the target head part. In this case, a scalp process can be performed through the process A or the process B in the first embodiment. According to this, by allowing fine water particles to permeate into the scalp, it is possible to reduce stimulation caused by an agent liquid, and it is possible to adjust the state of the hair. Further, in the first embodiment, cases of performing the processes A, B, C, D in FIG. 4A have been mainly described. However, as in the process E in FIG. 4A, even when the fine water particle application step is executed simultaneously with execution of the drying step, it is possible to obtain an effect substantially similar to that of the process C. Further, in the second embodiment, a case in which the fine water particle application step is executed after the completion of execution of the drying step has been mainly described. However, even when the fine water particle application step is executed after the completion of execution of the washing step and before the start of execution of the drying step, or simultaneously with execution of the drying step, it is possible to obtain a similar effect. Further, in the second embodiment, the hair care method without application of an agent has been described, but an agent such as a treatment agent may be applied to hair along with application of fine water particles. According to this, it is possible to further moisturize hair, and it is possible to more smoothly treat the hair. Thus, the present invention can be modified without departing from the gist thereof.

## Claims

1. A hair/scalp care method comprising:

   a washing step of washing a target head part that is one or both of hair and a scalp of a human body;
   a drying step of drying the target head part that has been washed in the washing step; and
   being **characterized in that** the method comprises a fine water particle application step of applying fine water particles having a temperature of 40°C or lower and having a size of 50 nanometers or less to the target head part.

2. The hair/scalp care method according to claim 1, further comprising an agent application step of applying an agent to the target head part,
   wherein the washing step is executed after a predetermined time period has passed from completion of execution of the agent application step.

3. The hair/scalp care method according to claim 2,

   wherein the agent application step is executed a plurality of times, and
   wherein the fine water particle application step is executed after completion of execution of one agent application step and before a start of execution of a next agent application step.

4. The hair/scalp care method according to claim 2,

   wherein the target head part is hair,
   wherein the fine water particle application step is executed after completion of execution of the agent application step and before a start of execution of the drying step, and
   wherein in the fine water particle application step, the fine water particles are applied to the hair in a direction from a hair tip side toward a hair root side of the hair.

5. The hair/scalp care method according to claim 1 or 2,

   wherein the target head part is hair,
   wherein the fine water particle application step is executed simultaneously with execution of the drying step or after completion of execution of the drying step, and
   wherein in the fine water particle application step, the fine water particles are applied to the hair in a direction from a hair root side toward a hair tip side of the hair.

6. The hair/scalp care method according to claim 2,

   wherein the target head part is hair,
   wherein the agent is a perming agent including a first agent and a second agent that are liquid,
   wherein the agent application step includes:

      a first agent application step of applying the first agent to the hair; and
      a second agent application step of applying the second agent to the hair after completion of execution of the first agent application step, and
      wherein the fine water particle application step is executed before a start of execution of the first agent application step, or after completion of execution of the first agent application step and before a start of execution of the second agent application step.

7. The hair/scalp care method according to claim 6,

   wherein the first agent has a function of cutting internal tissue of the hair, and
   wherein the second agent has a function of binding internal tissue of the hair cut by the first agent, the method further comprising:

      a first leaving step of leaving the hair to stand for a first predetermined time period immediately after completion of execution of the first agent application step; and
      a second leaving step of leaving the hair to stand for a second predetermined time period immediately after completion of execution of the second agent application step,
      wherein the fine water particle application step is executed after completion of execution of the first leaving step and before a start of execution of the second agent application step.

8. The hair/scalp care method according to claim 2,

   wherein the target head part is hair,
   wherein the agent is a bleaching agent, and
   wherein the fine water particle application step is executed after completion of execution of the washing step and before a start of execution of the drying step.

9. The hair/scalp care method according to claim 2, wherein the target head part is hair, the method further comprising:

   a finishing step of being executed after completion of execution of the drying step and arranging the hair,

wherein the fine water particle application step is executed in a period from before a start of execution of the agent application step to after completion of execution of the finishing step.

**10.** The hair/scalp care method according to claim 1 or 2, wherein the target head part is hair, the method further comprising:
a heat treatment step of heat-treating the hair.

**11.** The hair/scalp care method according to claim 10, wherein the fine water particle application step is executed before a start of execution of the heat treatment step.

**12.** The hair/scalp care method according to claim 1, wherein the fine water particle application step is executed after completion of execution of the washing step or after completion of execution of the drying step.

**13.** The hair/scalp care method according to claim 2, wherein an agent applied in the agent application step includes at least one of a coloring agent, a treatment agent, a perming agent, a bleaching agent, or a hair straightening agent.

**14.** A hair/scalp care device being **characterized in that** it comprises:

a fine water particle generating element (11) configured to be, by a temperature decrease, in an absorption state of absorbing moisture on a surface, and configured to be, by a temperature increase, in a release state of releasing moisture, having been absorbed, as fine water particles having a temperature of 40°C or lower and having a size of 50 nanometers or less;
applying means (12) configured to apply the fine water particles having been released from the fine water particle generating element (11) to a target head part that is one or both of hair and a scalp of a human body;
controlling means (23) configured to control the fine water particle generating element (11) and the applying means (12); and
an operation part (21) configured to be operated for causing the fine water particles to be applied to the target head part between a period before execution of washing of the target head part and a period after execution of drying of the target head part.

**15.** The hair/scalp care device according to claim 14, wherein the operation part (21) is configured to be operated for causing the fine water particles to be applied to the target head part between a period before execution of agent application to the target head part prior to the execution of the washing and a period after execution of drying of the target head part.

**16.** The hair/scalp care device according to claim 14, wherein the controlling means (23) is configured to apply fine water particles having a temperature equal to or higher than a glass-transition temperature of a protein structure of hair to the target head part.

**17.** The hair/scalp care device according to claim 14, wherein the target head part to which fine water particles are to be applied by the applying means (12) includes hair, and the fine water particle generating element (11) is configured to release the fine water particles larger than a single molecule of water to allow the fine water particles to remain in hair and to improve a state of cuticles of hair.

**18.** The hair/scalp care device according to claim 14, wherein the target head part to which fine water particles are to be applied by the applying means (12) includes hair, and the fine water particle generating element (11) is configured to release the fine water particles that are uncharged to allow the fine water particles to permeate into hair and to improve a state of cuticles of hair.

## Patentansprüche

**1.** Verfahren zur Haar-/Kopfhautpflege, umfassend:

einen Waschschritt des Waschens eines Ziel-Kopfteils, der eines oder beides von Haar und einer Kopfhaut eines menschlichen Körpers ist;
einen Trocknungsschritt des Trocknens des Ziel-Kopfteils, der in dem Waschschritt gewaschen wurde; und
**dadurch gekennzeichnet, dass** das Verfahren einen Schritt des Aufbringens feiner Wasserpartikel umfasst, bei dem feine Wasserpartikel, die eine Temperatur von 40 °C oder weniger aufweisen und eine Größe von 50 Nanometern oder weniger aufweisen, auf den Ziel-Kopfteil aufgebracht werden.

**2.** Verfahren zur Haar-/Kopfhautpflege nach Anspruch 1, ferner umfassend einen Mittelaufbringungsschritt des Aufbringens eines Mittels auf den Ziel-Kopfteil, wobei der Waschschritt ausgeführt wird, nachdem ein zuvor festgelegten Zeitraum nach Vollendung des Mittelaufbringungsschrittes verstrichen ist.

**3.** Verfahren zur Haar-/Kopfhautpflege nach Anspruch 2,

wobei der Mittelaufbringungsschritt mehrere Male ausgeführt wird, und
wobei der Schritt des Aufbringens feiner Wasserpartikel nach Vollendung der Ausführung eines einzelnen Mittelaufbringungsschrittes und vor einem Beginn der Ausführung eines nächsten Mittelaufbringungsschrittes ausgeführt wird.

**4.** Verfahren zur Haar-/Kopfhautpflege nach Anspruch 2,

wobei der Ziel-Kopfteil Haar ist,
wobei der Schritt des Aufbringens feiner Wasserpartikel nach Vollendung der Ausführung des Mittelaufbringungsschrittes und vor einem Beginn der Ausführung des Trocknungsschrittes ausgeführt wird, und
wobei in dem Schritt des Aufbringens feiner Wasserpartikel die feinen Wasserpartikel in einer Richtung von einer Haarspitzenseite zu einer Haarwurzelseite des Haares auf das Haar aufgebracht werden.

**5.** Verfahren zur Haar-/Kopfhautpflege nach Anspruch 1 oder 2,

wobei der Ziel-Kopfteil Haar ist,
wobei der Schritt des Aufbringens feiner Wasserpartikel gleichzeitig mit der Ausführung des Trocknungsschrittes oder nach Vollendung der Ausführung des Trocknungsschrittes ausgeführt wird, und
wobei in dem Schritt des Aufbringens feiner Wasserpartikel die feinen Wasserpartikel in einer Richtung von einer Haarwurzelseite zu einer Haarspitzenseite auf das Haar aufgebracht werden.

**6.** Verfahren zur Haar-/Kopfhautpflege nach Anspruch 2,

wobei der Ziel-Kopfteil Haar ist,
wobei das Mittel ein Dauerwellenmittel ist, das ein erstes Mittel und ein zweites Mittel enthält, die flüssig sind,
wobei der Mittelaufbringungsschritt enthält:

einen Schritt des Aufbringens des ersten Mittels zum Aufbringen des ersten Mittels auf das Haar; und
einen Schritt des Aufbringens des zweiten Mittels zum Aufbringen des zweiten Mittels auf das Haar nach Vollendung der Ausführung des Schrittes des Aufbringens des ersten Mittels, und
wobei der Schritt des Aufbringens feiner Wasserpartikel vor einem Beginn der Ausführung des Schrittes des Aufbringens des ersten Mittels oder nach Vollendung der Ausführung des Schrittes des Aufbringens des ersten Mittels und vor einem Beginn der Ausführung des Schrittes des Aufbringens des zweiten Mittels ausgeführt wird.

**7.** Verfahren zur Haar-/Kopfhautpflege nach Anspruch 6,

wobei das erste Mittel eine Funktion hat, inneres Gewebe des Haares zu zertrennen, und
wobei das zweite Mittel eine Funktion hat, durch das erste Mittel zertrenntes inneres Gewebe des Haares zu binden, wobei das Verfahren ferner umfasst:

einen ersten Einwirkungsschritt des Einwirkenlassens auf das Haar über einen ersten zuvor festgelegten Zeitraum unmittelbar nach Vollendung der Ausführung des Schrittes des Aufbringens des ersten Mittels; und
einen zweiten Einwirkungsschritt des Einwirkenlassens auf das Haar über einen zweiten zuvor festgelegten Zeitraum unmittelbar nach Vollendung der Ausführung des Schrittes des Aufbringens des zweiten Mittels,
wobei der Schritt des Aufbringens feiner Wasserpartikel nach Vollendung der Ausführung des ersten Einwirkungsschrittes und vor einem Beginn der Ausführung des Schrittes des Aufbringens des zweiten Mittels ausgeführt wird.

**8.** Verfahren zur Haar-/Kopfhautpflege nach Anspruch 2,

wobei der Ziel-Kopfteil Haar ist,
wobei das Mittel ein Bleichmittel ist, und
wobei der Schritt des Aufbringens feiner Wasserpartikel nach Vollendung der Ausführung des Waschschrittes und vor einem Beginn der Ausführung des Trocknungsschrittes ausgeführt wird.

**9.** Verfahren zur Haar-/Kopfhautpflege nach Anspruch 2, wobei der Ziel-Kopfteil Haar ist, wobei das Verfahren ferner umfasst:

einen Fertigstellungsschritt, der nach Vollendung der Ausführung des Trocknungsschrittes ausgeführt wird und das Haar in Form bringt,
wobei der Schritt des Aufbringens feiner Wasserpartikel in einem Zeitraum ab vor einem Beginn der Ausführung des Mittelaufbringungsschrittes bis nach Vollendung der Ausführung des Fertigstellungsschrittes ausgeführt wird.

**10.** Verfahren zur Haar-/Kopfhautpflege nach Anspruch 1 oder 2, wobei der Ziel-Kopfteil Haar ist, wobei das Verfahren ferner umfasst:
einen Wärmebehandlungsschritt des Wärmebehandelns des Haares.

**11.** Verfahren zur Haar-/Kopfhautpflege nach Anspruch 10, wobei der Schritt des Aufbringens feiner Wasserpartikel vor einem Beginn der Ausführung des Wärmebehandlungsschrittes ausgeführt wird.

**12.** Verfahren zur Haar-/Kopfhautpflege nach Anspruch 1, wobei der Schritt des Aufbringens feiner Wasserpartikel nach Vollendung der Ausführung des Waschschrittes oder nach Vollendung der Ausführung des Trocknungsschrittes ausgeführt wird.

**13.** Verfahren zur Haar-/Kopfhautpflege nach Anspruch 2, wobei ein in dem Mittelaufbringungsschritt aufgebrachtes Mittel mindestens eines von einem Färbemittel, einem Behandlungsmittel, einem Dauerwellenmittel, einem Bleichmittel und einem Haarglättungsmittel enthält.

**14.** Vorrichtung zur Haar-/Kopfhautpflege, **dadurch gekennzeichnet, dass** sie umfasst:
ein Element (11) zum Generieren feiner Wasserpartikel, das dazu ausgestaltet ist, sich durch eine Temperaturverringerung in einem Absorptionszustand zu befinden, in dem es Feuchtigkeit auf einer Oberfläche absorbiert, und dazu ausgestaltet ist, sich durch eine Temperaturerhöhung in einem Freisetzungszustand zu befinden, in dem es die absorbierte Feuchtigkeit als feine Wasserpartikel freisetzt, die eine Temperatur von 40 °C oder weniger aufweisen und eine Größe von 50 Nanometern oder weniger aufweisen;

ein Aufbringungsmittel (12), das dazu ausgestaltet ist, die feinen Wasserpartikel, die von dem Element (11) zum Generieren feiner Wasserpartikel freigesetzt wurden, auf einen Ziel-Kopfteil aufzubringen, der eines oder beides von Haar und einer Kopfhaut eines menschlichen Körpers ist;
ein Steuerungsmittel (23), das dazu ausgestaltet ist, das Element (11) zum Generieren feiner Wasserpartikel und das Aufbringungsmittel (12) zu steuern; und
einen Bedienteil (21), der dazu ausgestaltet ist, bedient zu werden, um zu veranlassen, dass die feinen Wasserpartikel zwischen einem Zeitraum vor der Ausführung des Waschens des Ziel-Kopfteils und einem Zeitraum nach der Ausführung des Trocknens des Ziel-Kopfteils auf den Ziel-Kopfteil aufgebracht werden.

**15.** Vorrichtung zur Haar-/Kopfhautpflege nach Anspruch 14, wobei der Bedienteil (21) dazu ausgestaltet ist, bedient zu werden, um zu veranlassen, dass die feinen Wasserpartikel zwischen einem Zeitraum vor der Ausführung der Mittelaufbringung auf den Ziel-Kopfteil vor der Ausführung des Waschens und einem Zeitraum nach der Ausführung des Trocknens des Ziel-Kopfteils auf den Ziel-Kopfteil aufgebracht werden.

**16.** Vorrichtung zur Haar-/Kopfhautpflege nach Anspruch 14, wobei das Steuerungsmittel (23) dazu ausgestaltet ist, feine Wasserpartikel, die eine Temperatur aufweisen, die gleich oder höher als eine Glasübergangstemperatur einer Proteinstruktur von Haar ist, auf den Ziel-Kopfteil aufzubringen.

**17.** Vorrichtung zur Haar-/Kopfhautpflege nach Anspruch 14, wobei der Ziel-Kopfteil, auf den feine Wasserpartikel durch das Aufbringungsmittel (12) aufgebracht werden sollen, Haar enthält, und das Element (11) zum Generieren feiner Wasserpartikel dazu ausgestaltet ist, die feinen Wasserpartikel freizusetzen, die größer als ein einzelnes Wassermolekül sind, um es den feinen Wasserpartikeln zu erlauben, in dem Haar zu verbleiben und einen Zustand der Schuppenschicht von Haar zu verbessern.

**18.** Vorrichtung zur Haar-/Kopfhautpflege nach Anspruch 14, wobei der Ziel-Kopfteil, auf den feine Wasserpartikel durch das Aufbringungsmittel (12) aufgebracht werden sollen, Haar enthält, und das Element (11) zum Generieren feiner Wasserpartikel dazu ausgestaltet ist, die feinen Wasserpartikel freizusetzen, die ungeladen sind, um es den feinen Wasserpartikeln zu erlauben, in das Haar einzudringen und einen Zustand der Schuppenschicht von Haar zu verbessern.

## Revendications

**1.** Méthode de soin de la chevelure/du cuir chevelu comprenant :

une étape de lavage dans laquelle une partie de tête cible, qui est l'une ou les deux parmi la chevelure et le cuir chevelu d'un corps humain, est lavée ;
une étape de séchage dans laquelle la partie de tête cible qui a été lavée dans l'étape de lavage est séchée ; et
**caractérisée en ce que** la méthode comprend une étape d'application de fines particules d'eau dans laquelle de fines particules d'eau ayant une température de 40°C ou moins et ayant une taille de 50 nanomètres ou moins sont appliquées à la partie de tête cible.

2. Méthode de soin de la chevelure/du cuir chevelu selon la revendication 1, comprenant en outre une étape d'application d'agent dans laquelle un agent est appliqué à la partie de tête cible,
dans laquelle l'étape de lavage est exécutée après qu'une période de temps prédéterminée s'est écoulée depuis l'achèvement de l'exécution de l'étape d'application d'agent.

3. Méthode de soin de la chevelure/du cuir chevelu selon la revendication 2,

   dans laquelle l'étape d'application d'agent est exécutée plusieurs fois, et
   dans laquelle l'étape d'application de fines particules d'eau est exécutée après l'achèvement de l'exécution d'une étape d'application d'agent et avant le début de l'exécution d'une étape d'application d'agent suivante.

4. Méthode de soin de la chevelure/du cuir chevelu selon la revendication 2,

   dans laquelle la partie de tête cible est la chevelure,
   dans laquelle l'étape d'application de fines particules d'eau est exécutée après l'achèvement de l'exécution de l'étape d'application d'agent et avant le début de l'exécution de l'étape de séchage, et
   dans laquelle, dans l'étape d'application de fines particules d'eau, les fines particules d'eau sont appliquées à la chevelure dans la direction partant du côté des pointes des cheveux vers le côté des racines des cheveux de la chevelure.

5. Méthode de soin de la chevelure/du cuir chevelu selon la revendication 1 ou 2,

   dans laquelle la partie de tête cible est la chevelure,
   dans laquelle l'étape d'application de fines particules d'eau est exécutée en même temps que l'exécution de l'étape de séchage ou après l'achèvement de l'exécution de l'étape de séchage, et
   dans laquelle, dans l'étape d'application de fines particules d'eau, les fines particules d'eau sont appliquées à la chevelure dans la direction partant du côté des racines des cheveux vers le côté des pointes des cheveux de la chevelure.

6. Méthode de soin de la chevelure/du cuir chevelu selon la revendication 2,

   dans laquelle la partie de tête cible est la chevelure,
   dans laquelle l'agent est un agent de permanente contenant un premier agent et un deuxième agent qui sont liquides,
   dans laquelle l'étape d'application d'agent comprend :

      une étape d'application de premier agent dans laquelle le premier agent est appliqué à la chevelure ; et
      une étape d'application de deuxième agent dans laquelle le deuxième agent est appliqué à la chevelure après l'achèvement de l'exécution de l'étape d'application de premier agent, et
      dans laquelle l'étape d'application de fines particules d'eau est exécutée avant le début de l'exécution de l'étape d'application de premier agent, ou après l'achèvement de l'exécution de l'étape d'application de premier agent, et avant l'étape d'exécution de l'étape d'application de deuxième agent.

7. Méthode de soin de la chevelure/du cuir chevelu selon la revendication 6,

   dans laquelle le premier agent a pour fonction de couper le tissu interne des cheveux, et
   dans laquelle le deuxième agent a pour fonction de lier le tissu interne des cheveux coupés par le premier agent,
   la méthode comprenant en outre :

      une première étape de pose dans laquelle la chevelure est laissée à reposer pendant une première période de temps prédéterminée immédiatement après l'achèvement de l'exécution de l'étape d'application de premier agent ; et
      une deuxième étape de pose dans laquelle la chevelure est laissée à reposer pendant une deuxième période de temps prédéterminée immédiatement après l'achèvement de l'exécution de l'étape d'application de deuxième agent,
      dans laquelle l'étape d'application de particules d'eau est exécutée après l'achèvement de l'exécution de la première étape de pose et avant le début de l'exécution de l'étape d'application du deuxième agent.

8. Méthode de soin de la chevelure/du cuir chevelu selon la revendication 2,

   dans laquelle la partie de tête cible est la chevelure,
   dans laquelle l'agent est un agent décolorant, et
   dans laquelle l'étape d'application de particules d'eau est exécutée après l'achèvement de l'exécution de l'étape de lavage et avant le début de l'exécution de l'étape de séchage.

**9.** Méthode de soin de la chevelure/du cuir chevelu selon la revendication 2, dans laquelle la partie de tête cible est la chevelure, la méthode comprenant en outre :

une étape de finition qui est exécutée après l'achèvement de l'exécution de l'étape de séchage et qui coiffe la chevelure,
dans laquelle l'étape d'application de particules d'eau est exécutée pendant une période allant d'avant le début de l'exécution de l'étape d'application d'agent à après l'achèvement de l'exécution de l'étape de finition.

**10.** Méthode de soin de la chevelure/du cuir chevelu selon la revendication 1 ou 2, dans laquelle la partie de tête cible est la chevelure, la méthode comprenant en outre :
une étape de traitement à la chaleur dans laquelle la chevelure est traitée à la chaleur.

**11.** Méthode de soin de la chevelure/du cuir chevelu selon la revendication 10, dans laquelle l'étape d'application de fines particules d'eau est exécutée avant le début de l'exécution de l'étape de traitement à la chaleur.

**12.** Méthode de soin de la chevelure/du cuir chevelu selon la revendication 1, dans laquelle l'étape d'application de fines particules d'eau est exécutée après l'achèvement de l'exécution de l'étape de lavage ou après l'achèvement de l'exécution de l'étape de séchage.

**13.** Méthode de soin de la chevelure/du cuir chevelu selon la revendication 2, dans laquelle un agent appliqué lors de l'étape d'application d'agent englobe au moins l'un parmi un agent de coloration, un agent de traitement, un agent de permanente, un agent de décoloration, et un agent de lissage des cheveux.

**14.** Dispositif de soin de la chevelure/du cuir chevelu, **caractérisé en ce qu'**il comprend :

un élément générateur de fines particules d'eau (11) configuré pour être, par une diminution de la température, dans un état d'absorption dans lequel l'humidité est absorbée sur une surface, et configuré pour être, par une augmentation de la température, dans un état de libération dans lequel l'humidité qui a été absorbée est libérée sous la forme de fines particules d'eau ayant une température de 40°C ou moins et ayant une taille de 50 nanomètres ou moins ;
un moyen d'application (12) configuré pour appliquer les fines particules d'eau qui ont été libérées par l'élément générateur de fines particules d'eau (11) à une partie de tête cible qui est l'un ou les deux parmi la chevelure et le cuir chevelu d'un corps humain ;
un moyen de commande (23) configuré pour commander l'élément générateur de fines particules d'eau (11) et le moyen d'application (12) ; et
une partie d'actionnement (21) configurée pour être actionnée de manière à provoquer l'application des fines particules d'eau à la partie de tête cible entre une période avant l'exécution d'un lavage de la partie de tête cible et une période après l'exécution d'un séchage de la partie de tête cible.

**15.** Dispositif de soin de la chevelure/du cuir chevelu selon la revendication 14, dans lequel la partie d'actionnement (21) est configurée pour être actionnée de manière à provoquer l'application des fines particules d'eau à la partie de tête cible entre une période avant l'exécution d'une application d'agent à la partie de tête cible antérieure à l'exécution du lavage et une période après l'exécution d'un séchage de la partie de tête cible.

**16.** Dispositif de soin de la chevelure/du cuir chevelu selon la revendication 14, dans lequel le moyen de commande (23) est configuré pour appliquer à la partie de tête cible de fines particules d'eau ayant une température égale ou supérieure à la température de transition vitreuse de la structure protéique des cheveux.

**17.** Dispositif de soin de la chevelure/du cuir chevelu selon la revendication 14, dans lequel la partie de tête cible à laquelle de fines particules d'eau doivent être appliquées par le moyen d'application (12) englobe la chevelure, et l'élément générateur de fines particules d'eau (11) est configuré pour libérer de fines particules d'eau plus grosses qu'une molécule d'eau isolée pour permettre aux fines particules d'eau de rester dans les cheveux et d'améliorer l'état des cuticules des cheveux.

**18.** Dispositif de soin de la chevelure/du cuir chevelu selon la revendication 14, dans lequel la partie de tête cible à laquelle de fines particules d'eau doivent être appliquées par le moyen d'application (12) englobe la chevelure, et l'élément générateur de fines particules d'eau (11) est configuré pour libérer de fines particules d'eau non chargées pour permettre aux fines particules d'eau de passer par perméation dans les cheveux et d'améliorer l'état des cuticules des cheveux.

*FIG. 1*

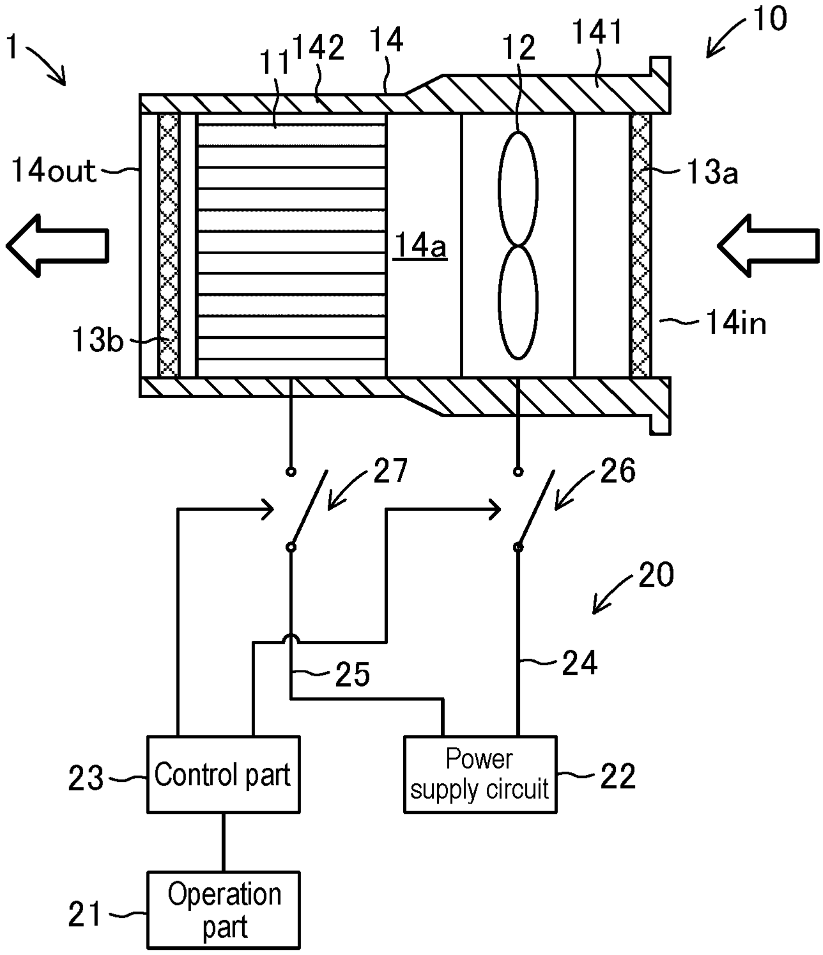
1
11
142
14
12
141
10
14out
13a
14a
13b
14in
27
26
20
25
24
23
Control part
Power supply circuit
22
21
Operation part

*FIG. 2*

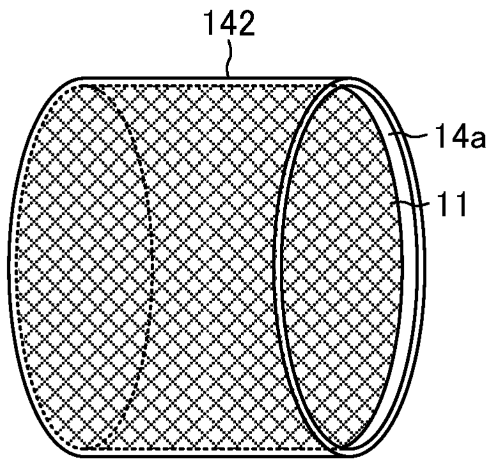
142
14a
11

*FIG. 3*

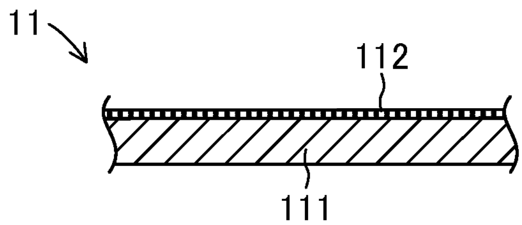

FIG. 4A

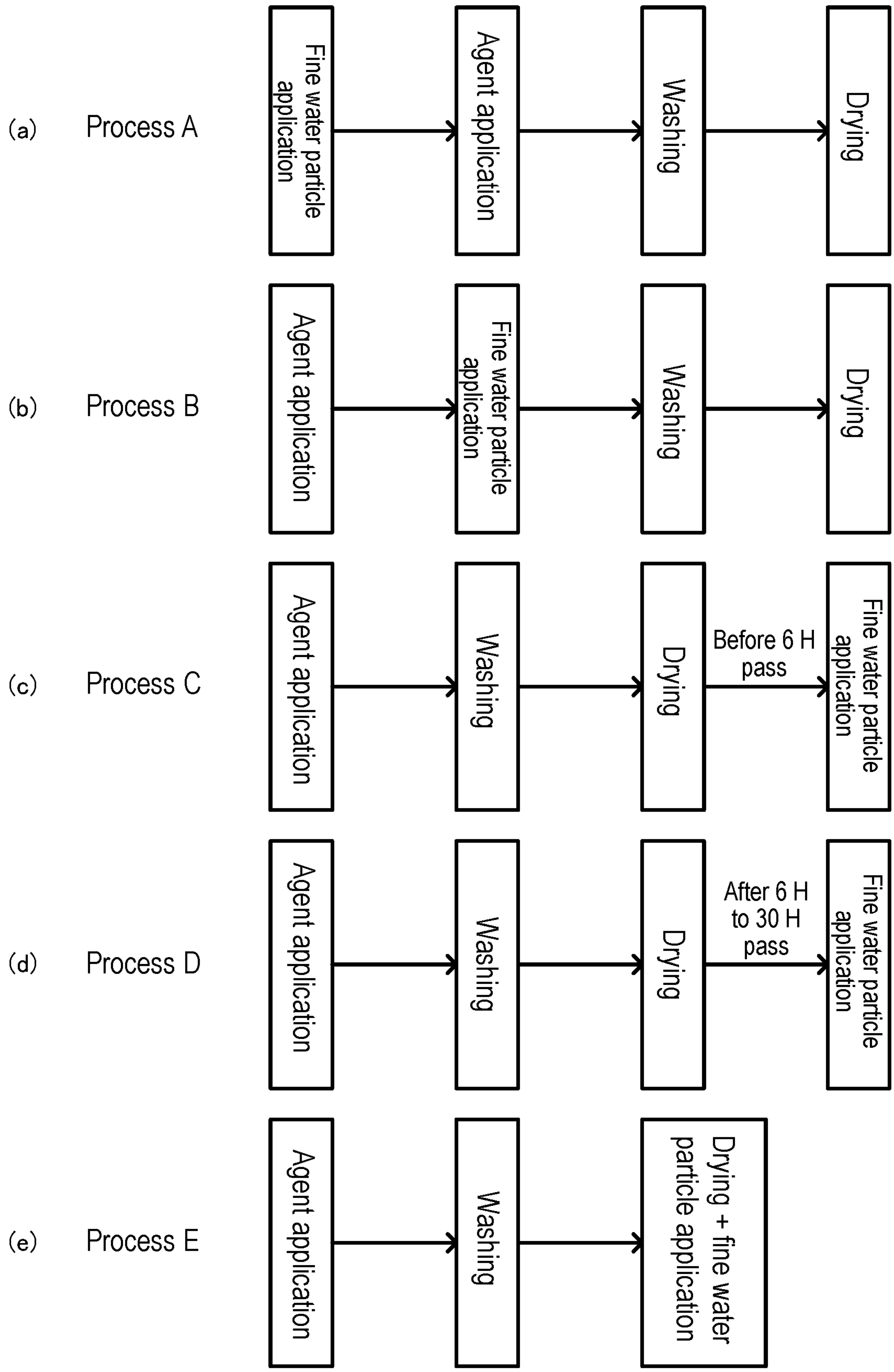
(a) Process A
Fine water particle application
Agent application
Washing
Drying
(b) Process B
Agent application
Fine water particle application
Washing
Drying
(c) Process C
Agent application
Washing
Drying
Before 6 H pass
Fine water particle application
(d) Process D
Agent application
Washing
Drying
After 6 H to 30 H pass
Fine water particle application
(e) Process E
Agent application
Washing
Drying + fine water particle application

FIG. 4B

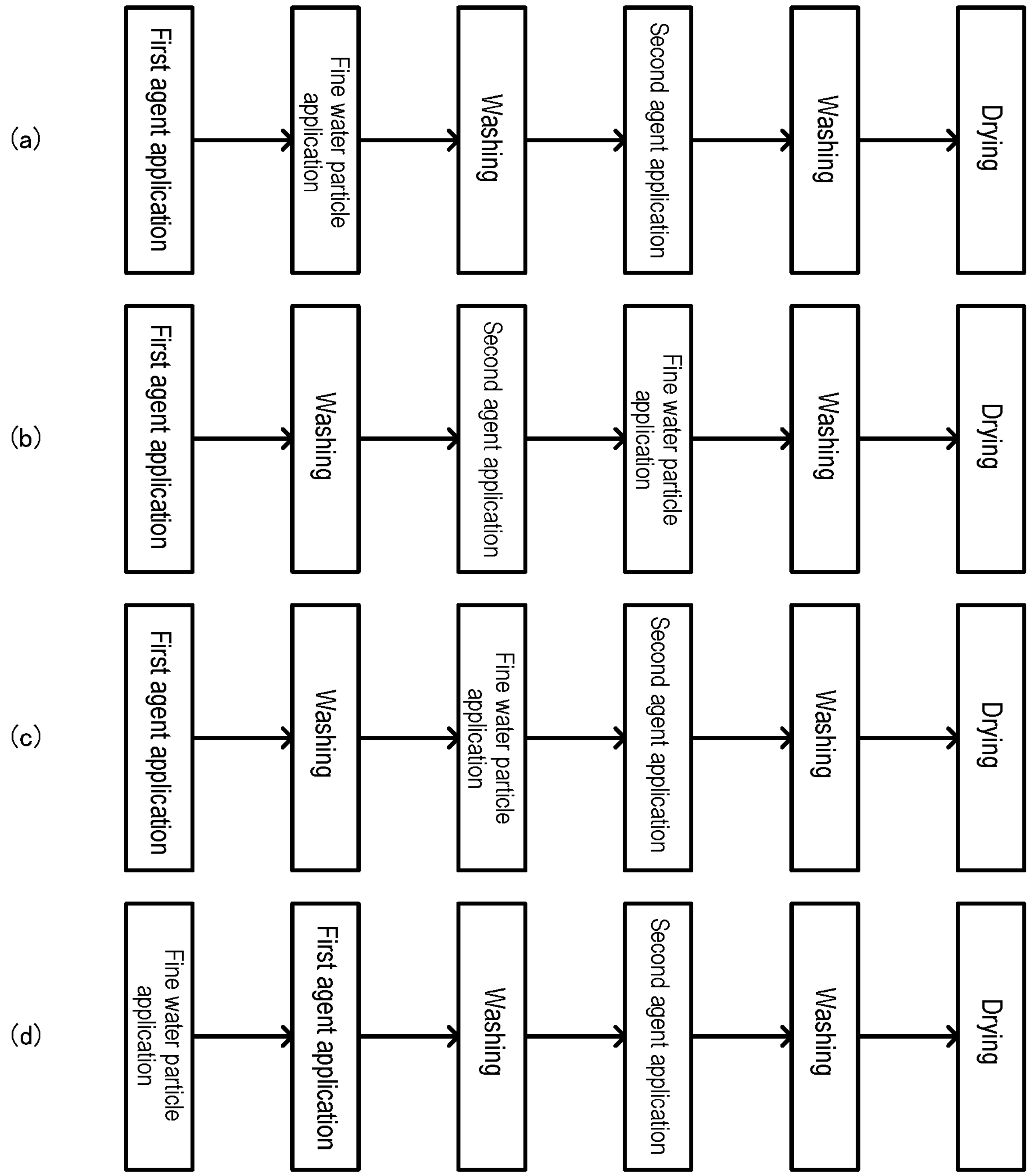
(a)
First agent application
Fine water particle application
Washing
Second agent application
Washing
Drying
(b)
First agent application
Washing
Second agent application
Fine water particle application
Washing
Drying
(c)
First agent application
Washing
Fine water particle application
Second agent application
Washing
Drying
(d)
Fine water particle application
First agent application
Washing
Second agent application
Washing
Drying

FIG. 4C

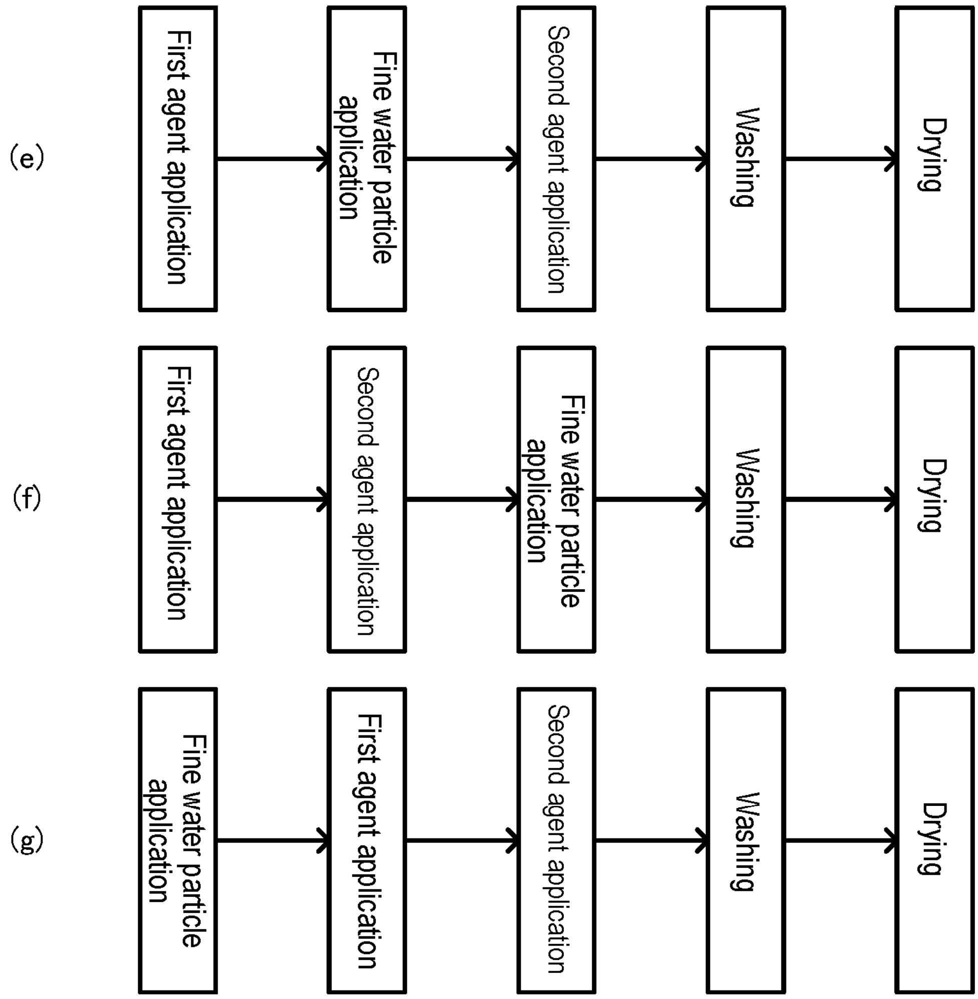
(e)
First agent application
Fine water particle application
Second agent application
Washing
Drying
(f)
First agent application
Second agent application
Fine water particle application
Washing
Drying
(g)
Fine water particle application
First agent application
Second agent application
Washing
Drying

FIG. 5

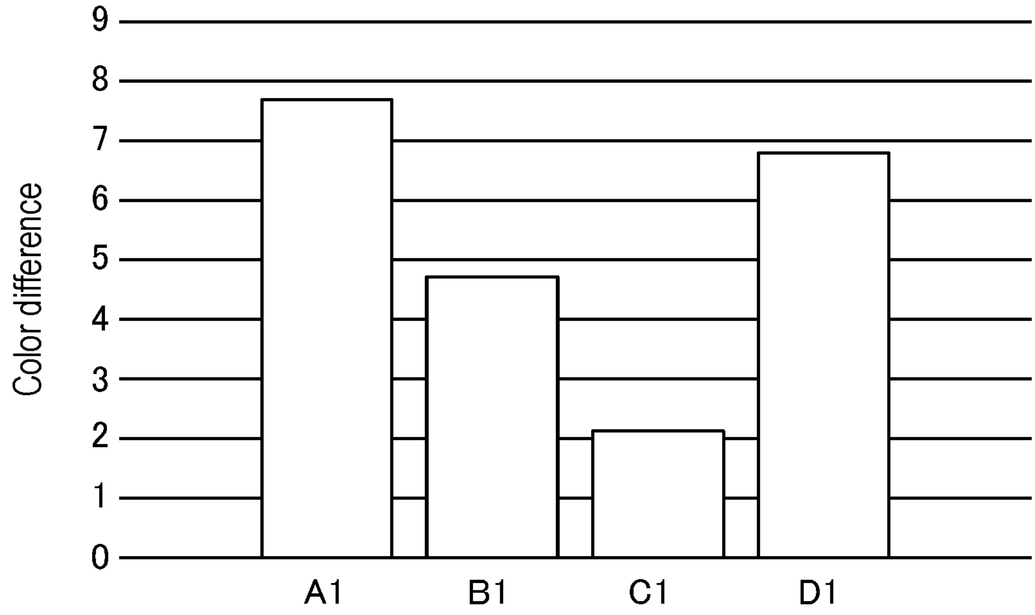
Color difference
9
8
7
6
5
4
3
2
1
0
A1
B1
C1
D1

FIG. 6

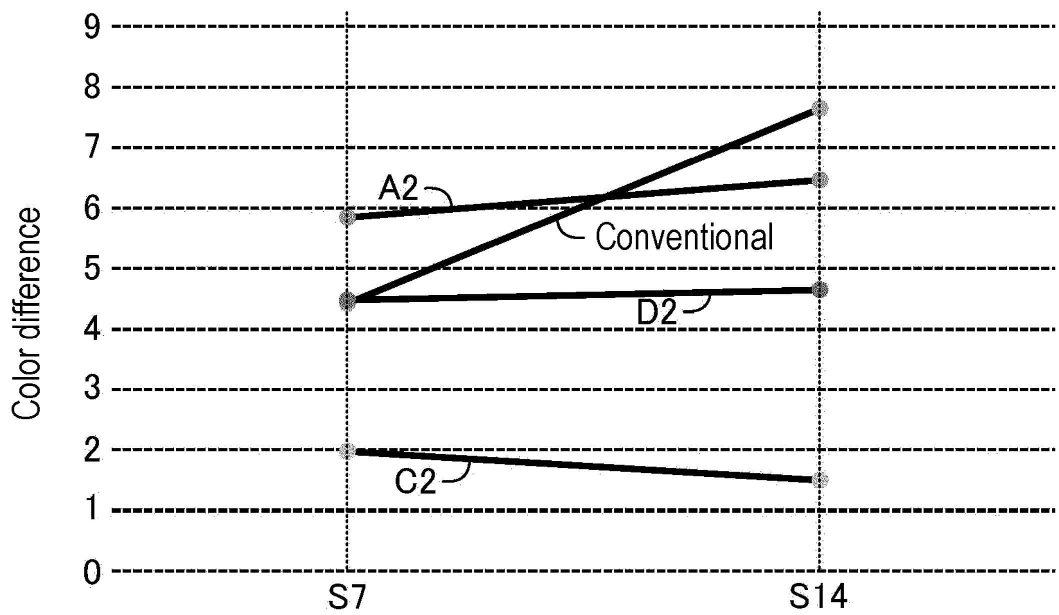
Color difference
9
8
7
6
5
4
3
2
1
0
A2
Conventional
D2
C2
S7
S14

*FIG. 7*

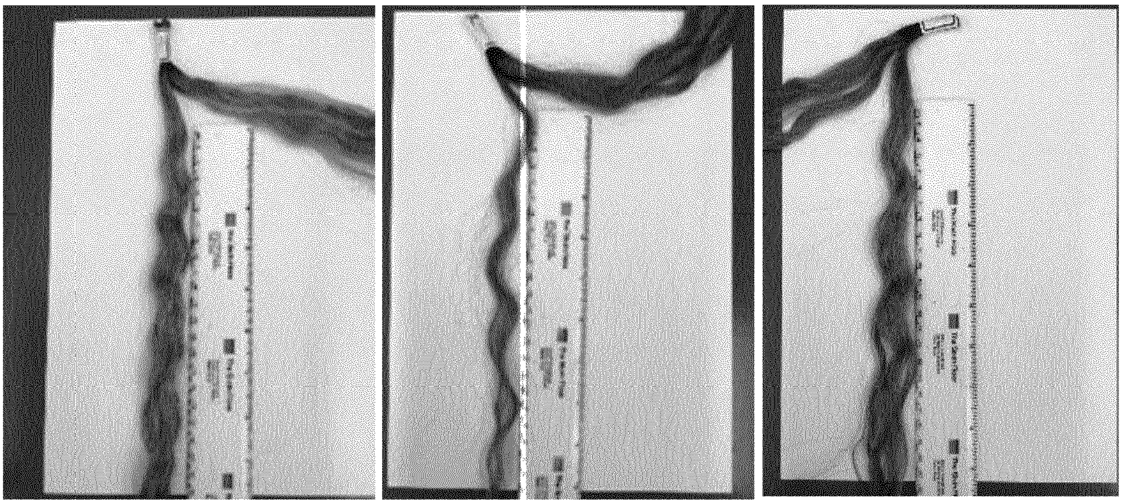

(a) (b) (c)

*FIG. 8*

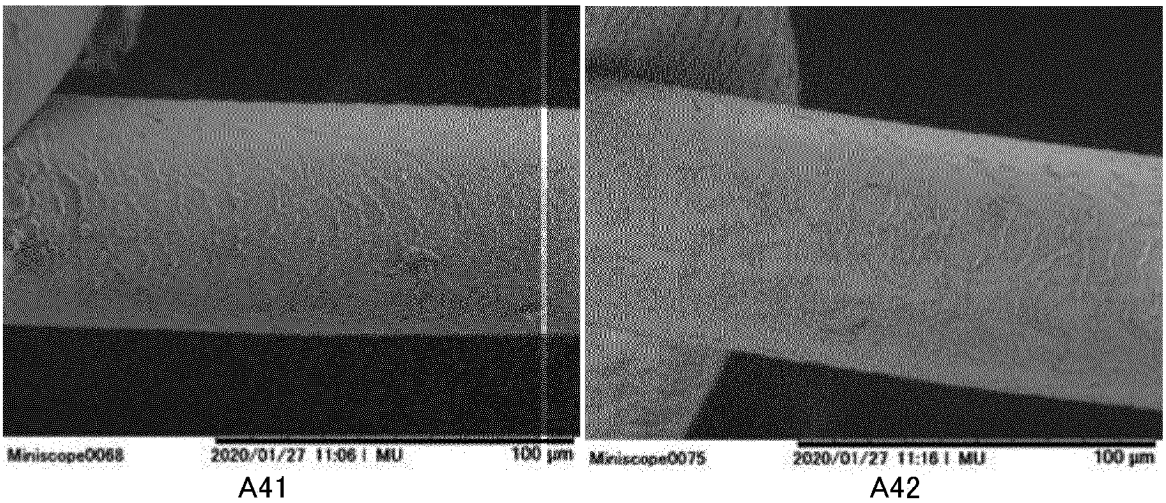


A41 A42

*FIG. 9*

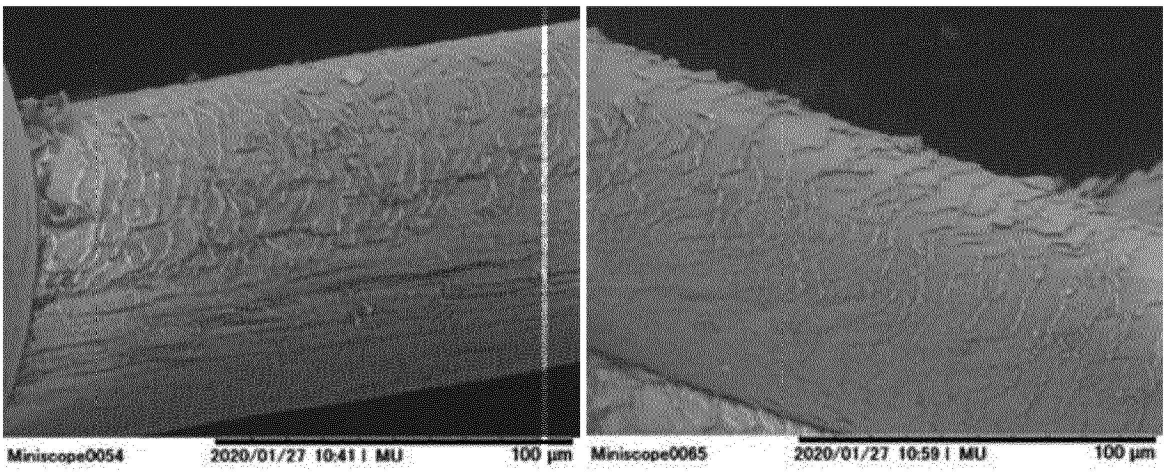
Miniscope0054
2020/01/27 10:41 I MU
100 µm
Miniscope0065
2020/01/27 10:59 I MU
100 µm

N1 N2

FIG. 10

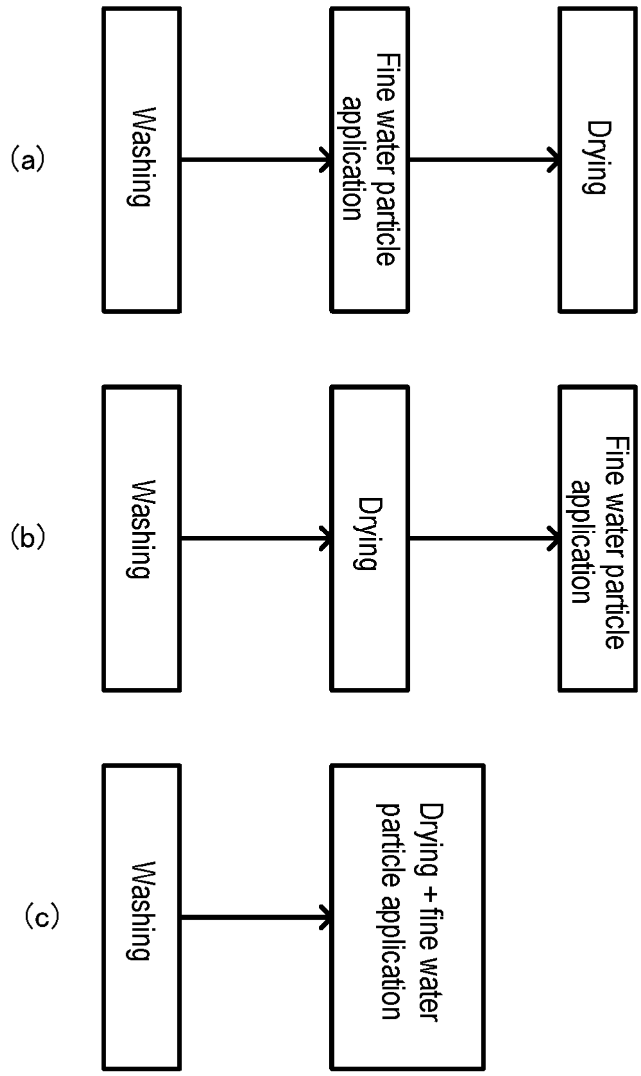
(a)
Washing
Fine water particle application
Drying
(b)
Washing
Drying
Fine water particle application
(c)
Washing
Drying + fine water particle application

*FIG. 11*

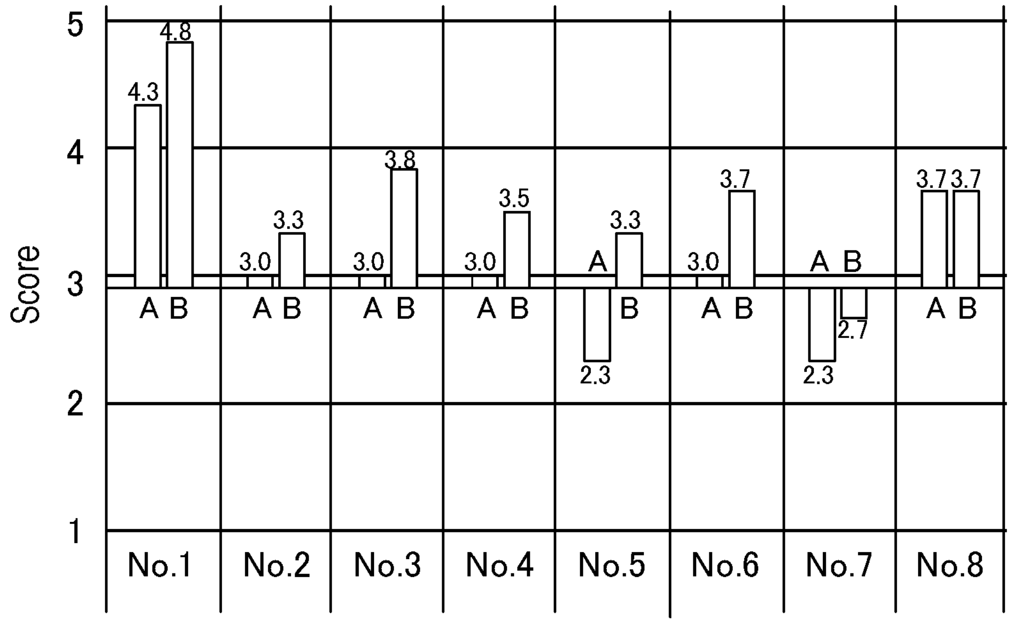
Score
5
4
3
2
1
4.3
4.8
3.0
3.3
3.0
3.8
3.0
3.5
2.3
3.3
3.0
3.7
2.3
2.7
3.7
3.7
A B
No.1
No.2
No.3
No.4
No.5
No.6
No.7
No.8

*FIG. 12*

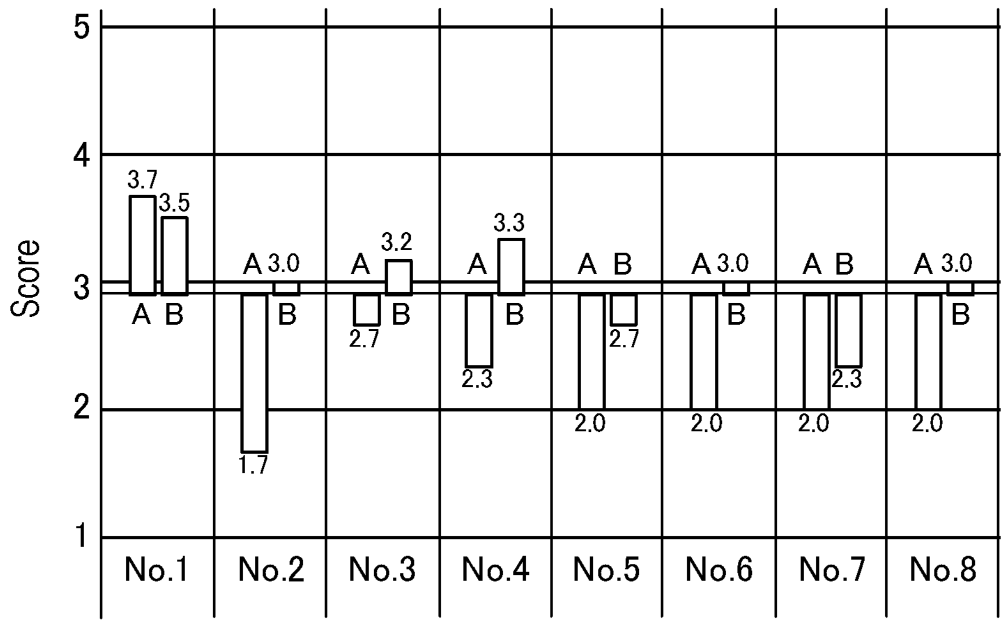
Score
5
4
3
2
1
3.7
3.5
1.7
3.0
2.7
3.2
2.3
3.3
2.0
2.7
2.0
3.0
2.0
2.3
2.0
3.0
A B
No.1
No.2
No.3
No.4
No.5
No.6
No.7
No.8

FIG. 13

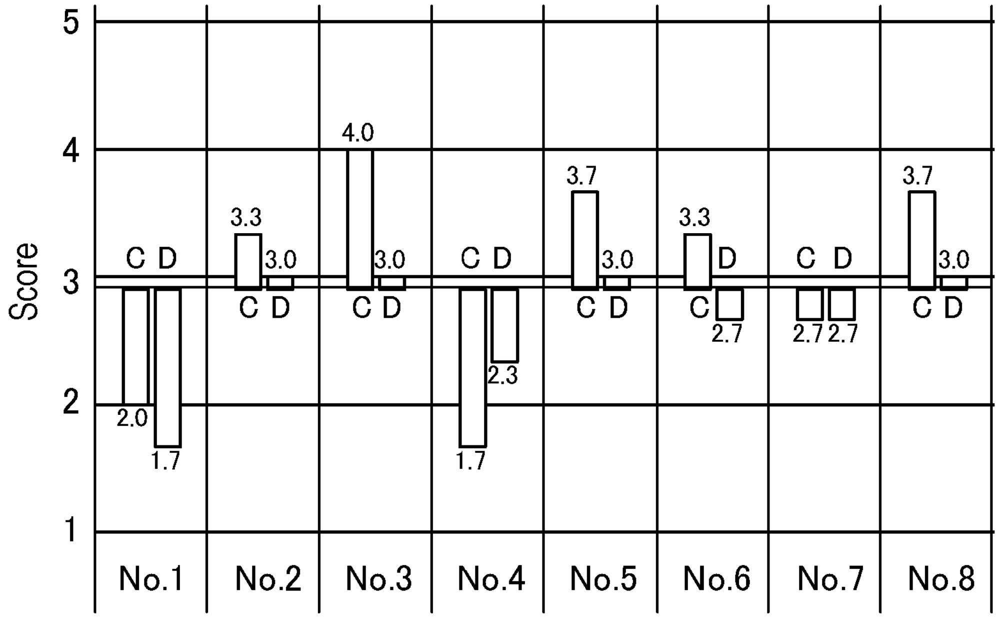
Score
5
4
3
2
1
C D
2.0
1.7
3.3
3.0
C D
4.0
3.0
C D
C D
2.3
1.7
3.7
3.0
C D
3.3
D
C
2.7
C D
2.7 2.7
3.7
3.0
C D
No.1
No.2
No.3
No.4
No.5
No.6
No.7
No.8

FIG. 14

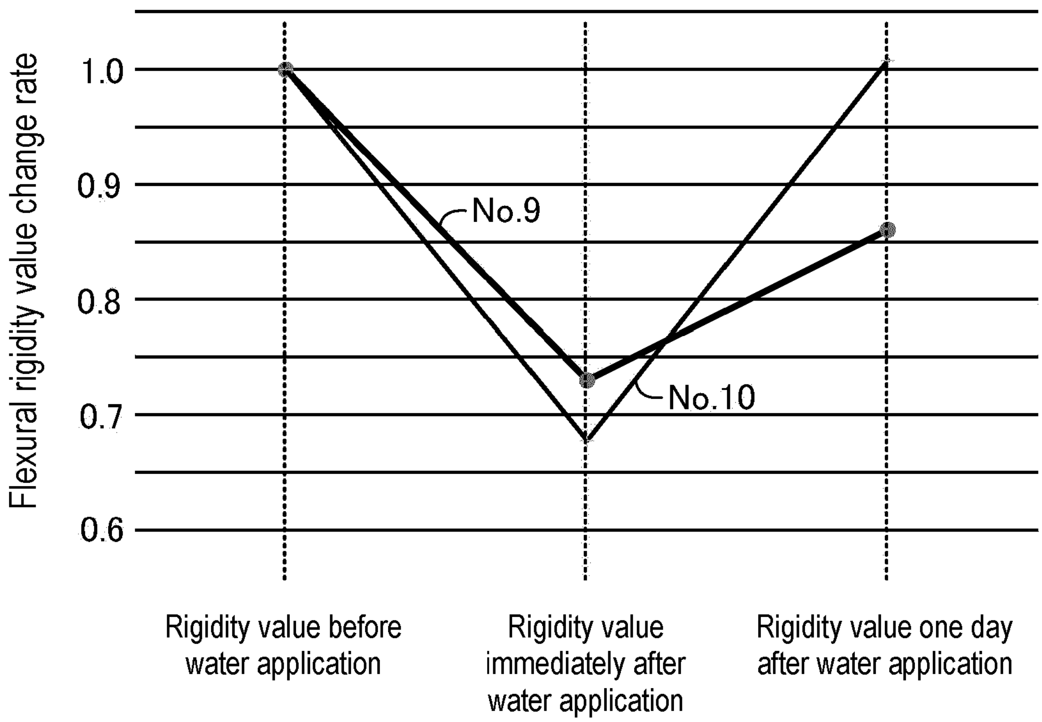
Flexural rigidity value change rate
1.0
0.9
0.8
0.7
0.6
No.9
No.10
Rigidity value before water application
Rigidity value immediately after water application
Rigidity value one day after water application

# FIG. 15

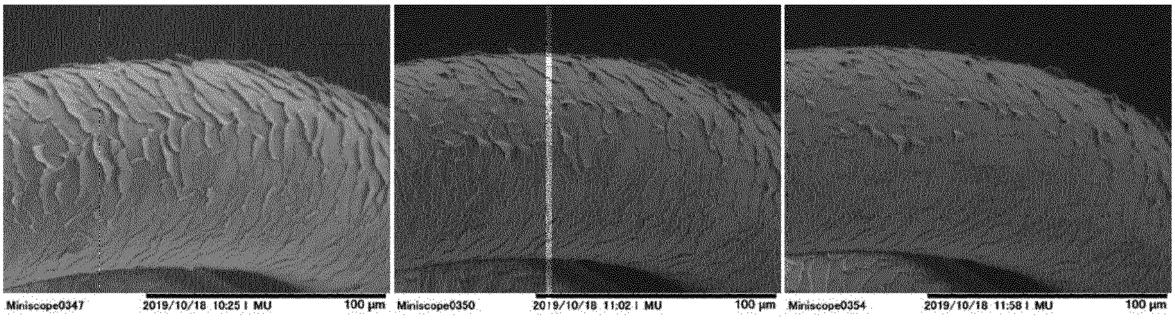


(a) (b) (c)

# FIG. 16

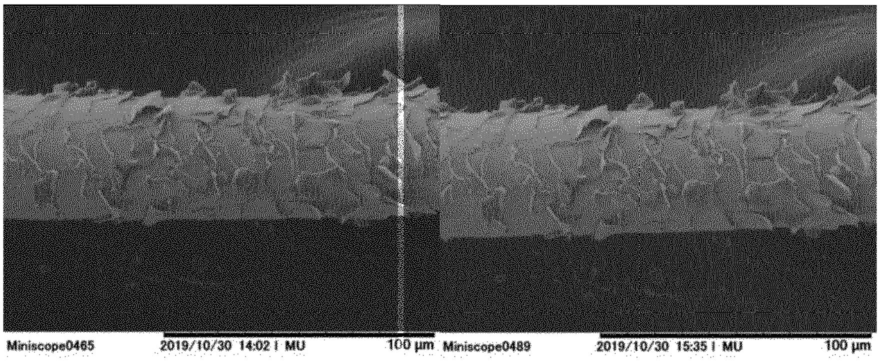


(a) (b)

# FIG. 17

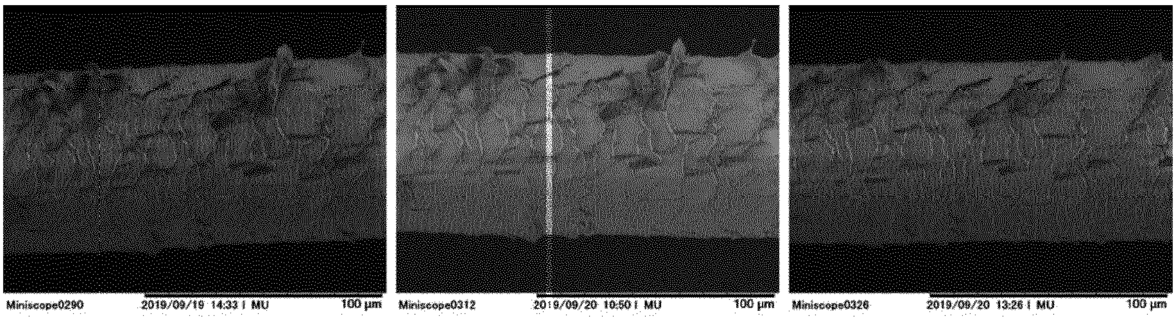


(a) (b) (c)

*FIG. 18*

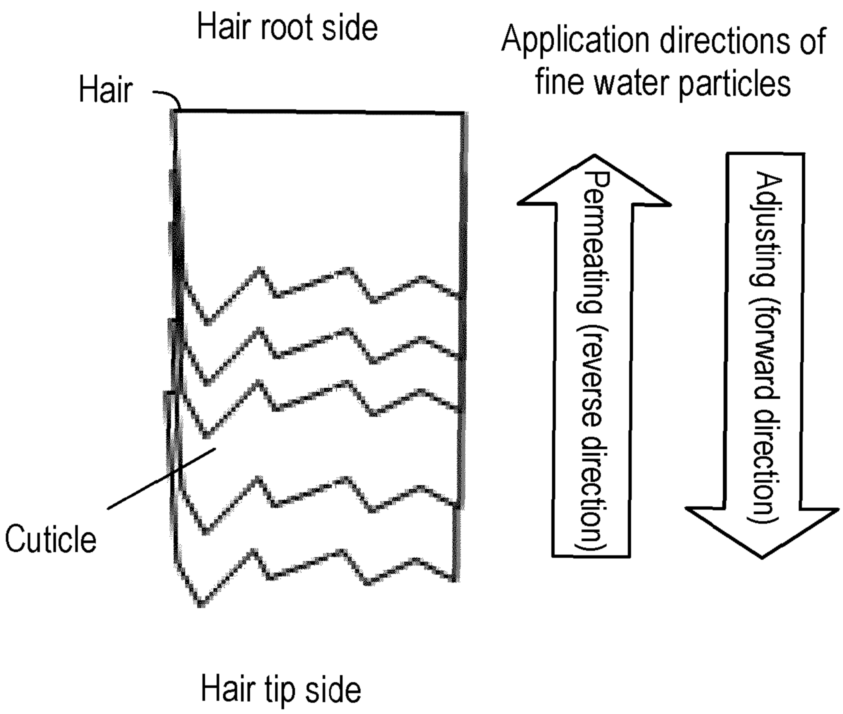
Hair root side
Hair
Application directions of fine water particles
Permeating (reverse direction)
Adjusting (forward direction)
Cuticle
Hair tip side

FIG. 19

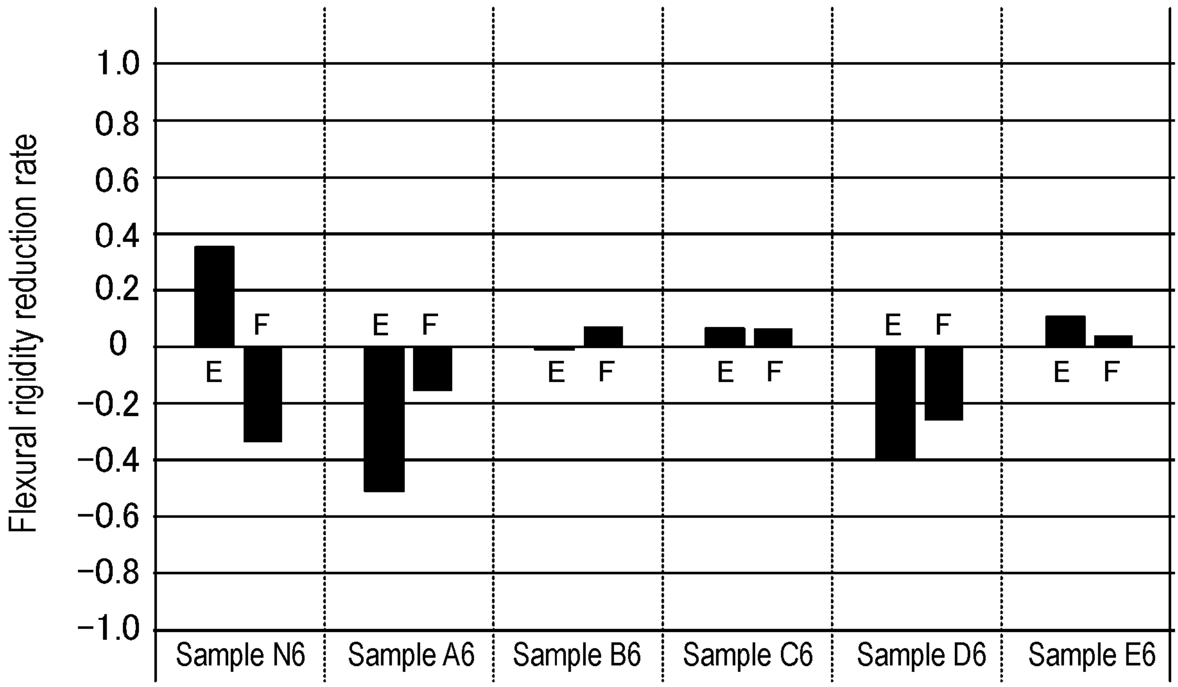
Flexural rigidity reduction rate
1.0
0.8
0.6
0.4
0.2
0
−0.2
−0.4
−0.6
−0.8
−1.0
E
F
Sample N6
Sample A6
Sample B6
Sample C6
Sample D6
Sample E6

FIG. 20

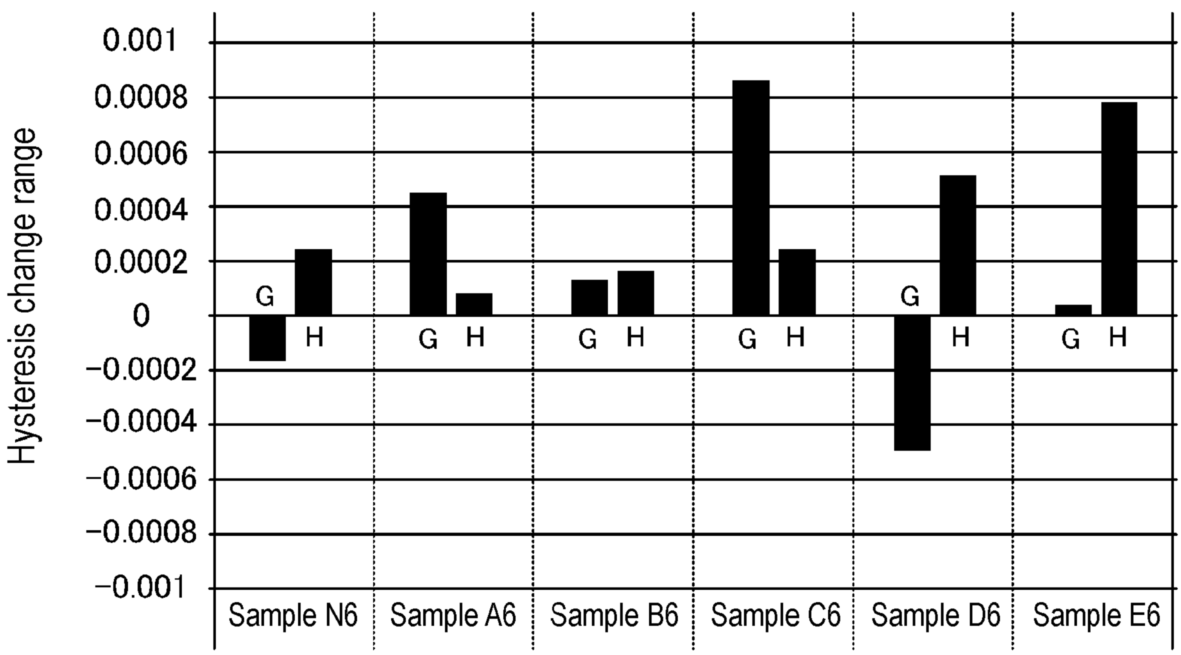
Hysteresis change range
0.001
0.0008
0.0006
0.0004
0.0002
0
−0.0002
−0.0004
−0.0006
−0.0008
−0.001
G
H
Sample N6
Sample A6
Sample B6
Sample C6
Sample D6
Sample E6

FIG. 21

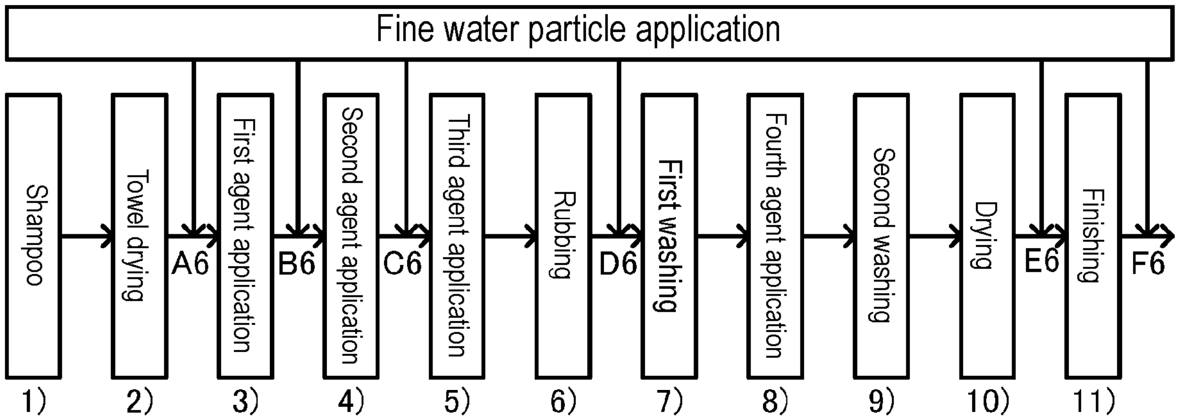


FIG. 22

| Agent | Properties |
|---|---|
| First agent | Misty |
| Second agent | No silicone/small amount of silicone, Mild-texture cream |
| Third agent | Larger amount of silicone, Hard-texture cream |
| Fourth agent | Larger amount of silicone/oil, Smooth-texture cream |

FIG. 23

| Timing | Effect | Suited hair type | Finish |
|---|---|---|---|
| C6 | Hair softness up | Low damage | Airy |
| D6 | Hair root side resilience up | Hair without resilience | Airy |
| E6 | Hair softness up | High damage | Fluffy |
| F6 | Hair softness up | High damage | Tidiness emphasized/heavy |

FIG. 24

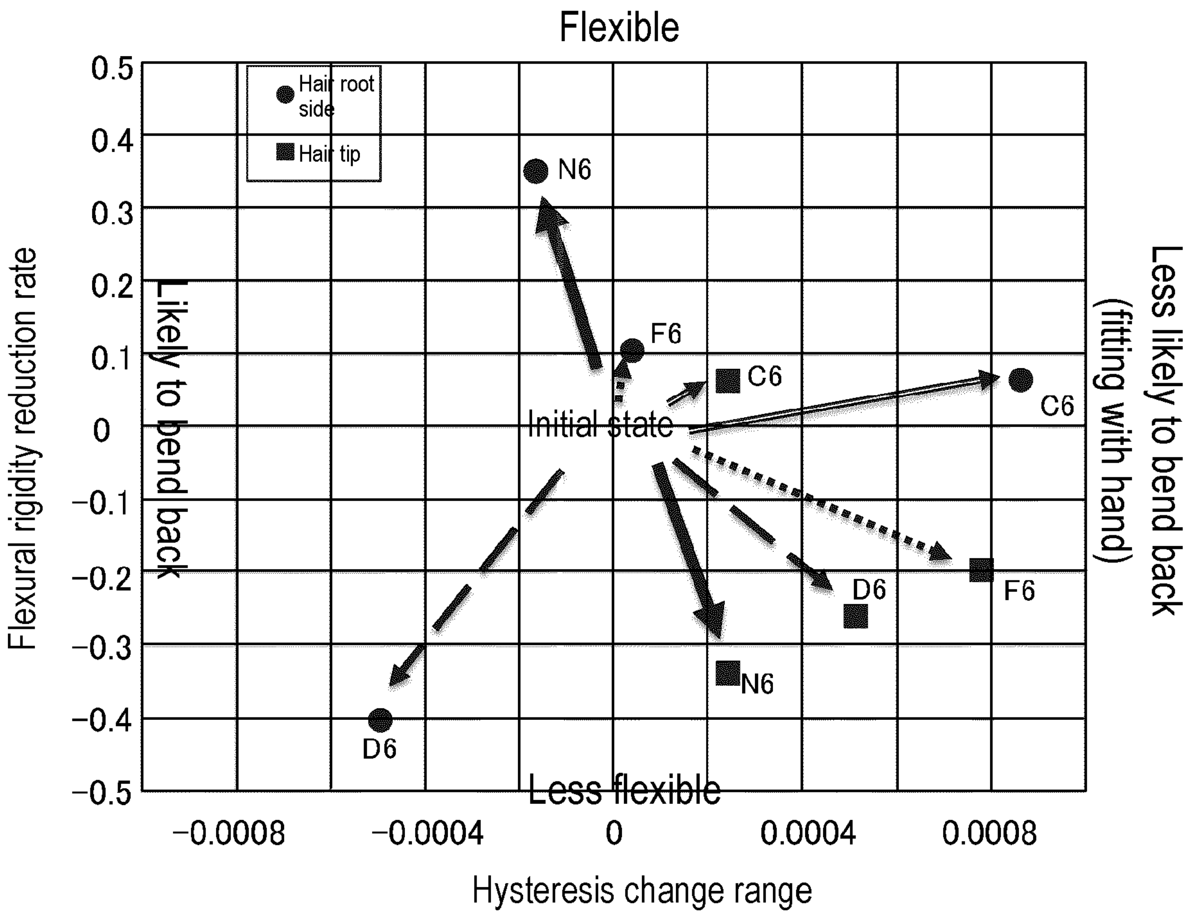
Flexible
Hair root side
Hair tip
N6
F6
C6
Initial state
C6
D6
F6
N6
D6
Less flexible
Likely to bend back
Less likely to bend back (fitting with hand)
Flexural rigidity reduction rate
0.5
0.4
0.3
0.2
0.1
0
−0.1
−0.2
−0.3
−0.4
−0.5
−0.0008
−0.0004
0
0.0004
0.0008
Hysteresis change range

FIG. 25A

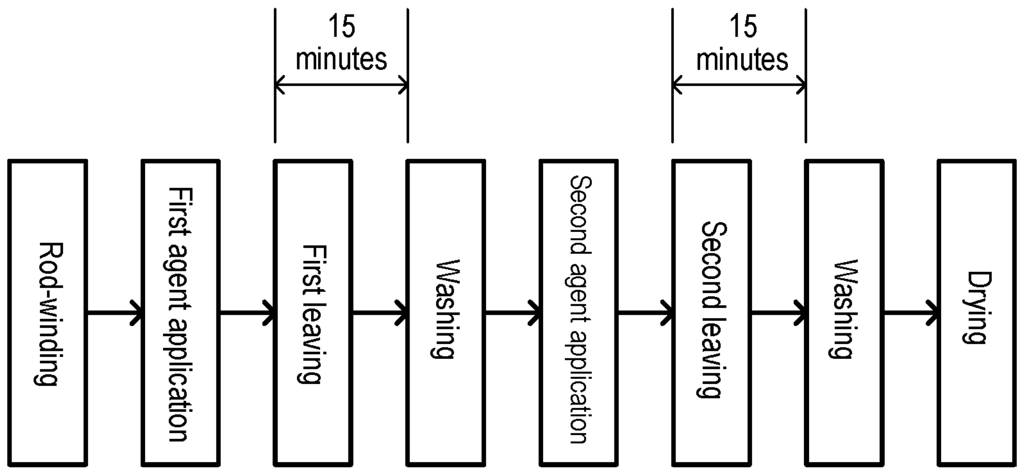


FIG. 25B

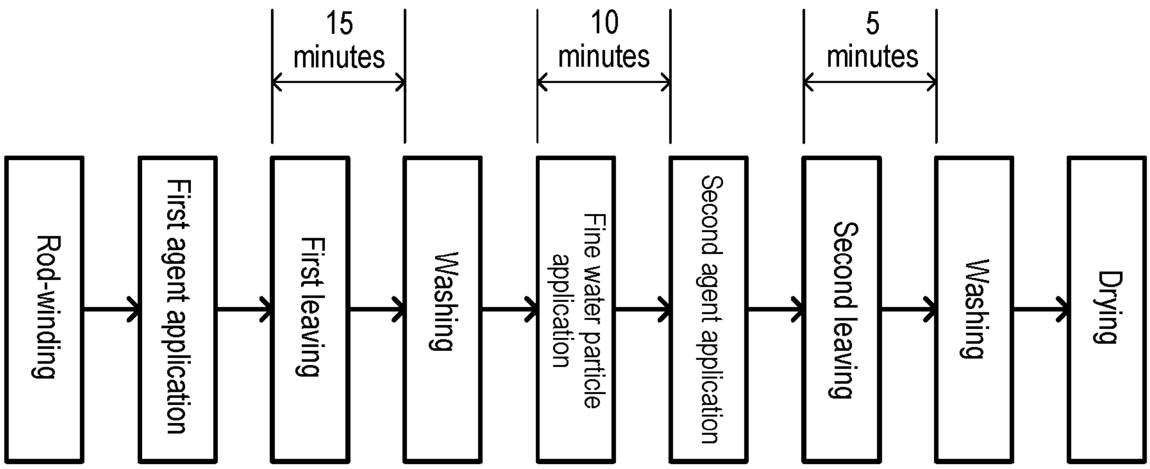

FIG. 26

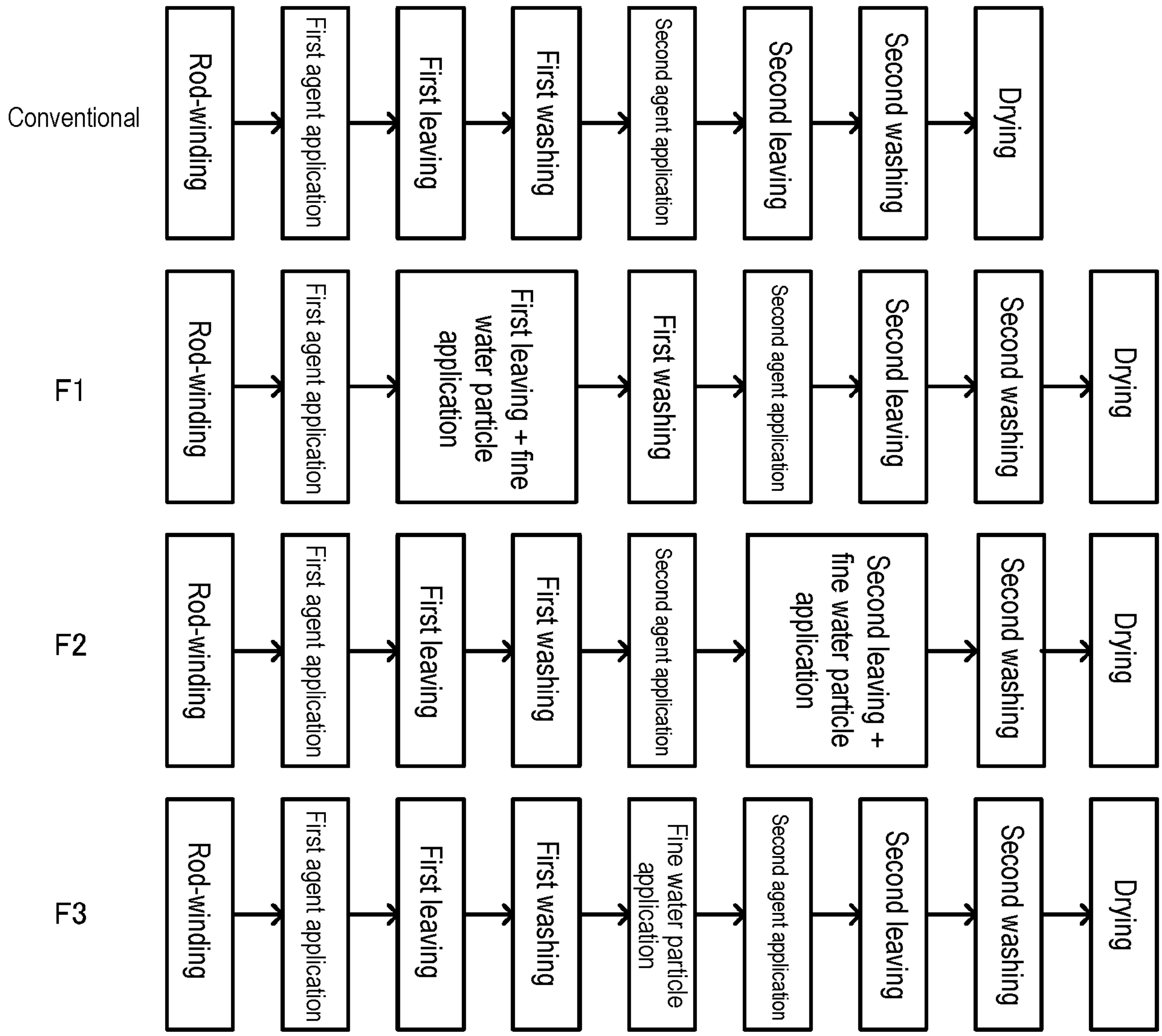
Conventional
Rod-winding
First agent application
First leaving
First washing
Second agent application
Second leaving
Second washing
Drying
F1
Rod-winding
First agent application
First leaving + fine water particle application
First washing
Second agent application
Second leaving
Second washing
Drying
F2
Rod-winding
First agent application
First leaving
First washing
Second agent application
Second leaving + fine water particle application
Second washing
Drying
F3
Rod-winding
First agent application
First leaving
First washing
Fine water particle application
Second agent application
Second leaving
Second washing
Drying

FIG. 27

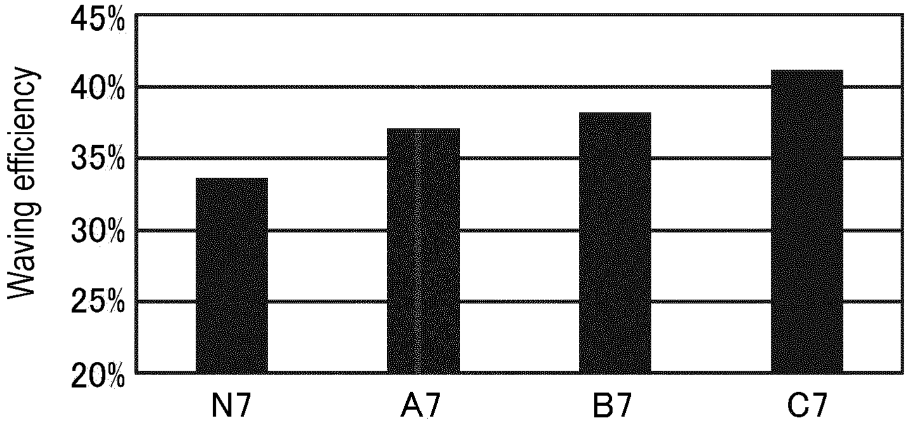
Waving efficiency
45%
40%
35%
30%
25%
20%
N7
A7
B7
C7

FIG. 28

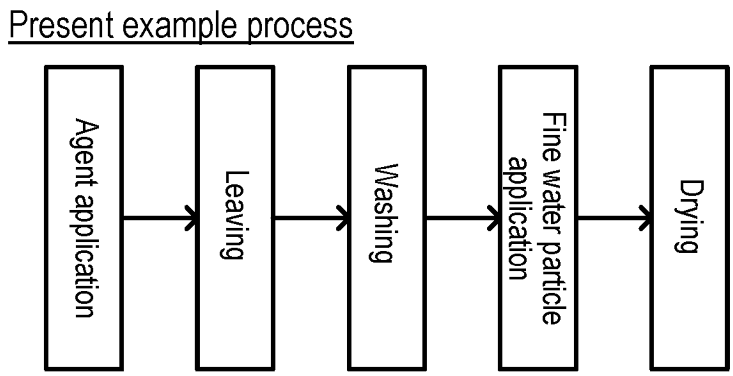


FIG. 29A

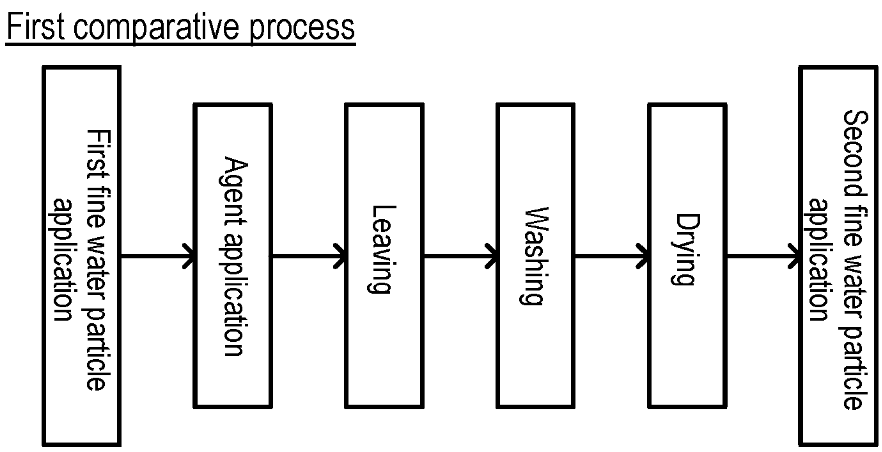


FIG. 29B

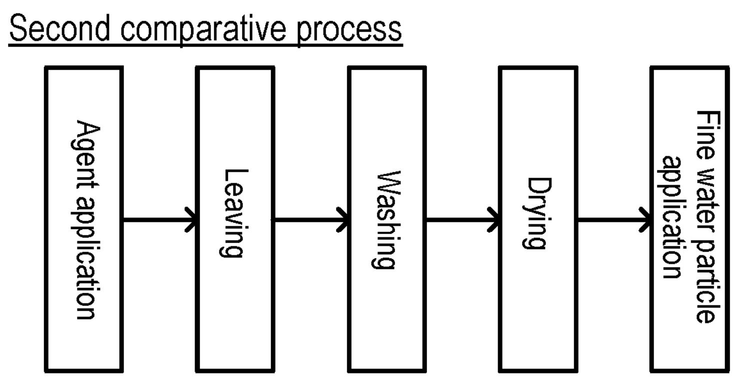

FIG. 30

B8 (First comparative process) | A8 (Present example process) | C8 (Second comparative process)

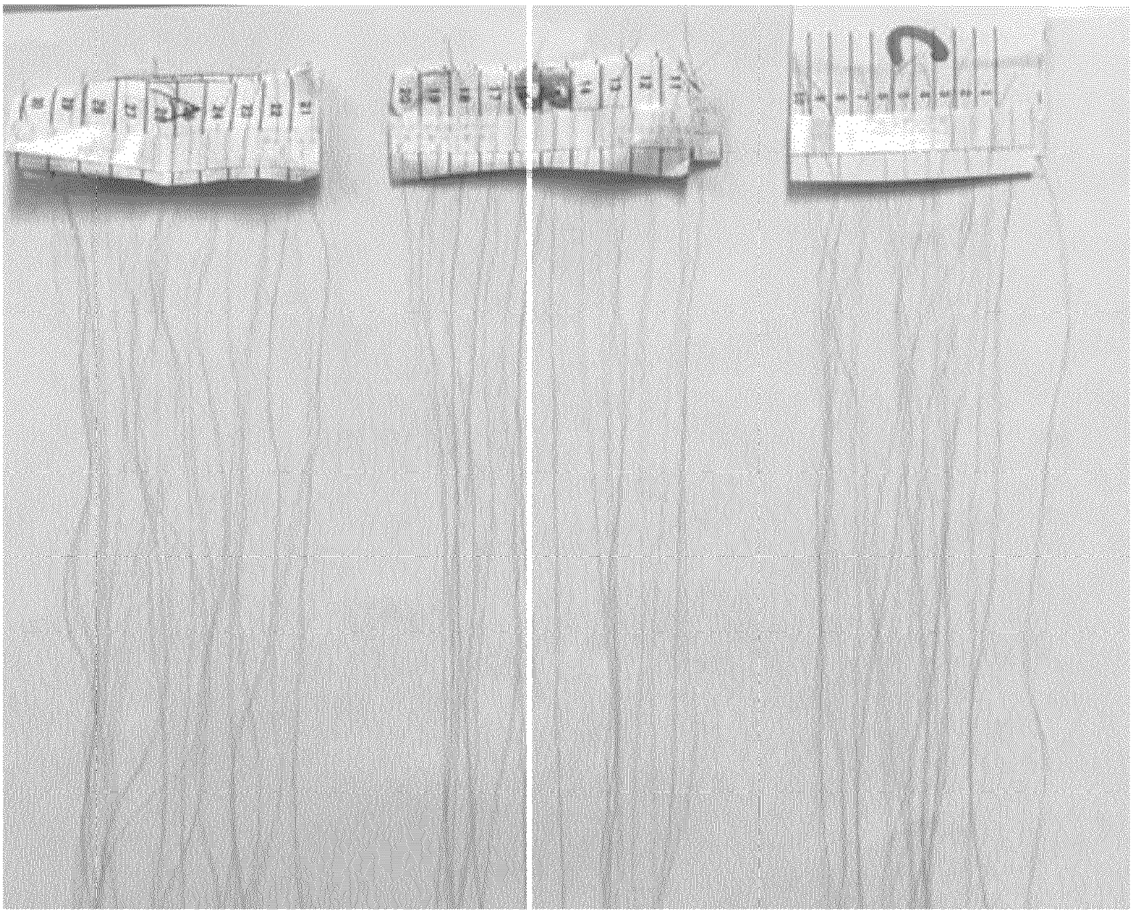

FIG. 31

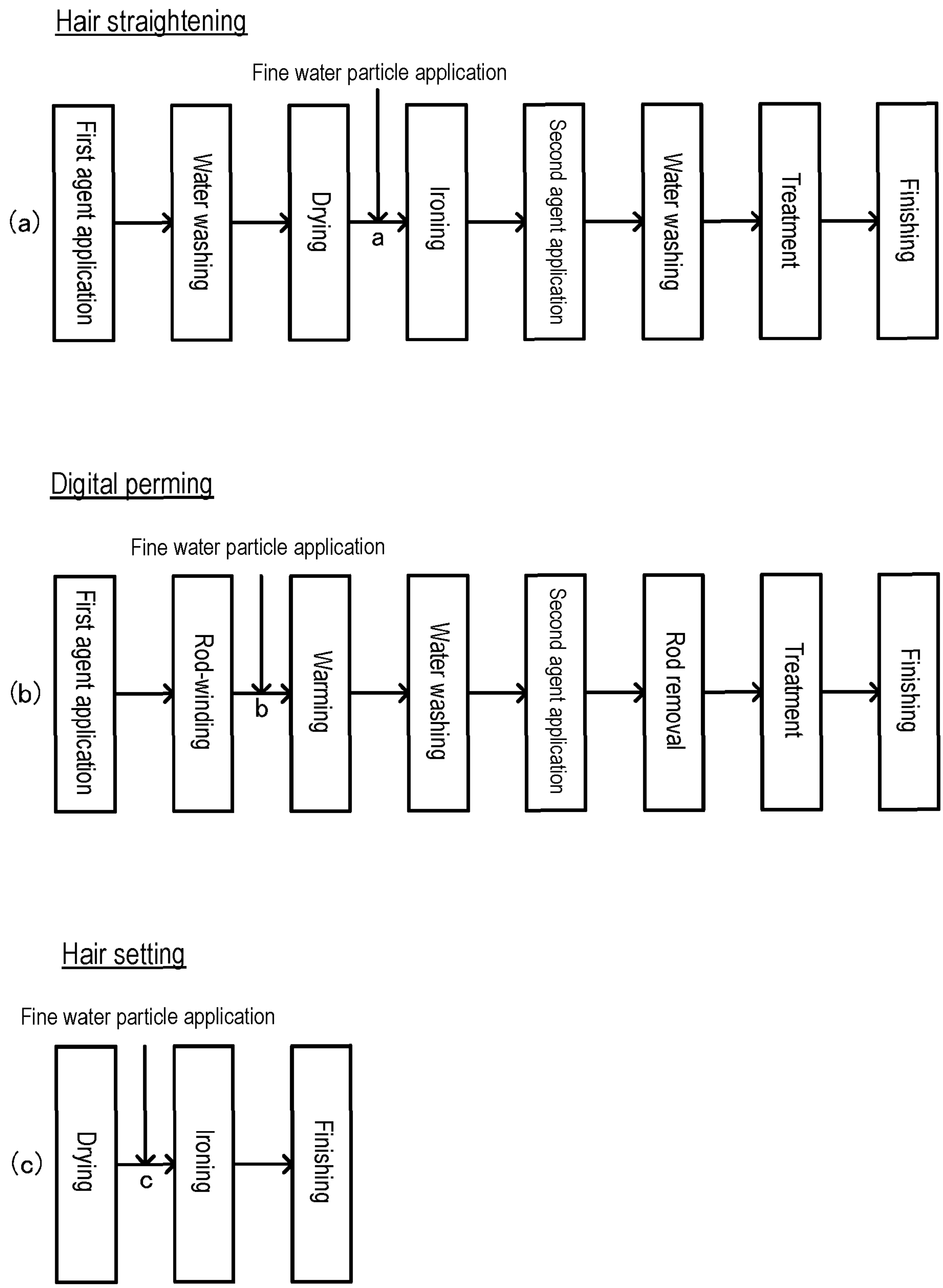
Hair straightening
Fine water particle application
(a)
First agent application
Water washing
Drying
a
Ironing
Second agent application
Water washing
Treatment
Finishing
Digital perming
Fine water particle application
(b)
First agent application
Rod-winding
b
Warming
Water washing
Second agent application
Rod removal
Treatment
Finishing
Hair setting
Fine water particle application
(c)
Drying
c
Ironing
Finishing

FIG. 32

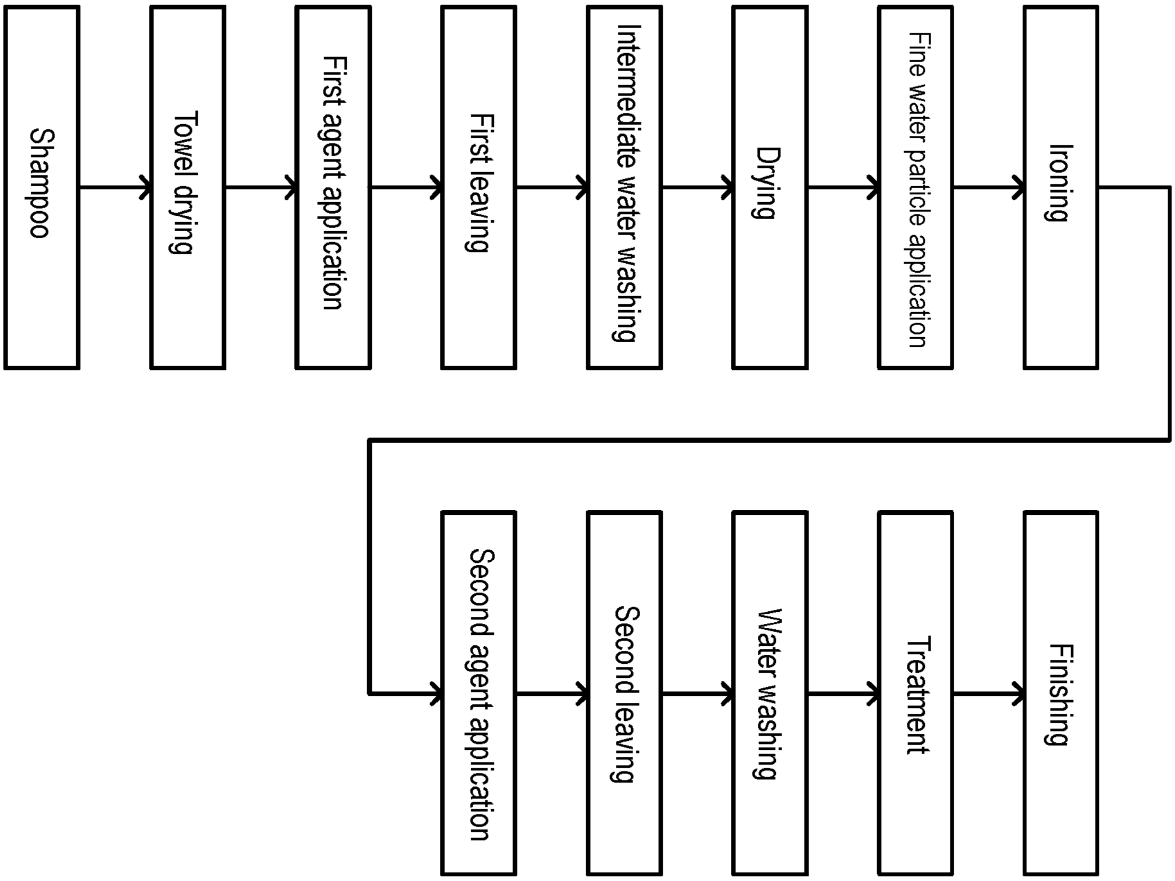
Shampoo
Towel drying
First agent application
First leaving
Intermediate water washing
Drying
Fine water particle application
Ironing
Second agent application
Second leaving
Water washing
Treatment
Finishing

*FIG. 33*

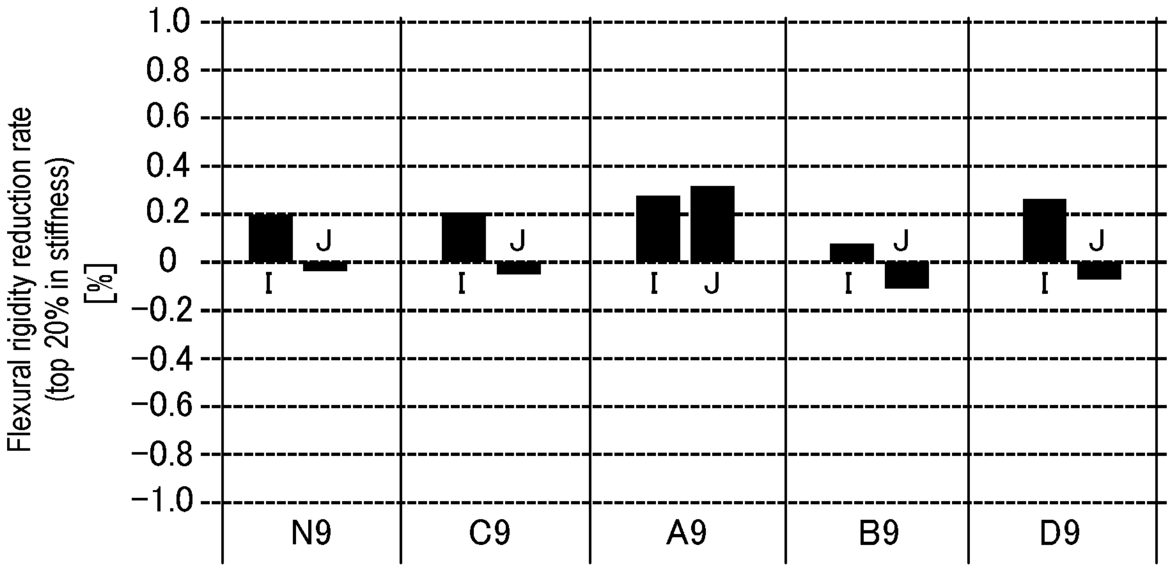
Flexural rigidity reduction rate
(top 20% in stiffness)
[%]
1.0
0.8
0.6
0.4
0.2
0
−0.2
−0.4
−0.6
−0.8
−1.0
I
J
N9
C9
A9
B9
D9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019077628 A
- JP 2020116032 A
- JP 2000201731 A